(19) Europäisches Patentamt / European Patent Office / Office européen des brevets

(11) **EP 2 994 146 B1**

(12) **EUROPEAN PATENT SPECIFICATION**

(45) Date of publication and mention
of the grant of the patent:
**03.03.2021 Bulletin 2021/09**

(21) Application number: **14794569.5**

(22) Date of filing: **08.05.2014**

(51) Int Cl.:
*A61K 33/24* (2019.01)   *A61K 9/14* (2006.01)
*A61K 9/16* (2006.01)   *A61K 9/10* (2006.01)
*A61P 3/06* (2006.01)   *A61P 21/04* (2006.01)
*A61P 25/00* (2006.01)   *A61P 25/02* (2006.01)
*A61P 25/04* (2006.01)   *A61P 25/28* (2006.01)
*A61P 27/02* (2006.01)   *A61P 43/00* (2006.01)

(86) International application number:
**PCT/US2014/037280**

(87) International publication number:
**WO 2014/182888 (13.11.2014 Gazette 2014/46)**

(54) **METHODS AND TREATMENT FOR CERTAIN DEMYELINATION AND DYSMYELINATION-BASED DISORDERS AND/OR PROMOTING REMYELINATION**

VERFAHREN UND BEHANDLUNG FÜR BESTIMMTE DEMYELISIERUNGS- UND DYSMYELINIERUNGS-VERMITTELTE ERKRANKUNGEN UND ODER ZUR REMYELINISIERUNGSFÖRDERUNG

MÉTHODES ET TRAITEMENT POUR CERTAINS TROUBLES DE LA DÉMYÉLINISATION ET DE LA DYSMYÉLINISATION ET/OU POUR FAVORISER LA REMYÉLINISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.05.2013 US 201361821040 P**

(43) Date of publication of application:
**16.03.2016 Bulletin 2016/11**

(73) Proprietors:
• **Clene Nanomedicine, Inc.**
  **Havre de Grace, MD 21078 (US)**
• **Zhang, Zhongyan**
  **Havre de Grace, Maryland 21078 (US)**
• **Mortenson, Mark G.**
  **Havre de Grace, Maryland 21078 (US)**

(72) Inventors:
• **ZHANG, Zhongyan**
  **Havre de Grace, Maryland 21078 (US)**
• **MORTENSON, Mark G.**
  **Havre de Grace, Maryland 21078 (US)**

(74) Representative: **Harris, Oliver John Richard et al**
**Novagraaf UK**
**Centrum**
**Norwich Research Park**
**Colney Lane**
**Norwich NR4 7UG (GB)**

(56) References cited:
**WO-A1-2011/006007    WO-A1-2011/006007
WO-A2-2012/061828    US-A1- 2006 068 026
US-A1- 2006 207 388    US-A1- 2007 140 966
US-A1- 2008 266 555    US-A1- 2009 169 807
US-A1- 2010 092 470**

• **DAN SONNE PEDERSEN ET AL: "Metallic gold slows disease progression, reduces cell death and induces astrogliosis while simultaneously increasing stem cell responses in an EAE rat model of multiple sclerosis", HISTOCHEMISTRY AND CELL BIOLOGY, SPRINGER, BERLIN, DE, vol. 138, no. 5, 22 July 2012 (2012-07-22) , pages 787-802, XP035124374, ISSN: 1432-119X, DOI: 10.1007/S00418-012-0996-2**

**(Cont. next page)**

- FROHMAN E M ET AL: "MOST PATIENTS WITH MULTIPLE SCLEROSIS OR A CLINICALLY ISOLATED DEMYELINATING SYNDROME SHOULD BE TREATED AT THE TIME OF DIAGNOSIS", ARCHIVES OF NEUROLOGY, AMERICAN MEDICAL ASSOCIATION, CHICAGO, IL, US, vol. 63, no. 4, 1 April 2006 (2006-04-01), pages 614-619, XP009072802, ISSN: 0003-9942, DOI: 10.1001/ARCHNEUR.63.4.614
- ROMMER P S ET AL: "Monoclonal antibodies in the therapy of multiple sclerosis; An overview", JOURNAL OF NEUROLOGY, STEINKOPFF-VERLAG, DA, vol. 255, no. 6, 1 December 2008 (2008-12-01), pages 28-35, XP019714358, ISSN: 1432-1459
- AKTAS O ET AL: "Neue therapeutische Konzepte bei der multiplen Sklerose /// Novel therapeutic strategies for multiple sclerosis - An overview", NERVENHEILKUNDE, SCHATTAUER, STUTTGART, DE, vol. 29, no. 5, 1 January 2010 (2010-01-01), pages 267-272, XP009182854, ISSN: 0722-1541

## Description

### FIELD OF THE INVENTION

The disclosure relates to methods and compositions for treating causes of dysmyelination and/or demyelination of neurons and/or preventing the development of myelin and axon-related diseases and/or promoting remyelination by administering to a subject in need thereof an effective amount (either therapeutic or prophylactic) and concentration of an elemental gold nanosuspension, and preferably a surface-clean gold-based nanocrystal suspension disclosed herein.

### BACKGROUND OF THE INVENTION

A demyelinating disease is any disease of the central nervous system ("CNS") and/or peripheral nervous system ("PNS"), in which the myelin sheaths of neurons become damaged. Damage to the myelin sheath typically adversely affects the conduction of signals in the affected nerves and/or results in some type of abnormal or inferior performance of the underlying neuron(s). The associated myelin damage results in deficiencies in any one of or all of: sensations, cognition, motor skills or other functions depending on which neurons/myelin sheaths are damaged or not normal.

The precise mechanisms of demyelination and dysmyelination are not clearly understood. Myelin is known to be a vital protein-based cover for neurons in each of the central nervous system and peripheral nervous system. This vital protein creates sheaths typically referred to as "myelin sheaths" around many neurons in a mammal. Myelin sheaths which are healthy and not defective will cause nerve signals to be both rapid and complete because healthy myelin sheaths permit electric potentials to be rapidly transmitted by neural axons; and/or promote healthy structure and/or function of the underlying neurons including, for example, loss of trophic and metabolic support. When myelin is removed, partially or completely from axons (e.g., demyelination), actual potential velocity of signals can slow by >> than 30 times their normal myelinated velocities.

Further, a myelin sheath is formed by something known as a plasmalemmal of glial cells (e.g., oligodendrocytes in the central nervous system and Schwann cells in the peripheral nervous system) also known as a plasma membrane. Myelin sheaths are generated at a relatively rapid pace during an active phase of myelination. Specifically, oligodendrocytes in the central nervous system need to produce sufficient myelin to result in natural "remyelination" during normal, healthy functioning. Thus, newly synthesized myelin is important to be produced on a regular basis.

Remyelination involves the generation of new myelin sheaths around denuded axons in the adult CNS. An immediate consequence of remyelination includes proper redistribution of ion channels at the nodes of Ranvier as well as the restoration of saltatory conduction. Thus remyelination partially resolves an increased energy demand that is observable by reduced axonal mitochondrial content. Further, remyelination results in the recovery of functional deficits caused by demyelination. Evidence also suggests that demyelinated axons are better protected from subsequent injury when they become remyelinated. Such remyelination may restore proper growth factor signaling between the oligodendrocyte and the axon. There is also evidence that the symbiotic relationship between the axon and oligodendrocyte is active and the role of myelin is not simply one of electrical insulation. Specifically, axons can become extensively damaged when oligodendrocyte cell bodies are targeted for ablation, even in the absence of any observable demyelination. Such process can result in dysmyelination or dysfunction.

Demyelination or dysmyelination has been associated with a large number of both acquired disorders and hereditary conditions of the central nervous system and the peripheral nervous system.

Experimental systems which create a set of conditions which attempt to obtain a result in an animal which correlates with or mimics at least some of the mechanisms/results responsible or associated with human diseases are well known. One of those systems is known as the Cuprizone Animal Model[15]. This "toxic demyelination model" results in alterations of mitochondrial morphology and it is speculated that the neuro-toxic properties of this copper-chelating compound are due to a disturbance of cellular respiration.[9] Cuprizone-induced demyelination results from degeneration of supporting oligodendrocytes rather than a direct attack on myelin sheaths.[10,11,12]

Moreover, the mechanisms responsible for oligodendroglial death in MS lesions are not clear. It is questionable whether similar pathomechanisms are responsible for oligodendrogial loss in Multiple Sclerosis ("MS") lesions and in the cuprizone model[9]. MS is presently regarded as a disorder with many different facets and features. Experts in the field challenge whether cuprizone-induced demyelination models the loss of myelin in human MS patients[9]. The specific pathogenesis of MS remains unknown.

Still further, disorders and diseases that do include demyelination that may be associated with the toxic demyelination models, such as the Cuprizone Animal Model, include Progressive Supranuclear Palsy, Alexander's Disease, Krabbe Disease, Metachromatic Leukodystrophy, Canvan Disease, Leukodistrophies, Encephalomyelitis, Central Pontine Myelolysis (CPM), Anti-MAG Disease, Pelizaeus-Merzbacher Disease, Refsum Disease, Cockayne Syndrome, Zellweger Syndrome, Guillain-Barre Syndrome (GBS), Van der Knapp Syndrome, chronic inflammatory demyelinating polyneuropathy (CIDP), multifocal motor neuropathy (MMN), Neuromyelitis Optica (NMO), Progressive Multifocal Leukoencephalopathy (PML), Wallerian Degeneration and some inherited diseases such as Adrenoleukodystrophy, Alexander's Disease, Mild Cognitive Impairment (MCI) also known as Age Related Cognitive Decline and Pelizaeus Merzbacher Disease (PMZ). For many of these aforementioned disorders, there are few to no cures and very few effective therapies, if any.

Neuromyelitis Optica (NMO), is also sometimes referred to as Devic's disease. NMO is a disorder of the central nervous system (CNS) that predominantly affects the optic nerve and spinal cord of patients. NMO is one of the major neuroimmunological diseases in Asia.

An NMO-immunoglobulin G (IgG) has been discovered in the sera of NMO patients, which binds at or near the blood-brain barrier in the mouse brain. The epitope of NMO-IgG was identified as aquaporin-4 (AQP4), a water channel densely expressed in astrocytic foot processes at the blood-brain barrier.

NMO is characterized by the occurrence of severe optic neuritis and myelitis, mostly observed as longitudinally extensive transverse myelitis (LETM), sometimes both occurring simultaneously and sometimes occurring sequentially. Most NMO patients have autoantibodies against AQP4 in their serum. Therefore, the NMO diagnostic criteria requires the presence of both optic neuritis and myelitis and fulfilment of at least two of the three supportive criteria: MRI evidence of a contiguous spinal cord lesion extending over three or more vertebral segments; negative results for the diagnostic criteria for MS on brain MRI34 conducted at onset; and NMO-IgG (or anti-AQP4 antibody) seropositivity.

Thus, NMO is now considered as an anti-AQP4 antibody-mediated astrocytopathy, and different from a demyelinating disorder such as MS. However, mammals having NMO clearly show the pathologic results of demyelination or dysmyelination.

## Comparison of Regeneration in the PNS and the CNS

Historically it has been believed that nerve regeneration is much more effective in the PNS than in the CNS. Researchers once thought that CNS neurons simply had less intrinsic ability to regenerate, but this paradigm was challenged by the discovery that CNS neurons could grow through a peripheral nerve graft. Comparisons of these two systems established that the inhibitory environment of the CNS is the greatest challenge for regeneration of CNS axons, and led to the discovery of several factors that encourage growth in the PNS or inhibit growth in the CNS. For example, oligodendrocyte myelin and Schwann cell myelin both contain inhibitory molecules. In the CNS, axonal outgrowth is also blocked at the site of injury by the glial scar, which is composed of reactive astrocytes and microglia. By contrast, no glial scar forms in the PNS, and the bands of Büngner formed by Schwann cells actually aid axon guidance and regeneration. Understanding these important differences in CNS and PNS regeneration can help to shape strategies for improving regeneration in nonpermissive environments, namely the CNS and chronically denervated PNS.

There remains a considerable need for materials and/or treatments to assist in stopping or retarding demyelination or dysmyelination and/or promoting remyelination and/or preserving or restoring myelin and/or axon functioning. Note that Pedersen et al (Histochemistry and Cell Biology, 2012, vol. 138, no. 5, pages 787 - 802) discloses that metallic gold slows disease progression, reduces cell death and induces astrogliosis while simultaneously increasing stem cell responses in an EAE rat model of multiple sclerosis, and that WO2011006007 discloses gold nanocrystals and electrochemical manufacturing processes therefor.

## DEFINITIONS

Throughout this specification and claims, the word "comprise," or variations such as "comprises" or "comprising," indicate the inclusion of any recited integer or group of integers but not the exclusion of any other integer or group of integers. The term "comprising" is inclusive or open-ended and does not exclude additional, unrecited elements or method steps. The phrase "consisting essentially of" indicates the inclusion of the specified materials or steps as well as those which do not materially affect the basic and novel characteristics of the claimed invention. As used herein, the term "consisting" refers only to indicated material or method steps.

As used herein, a "therapeutically effective amount" refers to an amount effective, at concentrations of gold nanocrystals and volume of suspension, and for periods of time and/or dosing necessary, to achieve a desired therapeutic result. A desired therapeutic result may include, but not be limited to, lessening of symptoms, prolonged survival, improved mobility or function, decreased severity of relapses, extended periods of remission, or the like. A "therapeutically effective amount" can achieve any one of the desired therapeutic results or any combination of multiple desired therapeutic results. A therapeutic result need not be a "cure". A therapeutic result also includes measured differences in the amount(s) of myelin damage, reduction in myelin demyelination and/or an increase in remyelination.

As used herein, a "prophylactically effective amount" refers to an amount effective, at concentrations of gold nanocrystals and volume of suspension, and for periods of time and/or dosing necessary, to achieve the desired prophylactic result. Typically, since a prophylactic dose is used in subjects prior to or at an earlier stage of disease, the prophylactically effective amount can be less than the therapeutically effective amount. A prophylactic result also includes measured differences in the amount(s) of myelin damage, reduction in myelin demylination and/or an increase in remyelination.

As used herein, the term "treatment" or "treating" refers to the administration of an elemental gold-based nanosuspension and in a preferred embodiment the novel gold-based nanocrystal suspension referenced as "CNM-Au8" herein, to a mammal in order to ameliorate or lessen the symptoms of a disease. Additionally, the terms "treatment" or "treating" refers to the administration of the aforementioned gold-based nanosuspensions to a mammal to prevent the progression of a disease. Preventing the progression of a disease also included measured differences in the amount(s) of myelin damage, reduction in myelin demylination and/or an increase in remyelination.

By "subject" or "individual" or "animal" or "patient" or "mammal," is meant any subject, particularly a mammalian subject, for whom diagnosis, prognosis, therapy and/or prevention is desired. Mammalian subjects include, but are not limited to, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; rodents such as mice, rats, hamsters and guinea pigs; and so on. In certain embodiments, the mammal is a human subject.

**SUMMARY** - of both claimed and non-claimed technology

The claimed invention provides an elemental gold nanosuspension for use in a method of treatment of a disease selected from the group consisting of Progressive Supranuclear Palsy, Alexander's Disease, Krabbe Disease, Metachromatic Leukodystrophy, Canvan Disease, Leukodistrophies, Central Pontine Myelolysis (CPM), Anti-MAG Disease, Pelizaeus-Merzbacher Disease, Refsum Disease, Cockayne Syndrome, Zellweger Syndrome, Guillain-Barre Syndrome (GBS), Van der Knapp Syndrome, chronic inflammatory demyelinating polyneuropathy (CIDP), multifocal motor neuropathy (MMN), Neuromyelitis Optica (NMO), Progressive Multifocal Leukoencephalopathy (PML), Mild Cognitive Impairment (MCI), Wallerian Degeneration, and Adrenoleukodystrophy. Preferred features of this claimed invention are set out in the dependent claims of the appended claim set.

Preferably, new gold nanocrystals are suspended in high purity water and the gold nanocrystals have nanocrystalline surfaces that are substantially free (as defined herein) from organic or other impurities or films, but remain stably suspended in the water. Specifically, the surfaces are "clean" relative to those made using chemical reduction processes that require chemical reductants and/or surfactants to grow gold nanoparticles from gold ions in solution. The majority of the grown gold nanocrystals have unique and identifiable surface characteristics such as spatially extended low index, crystal planes {111}, {110} and/or {100} and groups of such planes (and their equivalents). Resulting gold nanocrystalline suspensions or colloids that have desirable pH ranges such as 4.0 - 9.5, but more typically 5.0 -9.5 and zeta potential values of at least -20mV, and more typically at least -40mV and even more typically at least -50mV for the pH ranges of interest.

The shapes and shape distributions of these gold nanocrystals prepared according to the manufacturing process described below include, but are not limited to, triangles (e.g., tetrahedrons), pentagons (e.g., pentagonal bipyramids or decahedrons), hexagons (e.g., hexagonal bipyramids, icosahedrons, octahedrons), diamond (e.g., octahedrons, various elongated bipyramids, fused tetrahedrons, side views of bipyramids) and "others". The shape distribution(s) of nanocrystals containing the aforementioned spatially extended low index crystal planes (which form the aforementioned shapes) and having "clean" surfaces is unique.

Any desired average size of gold nanocrystals below 100 nm can be provided. The most desirable gold crystalline size ranges include those having an average crystal size or "mode" (as measured and determined by specific techniques disclosed in detail herein and reported as "TEM average diameter") that is predominantly less than 100 nm, and more typically less than 50 nm, even more typically less than 30 nm, and in many of the preferred embodiments disclosed herein, the mode for the nanocrystal size distribution is less than 21 nm and within an even more preferable range of 8-18 nm.

Any concentration of gold nanoparticle(s) can be provided to achieve a therapeutically effective amount or a prophylactically effective amount.

We describe a preferred process to produce these unique, clean-surfaced, gold nanocrystals stably suspended in water. The process involves the growth of the gold nanocrystals in water. Preferably, the water contains an added "process enhancer" which does not significantly bind to the formed nanocrystals, but rather facilitates nucleation/crystal growth during the electrochemical-stimulated growth processes. The process enhancer serves important roles in the process including providing charged ions in the electrochemical solution to permit the crystals to be grown. These novel electrochemical processes can occur in either a batch, semi-continuous or continuous process. These processes result in controlled gold nanocrystalline concentrations, controlled nanocrystal sizes and controlled nanocrystal size ranges; as well as controlled nanocrystal shapes and controlled nanocrystal shape distributions. Novel manufacturing assemblies are provided to produce these gold nanocrystals. Novel Tangential Flow Filtration ("TFF") techniques are used to obtain higher gold ppm's and be stable (i.e, suspensions with zeta potential values of at least -20mV, and more typically at least - 40mV and even more typically at least -50mV for the pH ranges of interest) in concentrations up to 3,000 ppm (i.e., 3,000 μg/ml).

Pharmaceutical compositions are described that are appropriate for systemic use, including oral, intravenous, subcutaneous, intraarterial, buccal, inhalation, aerosol, propellant or other appropriate liquid, etc., as described further herein.

Pharmaceutical compositions can include a therapeutically effective amount or a prophylactically effective amount of the gold nanocrystals to treat, ameliorate or prevent any of the medical/pathological conditions described in this application. Preferably, the gold nanocrystals are administered in an orally delivered liquid, wherein the gold nanocrystals remain in the water of manufacture, which may be concentrated or reconstituted, but preferably not dried to the point that the surfaces of the gold nanocrystals become completely dry or have their surfaces otherwise altered from their pristine state of manufacture.

It is important to recognize that in pharmaceutical products the objective is to establish the minimum dose necessary to achieve efficacy, thus minimizing potential for toxicity or complications. A new orally administered product with significantly greater potency can achieve efficacy at dose levels below those of prior art products, and/or can achieve substantially greater efficacy at equivalent dose levels. Clinical trials are required to confirm, for example, the therapeutically effective amount. However, titration to clinical effect can be achieved by, for example, varying concentration, volume, time and/or dosing frequency.

Pharmaceutical compositions are described that are appropriate for systemic use, including oral, intravenous, subcutaneous, intra-arterial, buccal, inhalation, aerosol, propellant or other appropriate liquid, etc., as described further herein.

Suitable dosage amounts and dosing regimens can be determined by the attending physician or veterinarian and may depend on the desired level of inhibiting and/or modifying activity, the particular condition being treated, the severity of the condition, whether the dosage is a therapeutically effective amount or a prophylactically effective amount, as well as the general age, health and weight of the subject.

The gold nanocrystals contained in an aqueous medium, may be administered in a single dose or a series of doses. While it is possible for the aqueous medium containing the metallic-based nanocrystals to be administered alone in, for example, colloid form, it may be acceptable to include the active ingredient mixture with other compositions and or therapies. Further, various pharmaceutical compositions can be added to the active ingredient(s)/suspension(s)/colloid(s).

Preferably, the gold nanocrystal suspensions or colloids (e.g., comprising aqueous gold-based metal) can be administered in conjunction with a second therapeutic agent. The second therapeutic agent could include a glucocorticoid.

Gold nanocrystal suspensions according to the present disclosure suitable for oral administration are presented typically as a stable solution, colloid or a partially stable suspension in water. However, such gold nanocrystals may also be included in a non-aqueous liquid, as discrete units such as liquid capsules, sachets or even tablets (e.g., drying-out suspensions or colloids to result in active ingredient gold-based nanocrystals so long as such processing does not adversely affect the functionality of the pristine gold nanocrystal surfaces) each containing a predetermined amount, of, for example, the gold nanocrystal active ingredient; as a powder or granules; as a solution, colloid or a suspension in an aqueous or as non-aqueous liquid; or as an oil-in-water liquid emulsion or a water-in-oil liquid emulsion. The gold nanocrystal active ingredient may also be combined into a bolus, electuary or paste. It should be understood that different elemental gold nanosuspensions may be used as the material for the treatments discussed herein.

Compositions suitable for oral administration in the mouth include lozenges comprising suspensions or colloids containing one or more active ingredient(s) gold nanocrystal in a flavored base, such as sucrose and acacia or tragacanth gum; pastilles comprising the gold nanocrystal active ingredient in an inert base such as a gelatin and a glycerin, or sucrose and acacia gum; and mouthwashes comprising the gold nanocrystal active ingredient in a suitable liquid carrier.

The gold nanocrystal suspensions or colloids may also be administered intranasally or via inhalation, for example by atomiser, aerosol or nebulizer means for causing one or more constituents in the solution or colloid (e.g., the gold nanocrystals) to be, for example, contained within a mist or spray.

Compositions for rectal administration may be presented as a suppository with a suitable carrier base comprising, for example, cocoa butter, gelatin, glycerin or polyethylene glycol.

Compositions suitable for vaginal administration may be presented as pessaries, tampons, creams, gels, pastes, foams or spray formulations containing in addition to the active ingredient such carriers as are known in the art to be appropriate.

Compositions suitable for parenteral administration include aqueous and non-aqueous isotonic sterile injection suspensions or colloids which may contain anti-oxidants, buffers, bactericides and solutes which render the composition isotonic with the blood of the intended recipient; and aqueous and non-aqueous sterile suspensions which may include suspending agents and thickening agents. The compositions may be presented in unit-dose or multi-dose sealed containers, for example, ampoules and vials, and may be stored in a freeze-dried (lyophilised) condition requiring only the addition of the sterile liquid carrier, for example water for injections, immediately prior to use. Extemporaneous injection solutions, colloids and suspensions may be prepared from sterile powders, granules and tablets of the kind previously described.

Preferred unit dosage compositions are those containing a daily dose or unit, daily subdose, as herein above described, or an appropriate fraction thereof, of the active ingredient.

It should be understood that in addition to the gold nanocrystal active ingredients particularly mentioned above, the compositions may include other agents conventional in the art having regard to the type of composition in question, for example, those suitable for oral administration may include such further agents as binders, sweeteners, thickeners, flavouring agents, disintegrating agents, coating agents, preservatives, lubricants, time delay agents and/or position release agents. Suitable sweeteners include sucrose, lactose, glucose, aspartame or saccharine. Suitable disintegrating agents include corn starch, methylcellulose, polyvinylpyrrolidone, xanthan gum, bentonite, alginic acid or agar. Suitable flavouring agents include peppermint oil, oil of wintergreen, cherry, orange or raspberry flavouring. Suitable coating agents include polymers or copolymers of acrylic acid and/or methacrylic acid and/or their esters, waxes, fatty alcohols, zein, shellac or gluten. Suitable preservatives include sodium benzoate, vitamin E, alpha-tocopherol, ascorbic acid, methyl paraben, propyl paraben or sodium bisulphite. Suitable lubricants include magnesium stearate, stearic acid, sodium oleate, sodium chloride or talc. Suitable time delay agents include glyceryl mono stearate or glyceryl distearate.

These elemental gold nanosuspensions, and preferably the substantially surface-clean or surface-pure gold nanocrystals suspended in high purity water, can be used to treat any disorder provided in the Background of the Invention, above. Further, the phrase "elemental gold nanosuspensions" or "elemental gold crystal nanosuspensions" or the like, should be understood as including the CNM-Au8 nanosuspensions expressly disclosed herein, but should also be understood as including other elemental gold nanosuspensions made by completely different techniques, so long as the general physical properties including, nanoparticle size, concentration(s), pH, etc., are within the same ranges as the physical properties of the CNM-Au8 nanosuspensions disclosed in detail herein, even if such nanosuspensions have certain drawbacks associated therewith.

## BRIEF DESCRIPTION OF THE FIGURES

FIG. 1 shows a first trough member 30a' wherein one plasma 4a is created. The output of this first trough member 30a' flows into a second trough member 30b'.

FIGs. 2A-2C show an alternative design of the trough member 30b' wherein the trough member portions 30a' and 30b' are contiguous.

FIG. 3 shows the trough member 30b' used in connection with FIGs. 2A-2C and Example 1 herein.

FIGs. 4A-4B show two cross-sectional views of two trough members 30.

FIG. 5A shows an AC transformer electrical wiring diagram for use in making the plasma 4 used in making the nanocrystalline suspension discussed in Example 1. FIG. 5B shows a schematic view of a transformer 60 and FIGs. 5C and 5D show schematic representations of two sine waves in phase and out of phase, respectively.

FIG. 6 shows a representative configuration for the electrode 1.

FIG. 7 shows a view of the gold wires 5a and 5b used in Example 1 herein.

FIG. 8 is a schematic of the power supply electrical setup used to generate the gold nanocrystal suspensions discussed in Example 1.

FIG. 9 shows a schematic cross-sectional view of a set of control devices 20 located on a trough member 30 with a liquid 3 flowing therethrough and into a storage container 41.

FIG. 10A shows a representative TEM photomicrograph of dried gold nanocrystals formed in connection with Example 1.

FIG. 10B shows a particle size distribution histogram from TEM measurements for the dried gold nanocrystals formed in connection with Example 1.

FIG. 10C shows the UV-Vis spectral patterns of the gold suspension made according to Example 1.

FIG. 11 is a schematic representation of a TFF apparatus used to concentrate the gold nanosuspensions.

FIG. 12 shows a perspective view of the device and process used to make coronal brain sections discussed in Example 2.

FIG. 13 is a bar chart which shows the relative amount of myelin staining present in mouse Groups 1-4 of Example 2.

FIGs. 14A-14D show TEM images of representative portions of the corpus callosum for a single mouse from each of mouse Groups 1-4, respectively, from Example 2.

FIG. 15 shows a bar chart of G-ratios measured/calibrated from observing about 100 axons in each corpus callosum TEM image set from one mouse from each of Groups 1-4, respectively, from Example 2.

FIG. 16 shows the data scatter patterns associated with the G-ratio calculations from one mouse from each of Groups 1-4, respectively, from Example 2..

FIGs. 17A-D show histograms of the actual G-ratio data compared to generated bell-shaped curves for one mouse from each of mouse Groups 1-4, respectively, from Example 2.

FIG. 18 shows a series of plots corresponding to the average amount of liquid consumed by each of mouse Groups 1-4 from Example 2 throughout the study.

FIG. 19 shows a series of plots corresponding to the average weight of each in Mouse Groups 1-4, from Example 2, as measured throughout the study.

FIGs. 20A-20F show several schematic views of the portions of the brain that are subject to the sample preparation techniques discussed in Example 3.

FIGs. 21A-21B shows the apparatus for holding and cutting brain slices utilized to obtain thin sections for the TEM images discussed in Example 3.

FIG. 22 shows a series of plots corresponding to the average weight gain of each mouse in Groups 1-7, starting at 8 weeks of age, as discussed in Example 3.

FIGs. 23A-23C correspond to TEM images, originally taken at 4,000x, of representative portions of the corpus callosum for mice from Group 1, Example 3.

FIGs. 24A-24E correspond to TEM images, originally taken at 4,000x, of representative portions of the corpus callosum for mice from Group 2, Example 3.

FIGs. 25A-25G correspond to TEM images, originally taken at 4,000x, of representative portions of the corpus callosum for mice from Group 3, Example 3.

FIGs. 26A-26E correspond to TEM images, originally taken at 4,000x, of representative portions of the corpus callosum for mice from Group 4, Example 3. Areas of observed remyelination are indicated by the arrows 201.

FIGs. 27A-27D correspond to TEM images, originally taken at 4,000x and 5,000x, of representative portions of the corpus callosum for mice from Group 5, Example 3. Areas of observed remyelination are indicated by the arrows 201.

FIGs. 28A-28G correspond to TEM images, originally taken at 4,000x, of representative portions of the corpus callosum for mice from Group 6, Example 3. Areas of observed remyelination are indicated by the arrows 201.

FIGs. 29A-29D correspond to TEM images, originally taken at 4,000x, of representative portions of the corpus callosum for mice from Group 7, Example 3. Areas of observed remyelination are indicated by the arrows 201.

FIGs. 30A-30C show representative TEM images, originally taken at about 16,000x, showing representative portions of the corpus callosum where axons are indicated as being damaged, demyelinated and/or dysmyelinated, by the black box and arrows 202S, in FIG. 30A, (and only the arrows 202 in FIG. 30B and FIG. 30C), relative to the Reference Axon marked by the star 203, for mice from Group 1, Example 3.

FIGs. 31A-31B show representative TEM images, originally taken at about 16,000x, showing representative portions of the corpus callosum where axons are indicated as being damaged, demyelinated and/or dysmyelinated, by the arrows 202, relative to the Reference Axon marked by the star 203, for mice from Group 2, Example 3.

FIGs. 32A-32B show representative TEM images, originally taken at about 16,000x, showing representative portions of the corpus callosum where axons are indicated as being damaged, demyelinated and/or dysmyelinated, by the arrows 202, relative to the Reference Axon marked by the star 203, for mice from Group 3, Example 3.

FIGs. 33A-33B show representative TEM images, originally taken at about 16,000x, showing representative portions of the corpus callosum where axons are indicated as being damaged, demyelinated and/or dysmyelinated, by the arrows 202, relative to the Reference Axon marked by the star 203, for mice from Group 4, Example 3.

FIGs. 34A-34B show representative TEM images, originally taken at about 16,000x, showing representative portions of the corpus callosum where axons are indicated as being damaged, demyelinated and/or dysmyelinated, by the arrows 202, relative to the Reference Axon marked by the star 203, for mice from Group 5, Example 3.

FIGs. 35A-35B show representative TEM images, originally taken at about 16,000x, showing representative portions of the corpus callosum where axons are indicated as being damaged, demyelinated and/or dysmyelinated, by the arrows 202, relative to the Reference Axon marked by the star 203, for mice from Group 6, Example 3.

FIGs. 36A-36B show representative TEM images, originally taken at about 16,000x, showing representative portions of the corpus callosum where axons are indicated as being damaged, demyelinated and/or dysmyelinated, by the arrows 202, relative to the Reference Axon marked by the star 203, for mice from Group 7, Example 3.

FIGs. 37A-37K show representative TEM images, originally taken at 16,000x or 40,000x, which correspond to representative portions of the corpus callosum of mice in Group 4. Areas of observed remyelination are indicated by the arrows 201M.

FIGs. 38A-38L show representative TEM images, originally taken at 16,000x or 40,000x, which correspond to representative portions of the corpus callosum of mice in Group 5. Areas of observed remyelination are indicated by the arrows 201M.

FIGs. 39A-39J show representative TEM images, originally taken at 16,000x or 40,000x, which correspond to representative portions of the corpus callosum of mice in Group 6. Areas of observed remyelination are indicated by the arrows 201M.

FIGs. 40A-40G show representative TEM images, originally taken at 16,000x or 40,000x, which correspond to representative portions of the corpus callosum of mice in Group 7. Areas of observed remyelination are indicated by the arrows 201M.

FIGs. 41A-41C show representative TEM photomicrograph images which correspond to representative portions of the corpus callosum of mice in Group 1, Example 3. These images are high magnification, 40,000x images, showing that inner (2041) and outer (204O) perimeters of the myelin have been labeled on each axon thereon.

FIGs. 42A-42D show representative TEM photomicrograph images which correspond to representative portions of the corpus callosum of mice in Group 2, Example 3. These images are high magnification, 40,000x images, showing that inner (2041) and outer (204O) perimeters of the myelin have been labeled on each axon thereon.

FIGs. 43A-43C show representative TEM photomicrograph images which correspond to representative portions of the corpus callosum of mice in Group 3, Example 3. These images are high magnification, 40,000x images, showing that inner and outer perimeters of the myelin have been labeled on each axon thereon.

FIGs. 44A-44B show representative TEM photomicrograph images which correspond to representative portions of the corpus callosum of mice in Group 4, Example 3. These images are high magnification, 40,000x images, showing that inner and outer perimeters of the myelin have been labeled on each axon thereon.

FIGs. 45A-45C show representative TEM photomicrograph images which correspond to representative portions of the corpus callosum of mice in Group 5, Example 3. These images are high magnification, 40,000x images, showing that inner and outer perimeters of the myelin have been labeled on each axon thereon.

FIGs. 46A-46B show representative TEM photomicrograph images which correspond to representative portions of the corpus callosum of mice in Group 6, Example 3. These images are high magnification, 40,000x images, showing that inner and outer perimeters of the myelin have been labeled on each axon thereon.

FIGs. 47A-47E show representative TEM photomicrograph images which correspond to representative portions of the corpus callosum of mice in Group 7, Example 3. These images are high magnification, 40,000x images, showing that inner and outer perimeters of the myelin have been labeled on each axon thereon.

FIGs. 48A, 48B and 48C contain Modified Bar Chart Histograms reporting G-ratio data, corresponding to mice in Group 1 (positive control), Group 2 (2 week negative control) and Group 3 (5 week negative control), respectively. These three Modified Bar Chart Histograms have been placed together for comparison purposes.

FIGs. 49A, 49B and 49C contain Modified Bar Chart Histograms reporting G-ratio data, corresponding to mice in Group 3 (5 week negative control), Group 5 and Group 7, respectively. These three Modified Bar Chart Histograms have been placed together for comparison purposes.

FIGs. 50A, 50B and 50C also contain Modified Bar Chart Histograms reporting G-ratio data, corresponding to mice in Group 3 (5 week negative control), Group 4 and Group 6, respectively. These three Modified Bar Chart Histograms have been placed together for comparison purposes.

FIG 51 contains Modified Bar Chart Histograms reporting G-ratio data, corresponding to mice for all of Groups 1-7.

## DETAILED DESCRIPTION - of both claimed and non-claimed technology

Hereon in, "embodiment" can relate to an embodiment of the claimed invention (as per the appended claims) or to an embodiment that does not form part of the claimed invention but can represent background art useful for understanding the claimed invention.

### Manufacturing Gold (CNM-Au8) Nanosuspensions

In a preferred embodiment, elemental gold nanocrystals are suspended in high purity water and the gold nanocrystals have nanocrystalline surfaces that are substantially free (as defined herein) from organic or other impurities or films. Specifically, the surfaces are "clean" relative to those made using chemical reduction processes that require chemical reductants and/or surfactants to form gold nanoparticles from gold ions in solution. The preferred gold nanocrystals are produced via novel manufacturing procedures, described in detail herein. The manufacturing procedures avoid the prior use of added chemical reductants and/or surfactants (e.g., organic compounds) or other agents which are typically carried along in, or on, the particles or are coated on the surface of the chemically reduced particles; or the reductants are subsequently stripped or removed using undesirable processes which themselves affect the particle.

In a preferred embodiment, the process involves the nucleation and growth of the elemental gold nanocrystals in water which contains a "process enhancer" or "processing enhancer" (typically an inorganic material or carbonate or such) which does not significantly bind to the formed nanocrystals, but rather facilitates nucleation/growth during electrochemical-stimulated growth process. The process enhancer serves important roles in the process including providing charged ions in the electrochemical solution to permit the crystals to be grown. The process enhancer is critically a compound(s) which remains in solution, and/or does not form a coating (e.g., an organic coating), and/or does not adversely affect the formed nanocrystals or the formed suspension(s), and/or is destroyed, evaporated, or is otherwise lost during the electrochemical process. A preferred process enhancer is sodium bicarbonate. Examples of other process enhancers are sodium carbonate, potassium bicarbonate, potassium carbonate, trisodium phosphate, disodium phosphate, monosodium phosphate, potassium phosphates or other salts of carbonic acid or the like. Further process enhancers may be salts, including sodium or potassium, of bisulfite or sulfite. Still other process enhancers to make gold nanocrystals for use as a medical treatment may be other salts, including sodium or potassium, or any material that assists in the electrochemical growth processes described herein; which is not substantially incorporated into or onto the surface of the gold nanocrystasl; and does not impart undesirable toxicity to the nanocrystals or to the suspension media containing the nanocrystals.

Desirable concentration ranges for the processing enhancer include typically 0.01 - 20 grams/gallon (0.0026-2.1730 mg/ml), more typically, 0.1 - 7.5 grams/gallon (0.0264 - 1.9813 mg/ml) and most typically, 0.5 - 2.04 grams/gallon (0.13210 - 0.54 mg/ml).

Because the grown gold nanocrystals have "bare" or "clean" surfaces of gold metal (e.g., in the zero oxidation state) the surfaces are highly reactive or are highly biocatalytic (as well as highly bioavailable). The nanocrystals are essentially surrounded by a water jacket. These features provide increased efficacy in vivo relative to nanoparticle surfaces that contain, for example, organic material present from reduction chemistry processes. The "clean" surfaces may also, or alternatively, reduce undesired toxicity of the nanocrystals, over those nanoparticles that contain coated or "dressed" surfaces. The increased efficacy of these "clean" gold nanocrystals may provide an increased therapeutic index via a lower dose needed to achieve a desired therapeutically effective amount or a desired prophylactically effective amount in a subject.

There are other important advantages of using the novel nanocrystals for treatment of a subject which include relative toxicity and/or relative speed of onset of benefits in a subject.

According to the processes herein, the preferred gold nanocrystals can be grown in a manner that provides unique and identifiable surface characteristics such as spatially extended low index, crystal planes {111}, {110} and/or {100} and groups of such planes (and their equivalents). The shapes of the gold nanocrystals prepared according to the processes described herein include, but are not limited to, triangles (e.g., tetrahedrons), pentagons (e.g., pentagonal bipyramids or decahedrons), hexagons (e.g., hexagonal bipyramids, icosahedrons, octahedrons), diamond (e.g., octahedrons, various eleongated bipyramids, fused tetrahedrons, side views of bipyramids) and "others". The percent of nanocrystals (i.e., grown by various embodiments set forth herein) containing the aforementioned spatially extended low index crystal planes and having "clean" surfaces is another novel aspect. Furthermore, the percent of tetrahedrons and/or pentagonal bipyramids formed or present in the nanocrystalline suspensions is/are also unique.

Any desired average size of gold nanocrystals below 100nm can be provided. The most desirable crystalline size ranges for treatments include those having an average crystal size or "mode" (as measured and determined by specific techniques disclosed in detail herein and reported as "TEM average diameter") that is predominantly less than 100 nm, and more typically less than 50 nm, even more typically less than 30 nm, and in many of the preferred embodiments disclosed herein, the mode for the nanocrystal size distribution is less than 21 nm and within an even more preferable range of 8-18nm.

Resulting gold nanocrystalline suspensions for treatments can be provided that have or are adjusted to have target pH ranges. When prepared with, for example, a sodium bicarbonate process enhancer, in the amounts disclosed in detail herein, the pH range is typically 8-9, which can be adjusted as desired.

The nature and/or amount of the surface change (i.e., positive or negative) on formed nanoparticles or nanocrystals can have a large influence on the behavior and/or effects of the nanoparticle/suspension or colloid. For example, protein coronas such as albumin coronas formed in vivo in a subject can be influenced by surface charge or surface characteristics of a nanoparticle. Such surface charges are commonly referred to as "zeta potential". It is known that the larger the zeta potential (either positive or negative), the greater the stability of the nanoparticles in the solution (i.e., the suspension is more stable). By controlling the nature and/or amount of the surface charges of formed nanoparticles or nanocrystals, the performance of such nanoparticle suspensions can be controlled.

Zeta potential is known as a measure of the electro-kinetic potential in colloidal systems and is also referred to as surface charge on particles. Zeta potential is the potential difference that exists between the stationary layer of fluid and the fluid within which the particle is dispersed. A zeta potential is often measured in millivolts (i.e., mV). The zeta potential value of approximately 20- 25mV is an arbitrary value that has been chosen to determine whether or not a dispersed particle is stable in a dispersion medium. Thus, when reference is made herein to "zeta potential", it should be understood that the zeta potential referred to is a description or quantification of the magnitude of the electrical charge present at the double layer.

The zeta potential is calculated from the electrophoretic mobility by the Henry equation:

$$U_E = \frac{2\varepsilon z f(ka)}{3\eta}$$

where z is the zeta potential, $U_E$ is the electrophoretic mobility, $\varepsilon$ is a dielectric constant, $\eta$ is a viscosity, $f(ka)$ is Henry's function. For Smoluchowski approximation $f(ka)$=1.5.

Zeta potentials ("ZP") for the gold nanocrystals prepared according the methods herein typically have a ZP of at least -20mV, more typically at least about -30mV, even more typically, at least about -40mV and even more typically at least about -50mV.

The suspensions can be concentrated to higher ppm levels (e.g., up to 5,000 ppm, but more typically up to 3,000 ppm) by using the TFF techniques discussed in Example 1 herein.

The following Examples all relate to the claimed invention. Their presence though should not be interpreted as meaning that the scope of protection is other than that set out by the appended claims, which are directed to an elemental gold nanosuspension for use in a method of treatment of a disease selected from the list of diseases recited in claim 1.

**EXAMPLE 1**

**Manufacturing Gold Nanosuspension "CNM-Au8" to be used for the Treatment of a Subject**

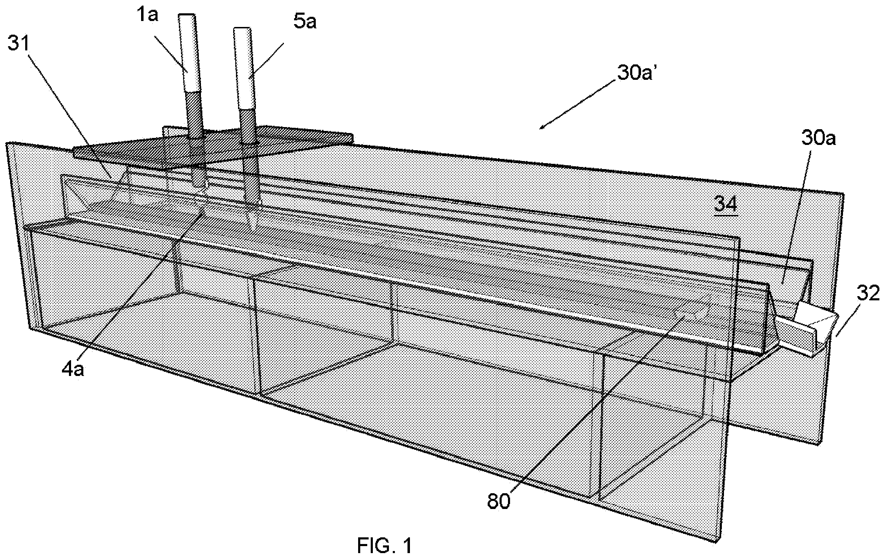

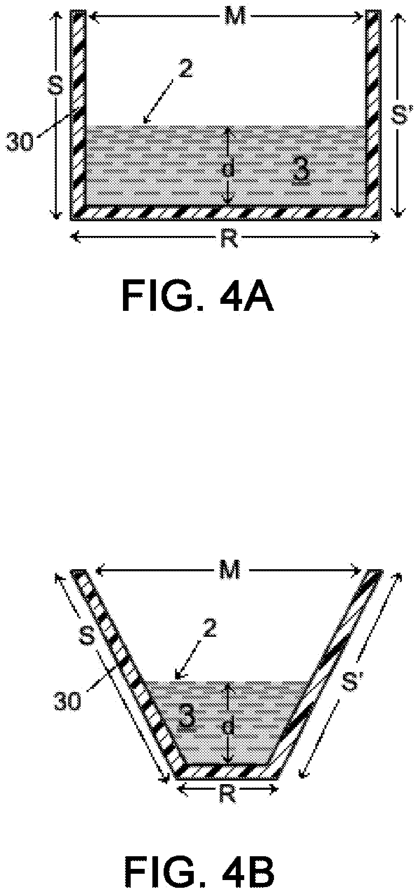

In general, the CNM-Au8 nanosuspensions utilized for treatment purposes in Examples 2 and 3 are concentrated CNM-Au8 "neat" nanosuspensions, the neat product being made by utilizing certain technology associated with the apparatuses generally shown in FIGs. 1, 2C, and 3. All trough members 30a' and 30b' in the aforementioned FIGs. were made from 1/8" (about 3mm) thick plexiglass, and 1/4" (about 6mm) thick polycarbonate, respectively. The support structure 34 (not shown in many of the FIGs. but shown in FIG. 1) was also made from plexiglass which was about ¼" thick (about 6-7mm thick). Each trough member 30a' was integral with trough member 30b'. The cross-sectional shape of the trough member 30a' described herein corresponded to that shape shown in FIG. 4B (i.e., was a trapezoidal-shaped cross-section). Relevant dimensions for 30a' were "S,S'" which measured about 1.5" (about 3.81cm), "M" which measured about 2.5" (about 6.35cm), "R" measured about 3/4" (about 1.9cm) and "d'" which measured about ½" (about 1.3cm).

Each trough member portion 30b' had a cross-sectional shape corresponding to FIG. 4A. The relevant dimensions for trough member portion 30b' are reported in Table 1 as "M" (i.e., inside width of the trough at the entrance and exact portion of the trough member 30b'), "$L_T$" (i.e., transverse length or flow length of the trough member 30b'), "S" (i.e., the height of the trough member 30b'), and "d''" (i.e., depth of the liquid 3" within the trough member 30b'). The thickness of each sidewall portion of trough 30b' also measured about 1/4" (about 6mm) thick.

The water 3 used as an input into the trough member 30a' (i.e., used in combination with the processing enhancer NaHCO3) was produced by a deionization process (referred to herein as de-ionized water). A mixed bed deionization filter was used. The total dissolved solvents ("TDS") after deionization treatment was about 0.2ppm, as measured by an Accumet® AR20 pH/conductivity meter.

Table 1

| | Run ID: | CNM-Au8 |
|---|---|---|
| Flow Rate: | In (ml/min) | 215 |
| Volts: | Set # 1 | 750 |
| | Set #'s 2-8 | 220 |
| Set #'s 1-8 frequency, Hz | | 60 |
| PE/Concentration (mg/mL) | | 0.54 |
| Wire Diameter (mm) | | 1.0 |
| Contact "$W_L$" (in/mm) | | 1/25.4 |
| Electrode Separation "y" (in/mm) | | .25/6.4 |
| Electrode Config. FIG. | | 7, 3 |
| Produced Au PPM | | 7.2 |
| Output Temp ºC at 32 | | 72 |
| Dimensions | Plasma 4 FIGs. | 1 |
| | Process FIGs. | 2C |
| | M (in/mm) | 1.5/38 |
| | LT (in/mm) | 36/914 |
| | d" (in/mm) | 1/25 |
| | S (in/mm) | 1.5/38 |
| Total Electrode Current Draw (A) | | 6.5 |
| Hydrodynamic r (nm) | | 17.95 |
| TEM Avg. Dia. (nm) | | 11.7 |
| Zeta Potential (mV) | | -42.9 |
| Set 1 | "c-c" (mm) | 76 |
| | electrode # | 1a |
| | "x" (in/mm) | 0.25/6.4 |
| | electrode # | 5a |
| Set 2 | "c-c" (mm) | 102 |
| | electrode # | 5b |
| | "x" (in/mm) | n/a |
| | electrode # | 5b' |
| | "c-c" (mm) | 76 |

| Set 3 | electrode # | 5c |
|---|---|---|
| | electrode # | 5c' |
| | "c-c" (mm) | 76 |
| Set 4 | electrode # | 5d |
| | electrode # | 5d' |
| | "c-c" (mm) | 127 |
| Set 5 | electrode # | 5e |
| | electrode # | 5e' |
| | "c-c" (mm) | 127 |
| Set 6 | electrode # | 5f |
| | electrode # | 5f' |
| | "c-c" (mm) | 152 |
| Set 7 | electrode # | 5g |
| | electrode # | 5g' |
| | "c-c" (mm) | 178 |
| Set 8 | electrode # | 5h |
| | electrode # | 5h' |
| | "c-c" (mm) | 76 |

Table 1 shows that the amount of processing enhancer (PE) (NaHCO$_3$) that was added to purified water was about 0.54 mg/ml. It should be understood that other amounts of this processing enhancer also function within the metes and bounds of the preferred embodiment of the invention. The purified water/ NaHCO$_3$ mixture was used as the liquid 3 input into trough member 30a'. The depth "d"' of the liquid 3' in the trough member 30a' (i.e., where the plasma(s) 4 is formed) was about 7/16" to about ½" (about 11mm to about 13mm) at various points along the trough member 30a'. The depth "d"' was partially controlled through use of the dam 80 (shown in FIG. 1). Specifically, the dam 80 was provided near the output end 32 of the trough member 30a' and assisted in creating the depth "d"' (shown in FIG. 4B as "d") to be about 7/6"-1/2" (about 11-13mm) in depth. The height of the dam 80 measured about ¼" (about 6mm) and the longitudinal length measured about ½" (about 13mm). The width was completely across the bottom dimension "R" of the trough member 30a'. Accordingly, the total volume of liquid 3' in the trough member 30a' during operation thereof was about 2.14in$^3$ (about 35ml) to about 0.89in$^3$ (about 14.58ml).

The rate of flow of the liquid 3' into the trough member 30a' as well as into trough member 30b', was about 215 ml/minute and the rate of flow out of the trough member 30b' at the point 32 was about 215 ml/minute. Other acceptable flow rates should be considered to be within the metes and bounds of manufacturing the preferred gold nanocrystalline suspensions.

Such flow of liquid 3' was obtained by utilizing a Masterflex® L/S pump drive 40 rated at 0.1 horsepower, 10-600rpm. The model number of the Masterflex® pump 40 was 7523-80. The pump drive had a pump head also made by Masterflex® known as Easy-Load Model No. 77201-60. In general terms, the head for the pump 40 is known as a peristaltic head. The precise settings on the pump were 215 milliliters per minute. Tygon® tubing having a diameter of 1/4" (i.e., size 06419-25) was placed into the peristaltic head. The tubing was made by Saint Gobain for Masterflex®. One end of the tubing was delivered to a first end 31 of the trough member 30'a.

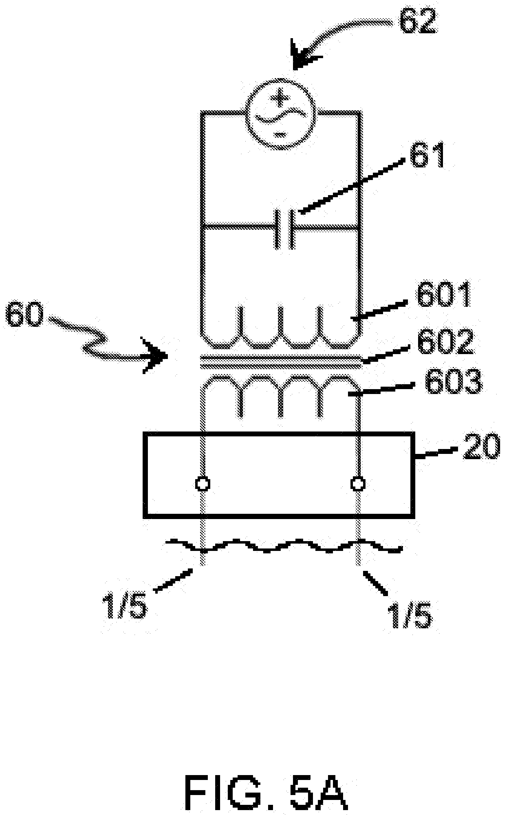

Table 1 shows that there was a single electrode set 1a/5a. The power source for each electrode set 1/5 was an AC transformer 60. Specifically, FIG. 5A shows a source of AC power 62 connected to a transformer 60. In addition, a capacitor 61 was provided so that, for example, loss factors in the circuit could be adjusted. The output of the transformer 60 was connected to the electrode(s) 1/5 through the control device 20. A preferred transformer for use with the device of Example 1 is one that uses alternating current flowing in a primary coil 601 to establish an alternating magnetic flux in a core 602 that easily conducts the flux.

When a secondary coil 603 is positioned near the primary coil 601 and core 602, this flux links the secondary coil 603 with the primary coil 601. This linking of the secondary coil 603 induces a voltage across the secondary terminals. The magnitude of the voltage at the secondary terminals is related directly to the ratio of the secondary coil turns to the primary coil turns. More turns on the secondary coil 603 than the primary coil 601 results in a step up in voltage, while fewer turns results in a step down in voltage.

Preferred transformer(s) 60 for use in the procedures described herein have deliberately poor output voltage regulation made possible by the use of magnetic shunts in the transformer 60. These transformers 60 are known as neon sign transformers. This configuration limits current flow into the electrode(s) 1/5. With a large change in output load voltage, the transformer 60 maintains output load current within a relatively narrow range.

The transformer 60 is rated for its secondary open circuit voltage and secondary short circuit current. Open circuit voltage (OCV) appears at the output terminals of the transformer 60 only when no electrical connection is present. Likewise, short circuit current is only drawn from the output terminals if a short is placed across those terminals (in which case the output voltage equals zero). However, when a load is connected across these same terminals, the output voltage of the transformer 60 should fall somewhere between zero and the rated OCV. In fact, if the transformer 60 is loaded properly, that voltage will be about half the rated OCV.

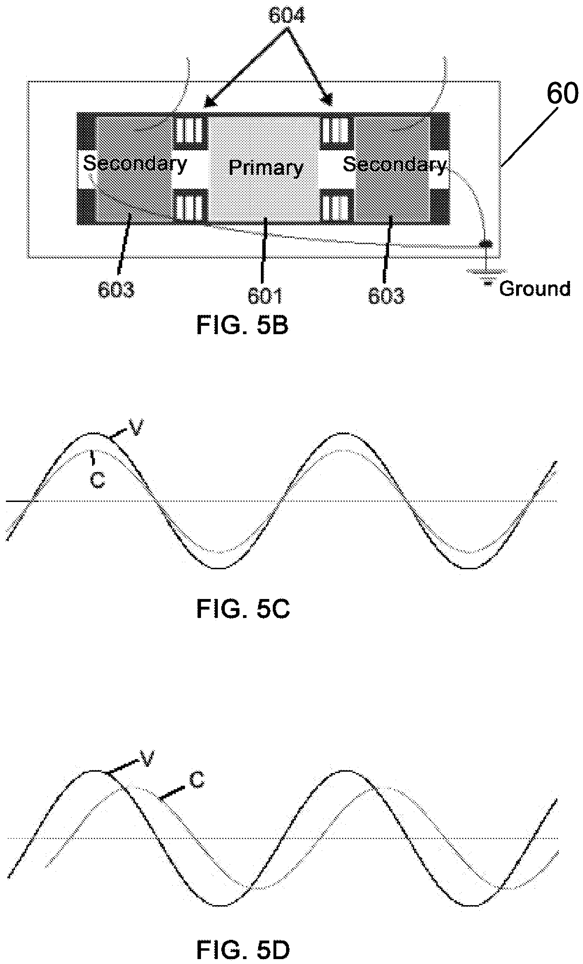

The transformer 60 is known as a Balanced Mid-Point Referenced Design (e.g., also formerly known as balanced midpoint grounded). This is most commonly found in mid to higher voltage rated transformers and most 60 mA transformers. This is the only type transformer acceptable in a "mid-point return wired" system. The "balanced" transformer 60 has one primary coil 601 with two secondary coils 603, one on each side of the primary coil 601 (as shown generally in the schematic view in FIG. 5B). This transformer 60 can in many ways perform like two transformers. Just as the unbalanced midpoint referenced core and coil, one end of each secondary coil 603 is attached to the core 602 and subsequently to the transformer enclosure and the other end of the each secondary coil 603 is attached to an output lead or terminal. Thus, with no connector present, an unloaded 15,000 volt transformer of this type, will measure about 7,500 volts from each secondary terminal to the transformer enclosure but will measure about 15,000 volts between the two output terminals.

In alternating current (AC) circuits possessing a line power factor of 1 (or 100%), the voltage and current each start at zero, rise to a crest, fall to zero, go to a negative crest and back up to zero. This completes one cycle of a typical sine wave. This happens 60 times per second in a typical US application. Thus, such a voltage or current has a characteristic "frequency" of 60 cycles per second (or 60 Hertz) power. Power factor relates to the position of the voltage waveform relative to the current waveform. When both waveforms pass through zero together and their crests are together, they are in phase and the power factor is 1, or 100%. FIG. 5C shows two waveforms "V" (voltage) and "C" (current) that are in phase with each other and have a power factor of 1 or 100%; whereas FIG. 5D shows two waveforms "V" (voltage) and "C" (current) that are out of phase with each other and have a power factor of about 60%; both waveforms do not pass through zero at the same time, etc. The waveforms are out of phase and their power factor is less than 100%.

The normal power factor of most such transformers 60 is largely due to the effect of the magnetic shunts 604 and the secondary coil 603, which effectively add an inductor into the output of the transformer's 60 circuit to limit current to the electrodes 1/5. The power factor can be increased to a higher power factor by the use of capacitor(s) 61 placed across the primary coil 601 of the transformer, 60 which brings the input voltage and current waves more into phase.

The unloaded voltage of any transformer 60 is important, as well as the internal structure thereof. Desirable unloaded transformers include those that are around 9,000 volts, 10,000 volts, 12,000 volts and 15,000 volts. However, these particular unloaded volt transformer measurements should not be viewed as limiting the scope acceptable power sources as additional embodiments. A specific desirable transformer for use in the procedures herein is made by Franceformer, Catalog No. 9060-P-E which operates at: primarily 120 volts, 60Hz; and secondary 9,000 volts, 60 mA.

Accordingly, the transformer 60 can be the same transformer, or can be a different transformer (as well as a different polarity). The choice of transformer, power factor, capacitor(s) 61, polarity, electrode designs, electrode location, electrode composition, cross-sectional shape(s) of the trough member 30a', local or global electrode composition, atmosphere(s), local or global liquid 3 flow rate(s), liquid 3' local components, volume of liquid 3' locally subjected to various fields in the trough member 30a', neighboring (e.g., both upstream and downstream) electrode sets, local field concentrations, the use and/or position and/or composition of any membrane used in the trough member, etc., are all factors which influence processing conditions as well as composition and/or volume of constituents produced in the liquid 3', nanocrystals and nanocrystal /suspensions or colloids made according to the various embodiments disclosed herein. Accordingly, a plethora of embodiments can be practiced according to the detailed disclosure presented herein.

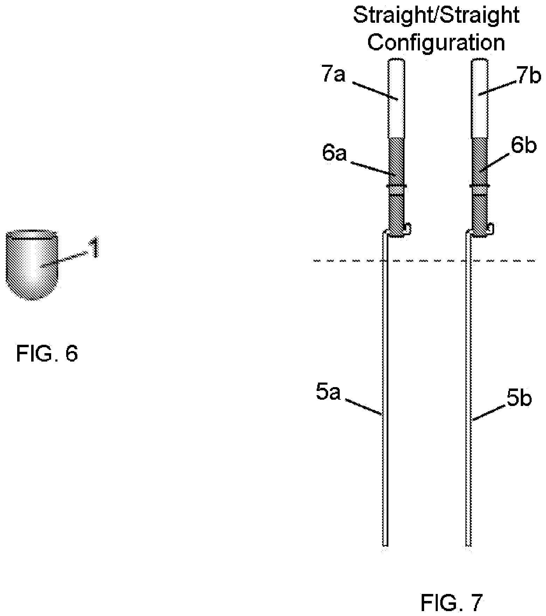

The plasma 4 was created with an electrode 1 similar in shape to that shown in FIG. 6, and weighed about 9.2 grams. This electrode was 99.995% (4N5) pure gold. The other electrode 5a measured about 1mm thick gold wire (99.995%) and having about 9mm submerged in the liquid 3'.

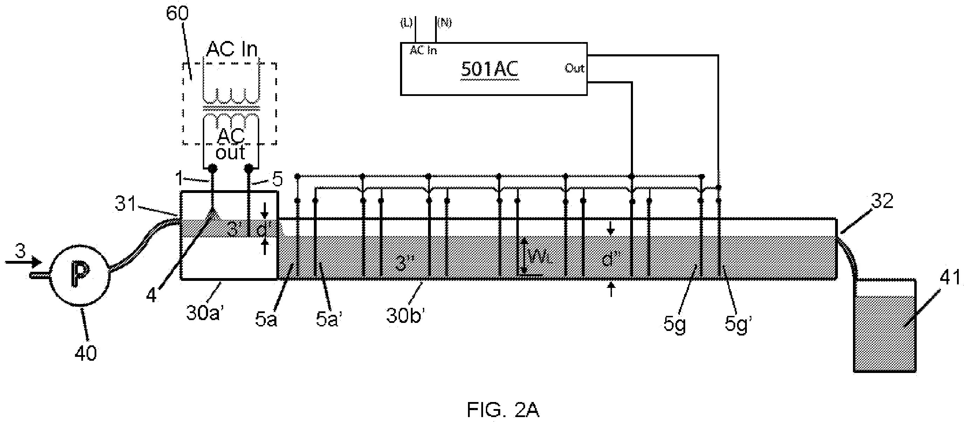

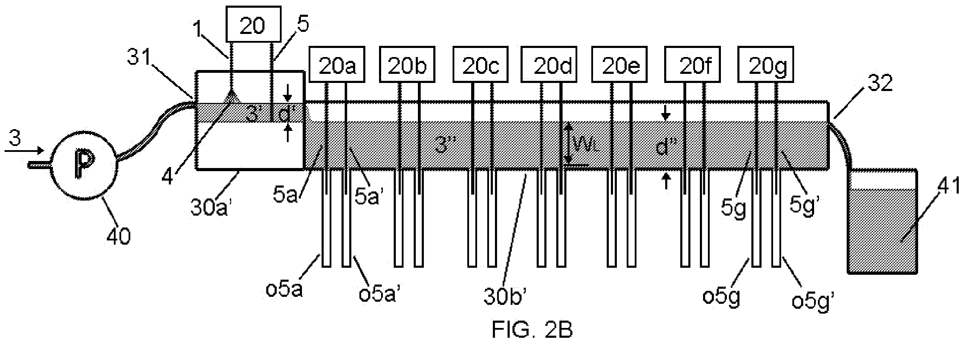

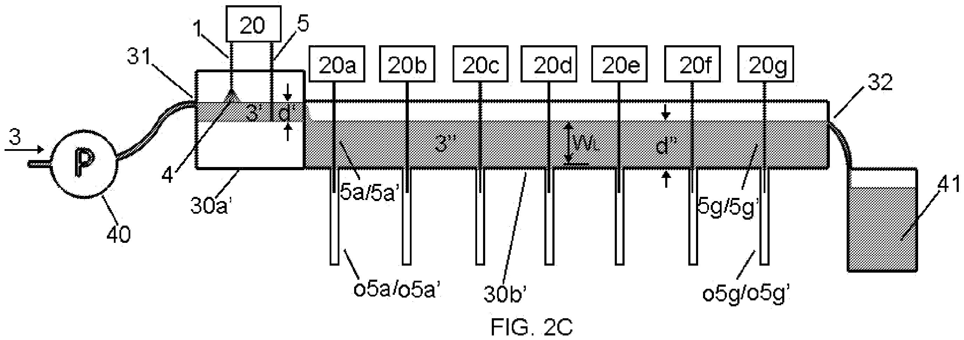

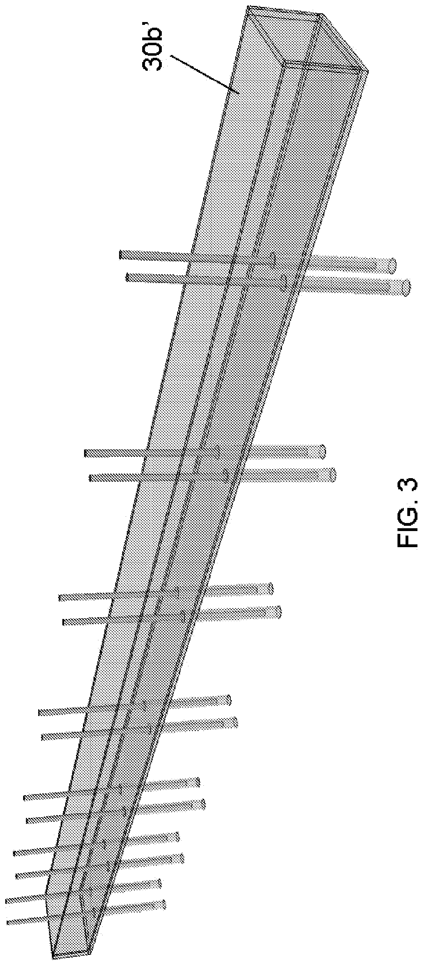

As shown in FIGs. 2A and 2C, the output from the trough member 30a' was the conditioned liquid 3' and this conditioned liquid 3' flowed directly into a second trough member 30b'. The second trough member 30b', shown in FIGs. 2A, 2C and 3 had measurements as reported in Table 1. This trough member 30b' contained about 885ml of liquid 3". Table 1 reports the electrode configuration, as shown in FIGs. 7 and 3, which means seven sets of electrodes 5/5' (shown in FIG. 7) were positioned as shown in FIG. 3 (i.e., perpendicular to the flow direction of the liquid 3"). Each of the electrode sets 5/5' comprised 99.99% pure gold wire measuring about 1.0mm in diameter, as reported in Table 1. The length of each wire electrode 5 that was in contact with the liquid 3" (reported as "$W_L$" in Table 1) measured about 1" (about 25.4mm). Other orientations fit within the metes and bounds of this disclosure.

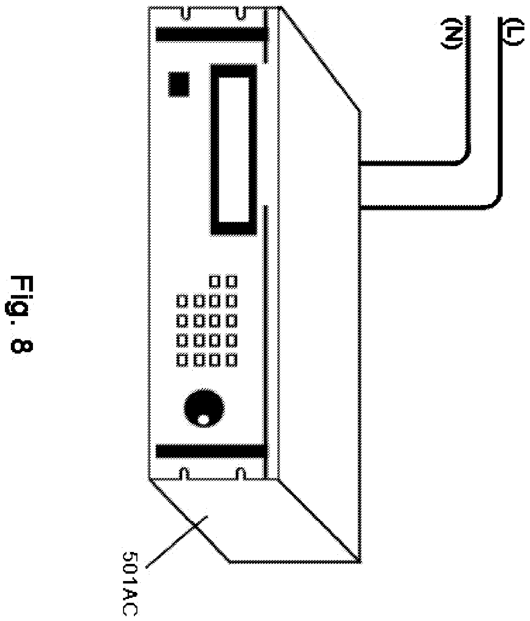

The AC power source (or transformer) 501AC, illustrated in FIG. 8, was used as the power supply. This transformer 501 AC was an AC power source (Chroma 61604) having an AC voltage range of 0-300V, a frequency range of 15-1000Hz and a maximum power rating of about 2kVA. With regard to FIGs. 2A, 2C and 3, each separate electrode set 5/5' (e.g., Set 2, Set 3 - Set 8 or Set 9) were electrically connected to the power supply 501AC as shown in FIG. 2A. Specifically, power supply 501AC was electrically connected to each electrode set, according to the wiring diagram show in FIG. 2A.

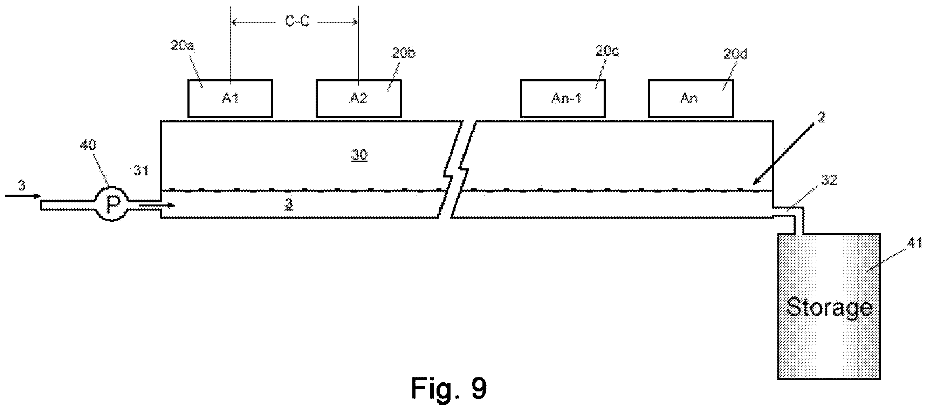

Table 1 refers to each of the electrode sets by "Set #" (e.g., "Set 1" through "Set 8"). Each electrode of the 1/5 or 5/5 electrode sets was set to operate at a specific voltage. The voltages listed in Table 1 are the voltages used for each electrode set. The distance "c-c" (with reference to FIG. 9) from the centerline of each electrode set to the adjacent electrode set is also reported. Further, the distance "x" associated with each electrode 1 utilized is also reported. For the electrode 5, no distance "x" is reported. Other relevant parameters are also reported in Table 1. All materials for the electrodes 1/5 were obtained from Hi-Rel having an address of 23 Lewis Street, Fort Erie, Ontario, Canada, L2A 2P6. With reference to FIGs. 2A, 2C and 3, each electrode 5/5' was first placed into contact with the liquid 3" such that it just entered the female receiver tube o5. After a certain amount of process time, gold metal was removed from each wire electrode 5 which caused the electrode 5 to thin (i.e., become smaller in diameter) which changed, for example, current density and/or the rate at which gold nanoparticles were formed. Accordingly, the electrodes 5 were moved toward the female receiver tubes o5 resulting in fresh and thicker electrodes 5 entering the liquid 3" at a top surface portion thereof. In essence, an erosion profile or tapering effect was formed on the electrodes 5 after some amount of processing time has passed (i.e., portions of the wire near the surface of the liquid 3" were typically thicker than portions near the female receiver tubes o5), and such wire electrode profile or tapering can remain essentially constant throughout a production process, if desired, resulting in essentially identical product being produced at any point in time after an initial pre-equilibrium phase during a production run allowing, for example, the process to be cGMP under current FDA guidelines and/or be ISO 9000 compliant as well.

The electrodes 5/5 were actuated or moved at a rate of about 1 inch per 8 hours. Samples were collected only from the equilibrium phase. The pre-equilibrium phase occurs because, for example, the concentration of nanocrystals produced in the liquid 3" increases as a function of time until the concentration reaches equilibrium conditions (e.g.,

substantially constant nucleation and growth conditions within the apparatus), which equilibrium conditions remain substantially constant through the remainder of the processing due to the control processes disclosed herein. The pre-equilibrium phase last about 30 minutes and produces about 1.7 gallons.

The eight electrode sets 1/5 and 5/5 were all connected to control devices 20 through 20g which automatically adjusted the height of, for example, each electrode 1/5 or 5/5 in each electrode set. Two female receiver tubes o5a/o5a' - o5g/o5g' were connected to a bottom portion of the trough member 30b' such that the electrodes in each electrode set 5/5 could be removably inserted into each female receiver tube o5 when, and if, desired. Each female receiver tube o5 was made of polycarbonate and had an inside diameter of about 1/8 inch (about 3.2mm) and was fixed in place by a solvent adhesive to the bottom portion of the trough member 30b'. Holes in the bottom of the trough member 30b' permitted the outside diameter of each tube o5 to be fixed therein such that one end of the tube o5 was flush with the surface of the bottom portion of the trough 30b'. The bottom portion of the tube o5 is sealed. The inside diameters of the tubes o5 effectively prevented any significant quantities of liquid 3" from entering into the female receiver tube o5. However, some liquid may flow into the inside of one or more of the female receiver tubes o5. The length or vertical height of each female receiver tube o5 was about 6 inches (about 15.24 cm) however, shorter or longer lengths fall within the metes and bounds of this disclosure. Further, while the female receiver tubes o5 are shown as being subsequently straight, such tubes could be curved in a J-shaped or U-shaped manner such that their openings away from the trough member 30b' could be above the top surface of the liquid 3," if desired.

The run described herein utilized the following processing enhancer. Specifically, about 2.04 grams/gallon (i.e., about 0.54 g/liter) of sodium hydrogen carbonate ("soda"), having a chemical formula of $NaHCO_3$, was added to and mixed with the water 3. The soda was obtained from Alfa Aesar and the soda had a formula weight of 84.01 and a density of about 2.159 $g/cm^3$.

In particular, a sine wave AC frequency at 60Hz was utilized to make the gold nanocrystal suspensions in accordance with the teachings herein. The AC power source 501AC utilized a Chroma 61604 programmable AC source. The applied voltage was about 220 volts. The applied current was between about 6 amps and about 6.5 amps.

Table 1 summarizes key processing parameters used in conjunction with FIGs. 1, 2A and 2C. Also, Table 1 discloses: 1) "Produced Au PPM" (e.g., gold nanocrystal concentrations); 2) "TEM Average Diameter" which is the mode, corresponding to the gold nanocrystal diameter that occurs most frequently, determined by the TEM analysis; and 3) "Hydrodynamic radius" as measured by the Zetasizer ZS-90. These physical characterizations were performed as discussed elsewhere herein.

**Transmission Electron Microscopy**

Specifically, TEM samples were prepared by utilizing a Formvar coated grid stabilized with carbon having a mesh size of 200. The grids were first pretreated by a plasma treatment under vacuum. The grids were placed on a microscope slide lined with a rectangular piece of filter paper and then placed into a Denton Vacuum apparatus with the necessary plasma generator accessory installed. The vacuum was maintained at 75 mTorr and the plasma was initiated and run for about 30 seconds. Upon completion, the system was vented and the grids removed. The grids were stable up to 7-10 days depending upon humidity conditions, but in all instances were used within 12 hours.

Approximately 1 μL of the CNM-Au8 nanocrystal suspension was placed onto grids and was allowed to air dry at room temperature for 20-30 minutes, or until the droplet evaporated. Upon complete evaporation, the grids were placed onto a holder plate until TEM analysis was performed.

A Philips/FEI Tecnai 12 Transmission Electron Microscope was used to interrogate all prepared samples. The instrument was run at an accelerating voltage of 100keV. After alignment of the beam, the samples were examined at various magnifications up to and including 630,000x. Images were collected via the attached Olympus Megaview III side-mounted camera that transmitted the images directly to a PC equipped with iTEM and Tecnai User Interface software which provided for both control over the camera and the TEM instrument, respectively.

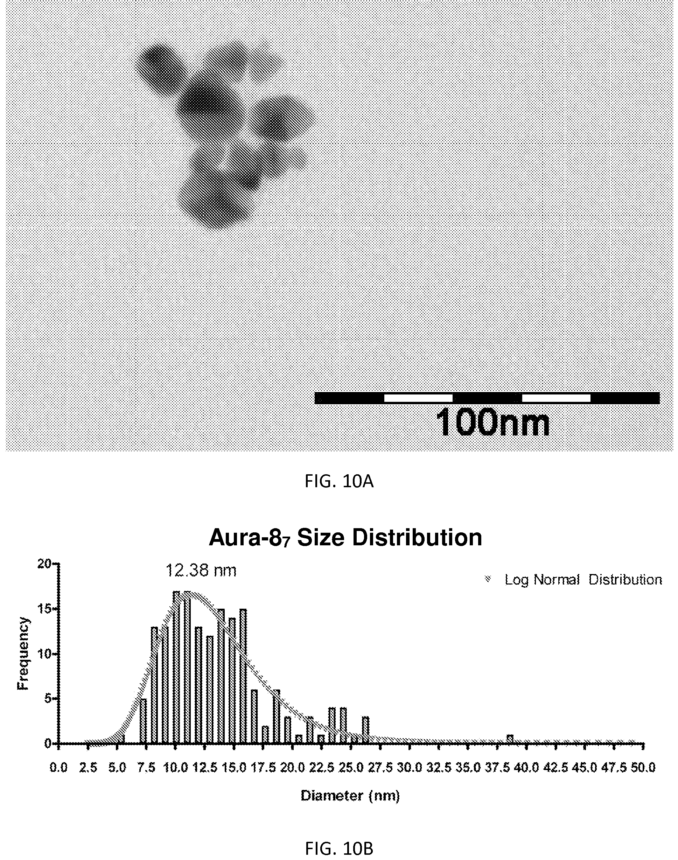

FIG. 10A shows a representative TEM photomicrograph of gold nanocrystals corresponding to a dried CNM-Au8 suspension, made according to the procedures above herein. FIG. 10B corresponds to the measured TEM size distribution used to calculate the TEM average diameter and referenced in Table 1.

**pH Measurements**

The pH measurements were made by using an Accumet® AR20 pH/conductivity meter wherein the pH probe was placed into a 50mL vial containing the samples of interest and allowed to stabilize. Three separate pH measurements were then taken and averaged per sample. The CNM-Au8 nanosuspension had a measured pH of about 9.08.

**UV-VIS Spectroscopy**

Energy absorption spectra were obtained for the samples by using UV-VIS spectroscopy. This information was acquired using a Thermofisher Evolution 201 UV-VIS spectrometer equipped with a double beam Czerny-Turner monochromator system and dual silicon photodiodes. Instrumentation was provided to support measurement of low-concentration liquid samples using one of a number of fused-quartz sample holders or "cuvettes." Data was acquired over the wavelength range between about 300-900nm with the following parameters: bandwidth of 1nm, data pitch of 0.5nm. A xenon flash lamp was the primary energy source. The optical pathway of the spectrometer was arranged to allow the energy beam to pass through the center of each sample cuvette. Sample preparation was limited to filling and capping the cuvettes and then physically placing the samples into the cuvette holder, within the fully enclosed sample compartment of the spectrometer. Optical absorption of energy of each sample was determined. Data output was measured and displayed as Absorbance Units (per Beer-Lambert's Law) versus wavelength.

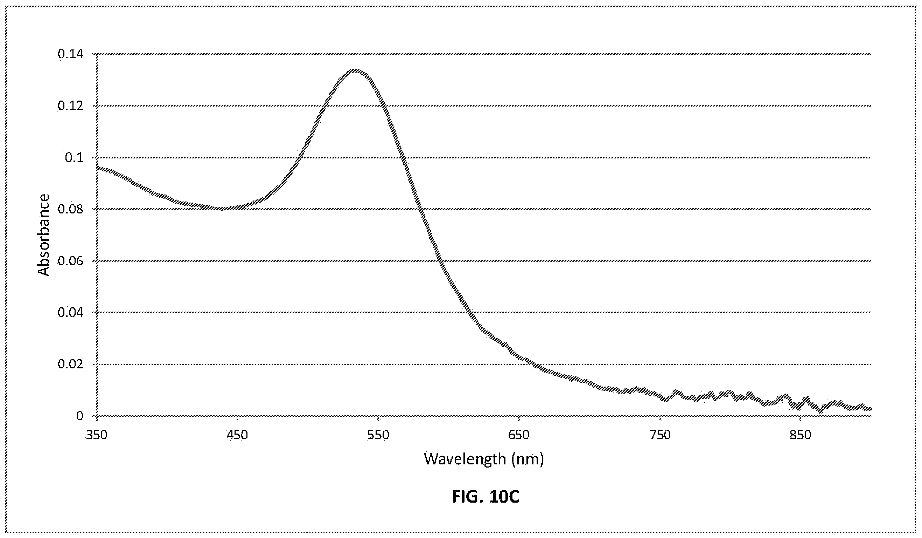

FIG. 10C shows UV-Vis spectral patterns for the CNM-Au8 suspension, for the wavelength range of about 350nm-900nm.

**Dynamic Light Scattering Zetasizer**

Dynamic light scattering (DLS) measurements of the CNM-Au8 suspension were performed on Zetasizer Nano ZS-90 DLS instrument. In DLS, as the laser light hits small particles and/or organized water structures around the nanocrystals (smaller than the wavelength), the light scatters in all directions, resulting in a time-dependent fluctuation in the scattering intensity. Intensity fluctuations are due to the Brownian motion of the scattering particles/water structure combination and contain information about the crystal size distribution.

The instrument was allowed to warm up for at least 30 min prior to the experiments. The measurements were made using square glass cell with 1cm path length, PCS8501. The following procedure was used:

1. First, 1ml of DI water was added into the cell using 1ml micropipette, then water was poured out of the cell to a waste beaker and the rest of the water was shaken off the cell measuring cavity. This step was repeated two more times to thoroughly rinse the cell.
2. 1ml of the sample was added into the cell using 1ml micropipette. After that all liquid was removed out of the cell with the same pipette using the same pipette tip and expelled into the waste beaker. 1ml of the sample was added again using the same tip.
3. The cell with the sample was placed into a temperature controlled cell block of the Zetasizer instrument with engraved letter facing forward. A new experiment in Zetasizer software was opened. The measurement was started 1min after the temperature equilibrated and the laser power attenuated to the proper value. The results were saved after all runs were over.
4. The cell was taken out of the instrument and the sample was removed out of the cell using the same pipette and the tip used if step 2.
5. Steps 2 to 4 were repeated two more times for each sample.
6. For a new sample, a new pipette tip for 1ml pipette was taken to avoid contamination with previous sample and steps 1 through 5 were repeated.

Data collection and processing was performed with Zetasizor software, version 6.20. The following parameters were used for all the experiments: Run Duration - 2o; Experiments - 10; Solvent - water, 0 mmol; Viscosity - 0.8872 cP; Refractive Index - 1.333; block temperature - +25°C. After data for each experiment were saved, the results were viewed on "Records View" page of the software. Particle size distribution (i.e., hydrodynamic radii) was analyzed in "Intensity PSD" graph. Dynamic light scattering techniques were utilized to obtain an indication of crystal sizes (e.g., hydrodynamic radii) produced according to this procedure. Hydrodynamic radius is reported in Table 1 as 19.43nm. Further, the measured zeta potential for the neat CNM-Au8 nanosuspension was -42.9mV.

**Atomic Absorption Spectroscopy**

The AAS values were obtained from a Perkin Elmer AAnalyst 400 Spectrometer system. Atomic absorption spectroscopy is used to determine concentration of species, reported in "ppm" (parts per million).

**I) Principle**

The technique of flame atomic absorption spectroscopy requires a liquid sample to be aspirated, aerosolized and mixed with combustible gases, such as acetylene and air. The mixture is ignited in a flame whose temperature ranges from about 2100 to about 2400 degrees C. During combustion, atoms of the element of interest in the sample are reduced to free, unexcited ground state atoms, which absorb light at characteristic wavelengths. The characteristic wavelengths are element specific and are accurate to 0.01 - 0.1nm. To provide element specific wavelengths, a light beam from a hollow cathode lamp (HCL), whose cathode is made of the element being determined, is passed through the flame. A photodetector detects the amount of reduction of the light intensity due to absorption by the analyte. A monochromator is used in front of the photodetector to reduce background ambient light and to select the specific wavelength from the HCL required for detection. In addition, a deuterium arc lamp corrects for background absorbance caused by non-atomic species in the atom cloud.

**II) Sample preparation**

10mL of sample, 0.6mL of 36%v/v hydrochloric acid and 0.15mL of 50%v/v nitric acid are mixed together in a glass vial and incubated for about 10 minutes in 70 degree C water bath. If gold concentration in the suspension is expected to be above 10ppm a sample is diluted with DI water before addition of the acids to bring final gold concentration in the range of 1 to 10ppm. For example, for a gold concentration around 100ppm, 0.5mL of sample is diluted with 9.5mL of DI water before the addition of acids. Aliquoting is performed with adjustable micropipettes and the exact amount of sample, DI water and acids is measured by an Ohaus PA313 microbalance. The weights of components are used to correct measured concentration for dilution by DI water and acids.

Each sample is prepared in triplicate and after incubation in water bath is allowed to cool down to room temperature before measurements are made.

**III) Instrument Setup**

The following settings are used for Perkin Elmer AAnalyst 400 Spectrometer system:

   **a) Burner head:** 10cm single-slot type, aligned in three axes according to the manufacture procedure to obtain maximum absorbance with a 2ppm Cu standard.
   **b) Nebulizer:** plastic with a spacer in front of the impact bead.
   **c) Gas flow:** oxidant (air) flow rate about 12 L/min, fuel (acetylene) flow rate about 1.9 mL/min.
   **d) Lamp/monochromator:** Au hollow cathode lamp, 10mA operating current, 1.8/1.35mm slits, 242.8nm wavelength, background correction (deuterium lamp) is on.

**IV) Analysis procedure**

   a) Run the Au lamp and the flame for approximately 30 minutes to warm up the system.
   b) Calibrate the instrument with 1ppm, 4ppm and 10ppm Au standards in a matrix of 3.7%v/v hydrochloric acid. Use 3.7%v/v hydrochloric acid as a blank.
   c) Verify calibration scale by measuring 4ppm standard as a sample. The measured concentration should be between 3.88ppm and 4.12ppm. Repeat step b) if outside that range.
   d) Measure three replicas of a sample. If the standard deviation between replicas is higher than 5%, repeat measurement, otherwise proceed to the next sample.
   e) Perform verification step c) after measuring six samples or more often. If verification fails, perform steps b) and c) and remeasure all the samples measured after the last successful verification.

**V) Data analysis**

Measured concentration value for each replica is corrected for dilution by water and acid to calculate actual sample concentration. The reported Au ppm value is the average of three corrected values for individual replica.

Table 1 references the AAS concentration result as "Produced Au PPM", with a corresponding value of about 7.2ppm.

**Tangential Flow Filtration (TFF)**

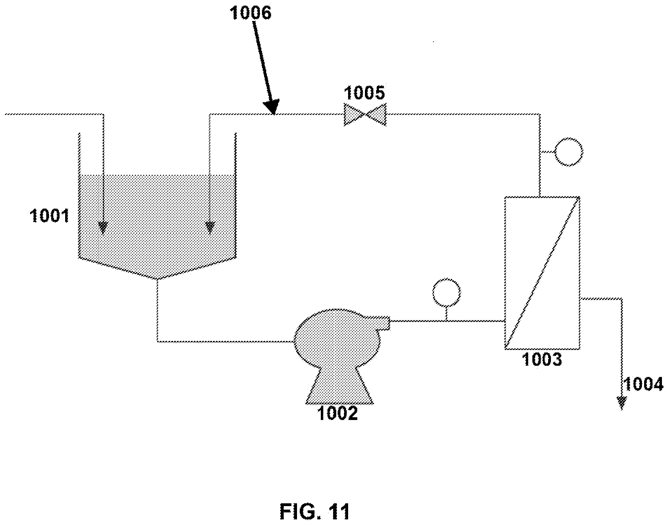

In order to increase the concentration of gold nanocrystals produced in the neat CNM-Au8 nanosuspension for use in Examples 2 and 3, a tangential flow filtration (TFF) process was utilized. Concentration in the TFF process is a pressure driven separation process that uses membranes to remove preferentially liquid comprising the suspension from the nanocrystals in the suspension. Thus, the TFF process results in a relatively higher concentration of gold nanocrystals in the liquid on one side of the membrane. In the TFF process, the CNM-Au8 suspension is pumped tangentially along the surface of the membrane. A schematic of a simple TFF system is shown in FIG. 11.

A feed tank 1001 provided CNM-Au8 suspension to a feed pump 1002 and into a filtration module 1003. The filtrate stream 1004 was discarded. Retentate was diverted through the retentate valve 1005 and returned as 1006 into the feed tank 1001. During each pass of the suspension over the surface of the membrane in the filtration module 1003, the applied pressure forced a portion of the liquid comprising the suspension through the membrane and into the filtrate stream, 1004. The gold nanocrystals are too large to pass through the membrane and are thus retained on the upper stream and swept along by the tangential flow into the retentate, 1006. The retentate, having a higher concentration of gold nanocrystals, was returned back to the feed tank, 1001. If there is no diafiltration buffer added to the feed tank, then the liquid volume in the feed tank, 1001, was reduced by the amount of filtrate (i.e., liquid) removed and the gold nanocrystals were concentrated in the suspension.

In this example, Millipore Pellicon XL cassettes were used with 5kDa and 10kDa MWCO cellulose membranes. The retentate pressure was set to 40 PSI by a retentate valve, 1005. 10kDa membrane allowed approximately 4 times higher filtrate flow rate relative to a 5kDa membrane under the same transmembrane pressure, which is expected for a larger pore size. At the same time, pores of 10kDa membrane are small enough to retain all formed gold nanocrystals in the retentate thereby concentrating the gold nanocrystals in the CNM-Au8 suspension. After passing the CNM-Au8 suspension through the TFF system, a desired concentration of suspended gold nanocrystals in the CNM-Au8 suspension was achieved and increased from about 7.2 ppm to about 51 ppm (for Example 2); and the concentration of suspended gold nanocrystals in the CNM-Au8 suspension was increased from about 7.2 ppm to about 50 ppm and 1000ppm (for two different nanosuspensions used in Example 3).

The concentrated CNM-Au8 nanocrystal suspension was characterized for both hydrodynamic radius and zeta potential to determine if the concentration step affected either value. For the Example 2 suspensions, the measured zeta potential of the concentrated CNM-Au8 (51ppm) nanosuspension was about -45.5mV and the measured hydrodynamic radius was about 18 nm. For the Example 3 suspensions, the measured zeta potential of the concentrated CNM-Au8 (50ppm) nanosuspension was about -43.6mV and the measured hydrodynamic radius was about 18.6 nm; and the measured zeta potential of the concentrated CNM-Au8 (1000ppm) nanosuspension was about -47.2mV and the measured hydrodynamic radius was about 20.1 nm. Accordingly, the concentration step did not adversely affect either of these important physical characterization parameters.

Detailed production methods and detailed physical characterization of neat CNM-Au8 suspensions can be found in International Application PCT/US2010/041427, published on October 3, 2013, and entitled, Novel Gold-Based Nanocrystals for Medical Treatments and Electrochemical Manufacturing Processes Therefor.

**Methods for Using Gold (Preferably CNM-Au8) Nanosuspensions as Treatments**

Described are methods for preventing demyelination or dysmyelination and/or promoting myelination or remyelination of neurons including CNS neurons and/or PNS neurons. Elemental gold crystal nanosuspensions may relieve the inhibition of CNS myelination and/or promote CNS neuronal cell survival and/or decrease or inhibit expression of some antagonist. Preferably, the CNM-Au8 nanocrystalline suspensions of Example 1 are used with such methods.

Also described is a method of promoting myelination of neurons (including CNS neurons) in a mammal comprising administering to a mammal, in need thereof, an effective amount (either therapeutic or prophylactic) of a composition comprising an elemental gold crystal nanosuspension. Preferably, the CNM-Au8 nanocrystalline suspensions of Example 1 are used with such methods.

Also described are methods for treating a disease, disorder, pathological state and/or an injury associated with dysmyelination or demyelination, in an animal (e.g. a mammal) suffering from such condition, the method comprising, consisting essentially of, or consisting of administering to the mammal in need thereof a therapeutically effective amount of a gold crystal nanosuspension, and preferably, the CNM-Au8 nanocrystalline suspensions of Example 1 are used.

Also described are methods for promoting survival of CNS neurons and/or PNS neurons in a mammal in need thereof comprising administering an effective amount of a gold crystal nanosuspension. Preferably, the CNM-Au8 nanocrystalline suspensions of Example 1 are used with such methods.

Also described are methods for promoting oligodendrocyte differentiation in a mammal, comprising administering to a mammal in need thereof an effective amount of a composition comprising a gold crystal nanosuspension. Preferably, the CNM-Au8 nanocrystalline suspensions of Example 1 are used with such methods.

Also described are methods for decreasing or inhibiting expression of damaged myelin, demyelination or dysmyelination relative to the absence of providing an effective amount of a gold nanocrystalline suspension, comprising modifying the myelination of neurons (CNS and/or PNS) with a composition comprising an effective amount (both prophylactic and therapeutic) of a gold nanocrystalline suspension. Preferably, the CNM-Au8 nanocrystalline suspensions of Example 1 are used with such methods.

Also described are methods for inhibiting or decreasing undesirable pathological events associated with myelin damage in the absence of an effective amount of the CNM-Au8 nanocrystalline suspension of Example 1 being provided, comprising providing a subject in need thereof a composition comprising a CNM-Au8 nanosuspension.

In such treatment methods nanocrystalline suspensions, preferably CNM-Au8 nanocrystalline suspensions, can be administered via oral administration, injections and/or nasally.

Preferably, a CNM-Au8 nanosuspension may administered by bolus injection or chronic infusion. A CNM-Au8 nanosuspension may be administered directly into the central nervous system by, for example, intrathecal or epidural placement. A CNM-Au8 nanosuspension may be administered directly into a chronic lesion where myelin damage is expressed. A CNM-Au8 nanosuspension may be administered directly into the bloodstream of a mammal.

The gold nanosuspensions, and preferably the CNM-Au8 nonocrystalline suspensions, is/are preferably administered as a treatment for a disease that includes Progressive Supranuclear Palsy, Alexander's Disease, Krabbe Disease, Metachromatic Leukodystrophy, Canvan Disease, Leukodistrophies, Encephalomyelitis, Central Pontine Myelolysis (CPM), Anti-MAG Disease, Pelizaeus-Merzbacher Disease, Refsum Disease, Cockayne Syndrome, , Zellweger Syndrome, Guillain-Barre Syndrome (GBS), Van der Knapp Syndrome, chronic inflammatory demyelinating polyneuropathy (CIDP), multifocal motor neuropathy (MMN), Neuromyelitis Optica (NMO), Progressive Multifocal Leukoencephalopathy (PML), Wallerian Degeneration and some inherited diseases such as Adrenoleukodystrophy, Alexander's Disease, Mild Cognitive Impairment (MCI) also known as Age Related Cognitive Decline and Pelizaeus Merzbacher Disease (PMZ). A gold nanocrystalline suspension can be prepared and used as a therapeutic agent that stops, reduces, prevents, or inhibits the ability of damaging events leading to dysmyelination, demyelination and/or those events that negatively regulate myelination and/or neuronal survival and/or increase the expression of good myelin (e.g., remyelination) or good myelin/axon interactions.

Described are methods for treating, in a subject, a disease, disorder or injury associated with dysmyelination or demyelination (e.g., neuromyelitis optica in a subject suffering from such disease) the method comprising, consisting essentially of, or consisting of administering to the subject a therapeutically effective amount of a gold nanosuspension, and in a preferred embodiment a CNM-Au8 nanosuspension, by titration to clinical effect by varying concentration, volume and/or dosing frequency.

Also described is a method for promoting myelination of neurons (including remyelination) in a mammal comprising, consisting essentially of, or consisting of administering an effective amount (both therapeutic and prophylactic) of a gold nanosuspension, and preferably a CNM-Au8 nanosuspension.

Also described are methods for treating a disease, disorder or injury associated with oligodendrocyte death or lack of differentiation, e.g., neuromyelitis optica, Pelizaeus Merzbacher disease or globoid cell leukodystrophy (Krabbe's disease), in an animal suffering from such disease, the method comprising, consisting essentially of, or consisting of administering to the animal an effective amount of a gold nanosuspension, and preferably, a CNM-Au8 nanosuspension.

Also described is a method for promoting proliferation, differentiation and survival of oligodendrocytes in a mammal comprising, consisting essentially of, or consisting of administering a therapeutically effective amount of a gold nanosuspension, and preferably, a CNM-Au8 nanosuspension.

A gold nanosuspension, and preferably, a CNM-Au8 nanosuspension, to be used in the treatment methods disclosed herein, can be prepared and used as a therapeutic agent that stops, reduces, prevents, or inhibits the ability of pathological events that negatively regulate myelination of neurons by oligodendrocytes. Additionally, a gold nanosuspension, and preferably a CNM-Au8 nanosuspension, to be used in treatment methods disclosed herein, can be prepared and used as a therapeutic agent that stops, reduces, prevents, or inhibits the ability of pathologic events to negatively regulate oligodendrocyte differentiation, proliferation and survival.

Also described is a method of inducing oligodendrocyte proliferation or survival to treat a disease, disorder or injury involving the destruction of oligodendrocytes or myelin comprising delivering to a mammal, at or near the site of the disease, disorder or injury, gold nanocrystals from a CNM-Au8 nanosuspension in an amount sufficient to reduce inhibition of axonal extension and/or promote myelination and/or ameliorate demyelination.

In such treatment methods, the gold nanosuspensions can be administered via oral administration, injections and/or nasally.

A gold nanosuspension, and preferably a CNM-Au8 nanosuspension, may administered by bolus injection or chronic infusion. A CNM-Au8 nanosuspension may be administered directly into the central nervous system by, for example, intrathecal or epidural placement. A CNM-Au8 nanosuspension may be administered directly into a chronic lesion where myelin damage is expressed. A CNM-Au8 nanosuspension may be administered directly into the bloodstream of a mammal

Diseases or disorders which may be treated or ameliorated by the described methods include diseases, disorders or injuries which relate to dysmyelination or demyelination of mammalian neurons. Specifically, such diseases and disorders include those in which the myelin which surrounds the neuron is either absent, incomplete, not formed properly or is deteriorating. Such diseases include, but are not limited to, Progressive Supranuclear Palsy, Alexander's Disease, Krabbe Disease, Metachromatic Leukodystrophy, Canvan Disease, Leukodistrophies, Encephalomyelitis, Central Pontine Myelolysis (CPM), Anti-MAG Disease, Pelizaeus-Merzbacher Disease, Refsum Disease, Cockayne Syndrome, , Zellweger Syndrome, Guillain-Barre Syndrome (GBS), Van der Knapp Syndrome, chronic inflammatory demyelinating polyneuropathy (CIDP), multifocal motor neuropathy (MMN), Neuromyelitis Optica (NMO), Progressive Multifocal Leukoencephalopathy (PML), Mild Cognitive Impairment (MCI) also known as Age Related Cognitive Decline, Wallerian Degeneration and some inherited diseases such as Adrenoleukodystrophy, Alexander's Disease, and Pelizaeus Merzbacher Disease (PMZ).

Diseases or disorders which may be treated or ameliorated by the described methods include diseases, disorders or injuries which relate to the death or lack of proliferation or differentiation of oligodendrocytes. Such disease include, but are not limited to, Progressive Supranuclear Palsy, Alexander's Disease, Krabbe Disease, Metachromatic Leukodystrophy, Canvan Disease, Leukodistrophies, Encephalomyelitis, Central Pontine Myelolysis (CPM), Anti-MAG Disease, Pelizaeus-Merzbacher Disease, Refsum Disease, Cockayne Syndrome, , Zellweger Syndrome, Guillain-Barre Syndrome (GBS), Van der Knapp Syndrome, chronic inflammatory demyelinating polyneuropathy (CIDP), multifocal motor neuropathy (MMN), Neuromyelitis Optica (NMO), Progressive Multifocal Leukoencephalopathy (PML), Mild Cognitive Impairment (MCI) also known as Age Related Cognitive Decline, Wallerian Degeneration and some inherited diseases such as Adrenoleukodystrophy, Alexander's Disease, and Pelizaeus Merzbacher Disease (PMZ)..

Diseases or disorders which may be treated or ameliorated by the described methods include neurodegenerate disease or disorders. Such diseases include, but are not limited to, Progressive Supranuclear Palsy, Alexander's Disease, Krabbe Disease, Metachromatic Leukodystrophy, Canvan Disease, Leukodistrophies, Encephalomyelitis, Central Pontine Myelolysis (CPM), Anti-MAG Disease, Pelizaeus-Merzbacher Disease, Refsum Disease, Cockayne Syndrome, , Zellweger Syndrome, Guillain-Barre Syndrome (GBS), Van der Knapp Syndrome, Mild Cognitive Impairment (MCI) also known as Age Related Cognitive Decline, chronic inflammatory demyelinating polyneuropathy (CIDP), multifocal motor neuropathy (MMN), Neuromyelitis Optica (NMO), Progressive Multifocal Leukoencephalopathy (PML), Wallerian Degeneration and some inherited diseases such as Adrenoleukodystrophy, Alexander's Disease, and Pelizaeus Merzbacher Disease (PMZ).

Examples of additional diseases, disorders or injuries which may be treated or ameliorated by the described methods include, but are not limited, to spinal cord injuries, chronic myelopathy or rediculopathy, traumatic brain injury, motor neuron disease, axonal shearing, contusions, paralysis, post radiation damage or other neurological complications of chemotherapy, stroke, large lacunes, medium to large vessel occlusions, leukoariaosis, acute ischemic optic neuropathy, vitamin E deficiency (isolated deficiency syndrome, AR, Bassen-Komzweig syndrome), B12, B6 (pyridoxine-pellagra), thiamine, folate, nicotinic acid deficiency, Marchiafava-Bignami syndrome, Metachromatic Leukodystrophy, Trigeminal neuralgia, Bell's palsy, or any neural injury which would require axonal regeneration, remyelination or oligodendrocyte survival or differentiation/proliferation.

## EXAMPLE 2

### Cuprizone Demyelination Model - 16 Mouse Pilot Study

The goal of this pilot study was to determine if "CNM-Au8" nanocrystalline suspensions concentrated to 51ppm and consumed *ad libitum* might influence the amount or degree of myelin sheath damage (or repair) which typically occurs during cuprizone-induced demyelination of neurons in a mouse brain. The Cuprizone mouse model is intended to simulate myelin sheath damage in mammals for multiple diseases that express themselves pathologically as demyelination or dysmyelination.

A total of 16 C57BL6 male mice were separated into 4 groups (four per group), as shown in Table 2. Two extra mice were used as a backup and were not needed in the study. The mice were 8 weeks old at the start of the study.

In an attempt to induce demyelination by introducing toxic cuprizone, and observe possible reduction of demyelination and/or the promotion of remyelination by treatment with gold nanosuspensions, two of the four groups were fed Cuprizone Feed for 5 weeks. CNM-Au8 nanosuspensions (gold nanocrystal concentration of 51 ppm) were provided *ad libitum* (as the only drinking liquid for the mice) for all mice in Groups 3 and 4 (both treatment and control), as shown in Table 2. All mice were observed during the study and any abnormal behaviors would be recorded.

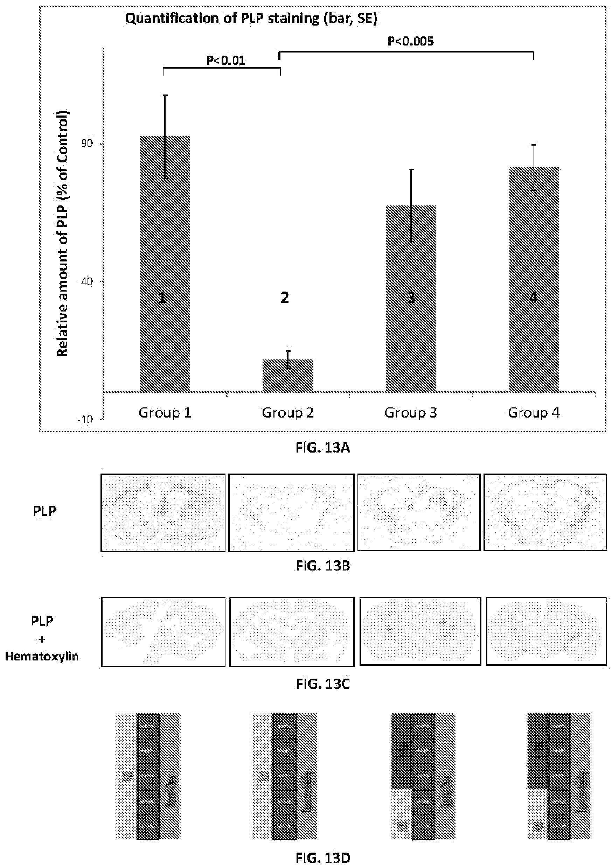

All mice were euthanized after 5 weeks of the study, as shown in Table 2. In each of the four groups, a predetermined area of the brain from three of the four mice was fixed for immunostaining (e.g., to determine the relative amount of myelin present). Specifically, the coronal area from bregma-0.82 mm to bregma-1.82 mm is the area of primary interest in this animal model.[1,2] These portions of the brain for three of the four mice in each group were stained for the presence of myelin proteolipid protein ("PLP"), as discussed in the literature.[3] The staining results are shown in FIGs. 13A- 13C. The brain from the fourth mouse in each group was processed differently and specifically prepared for transmission electron microscopy ("TEM") investigations.

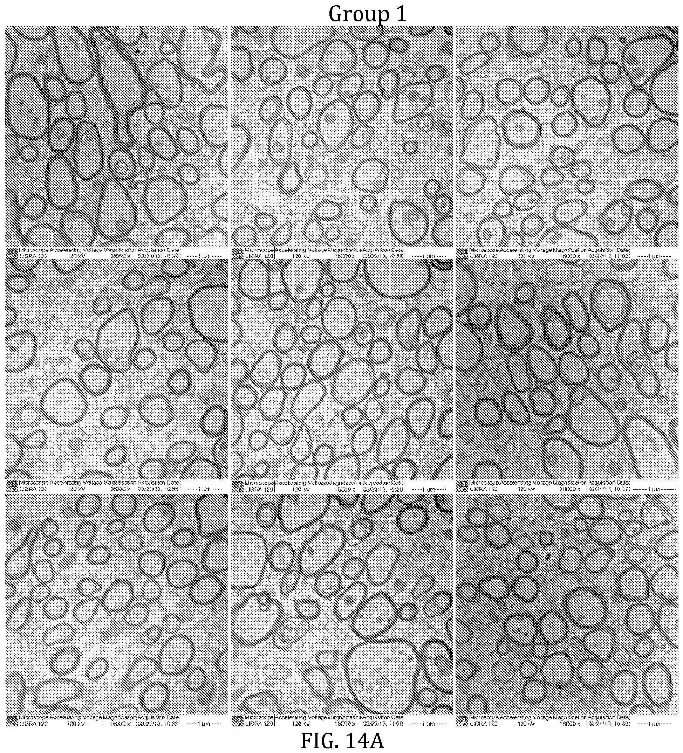

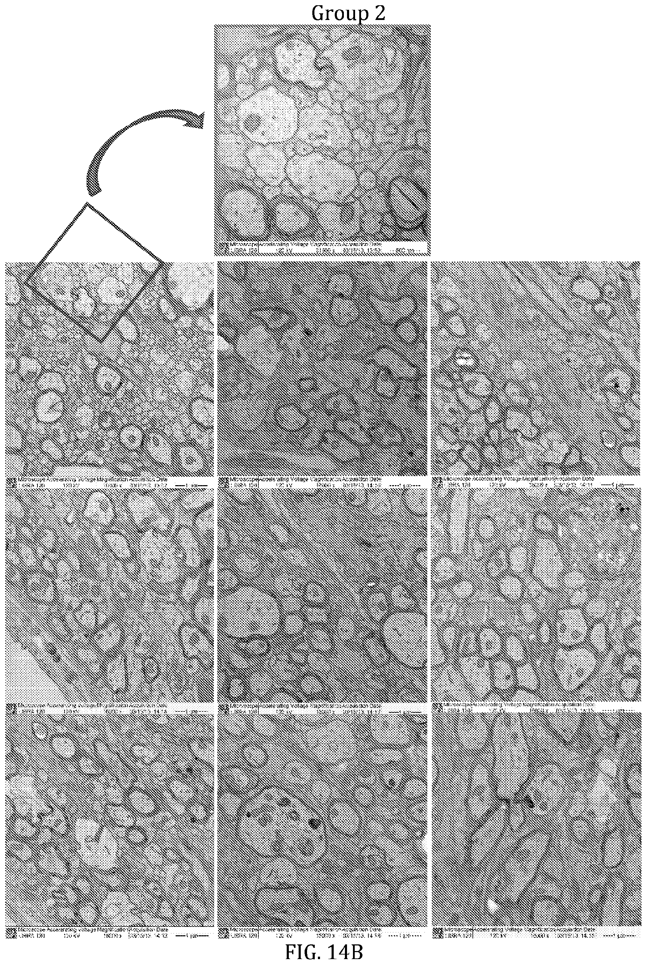

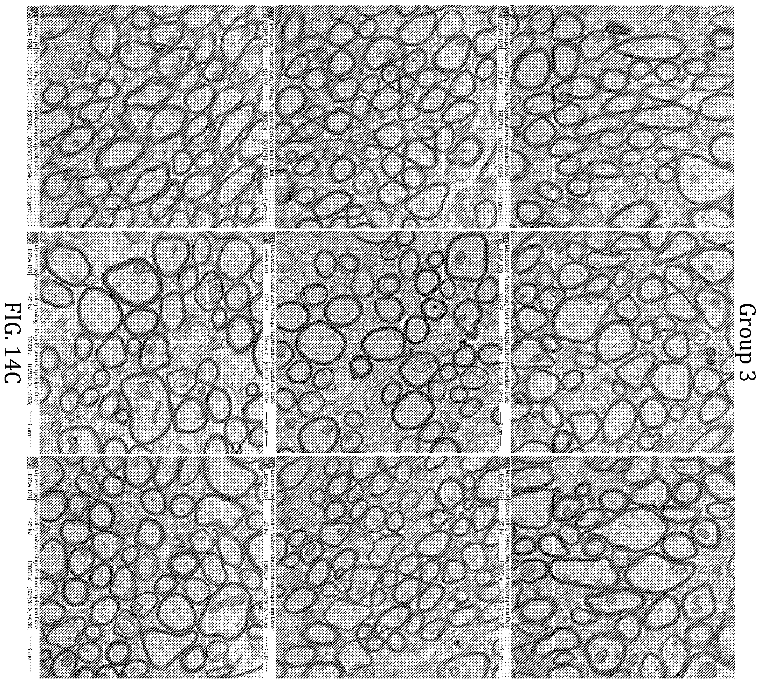

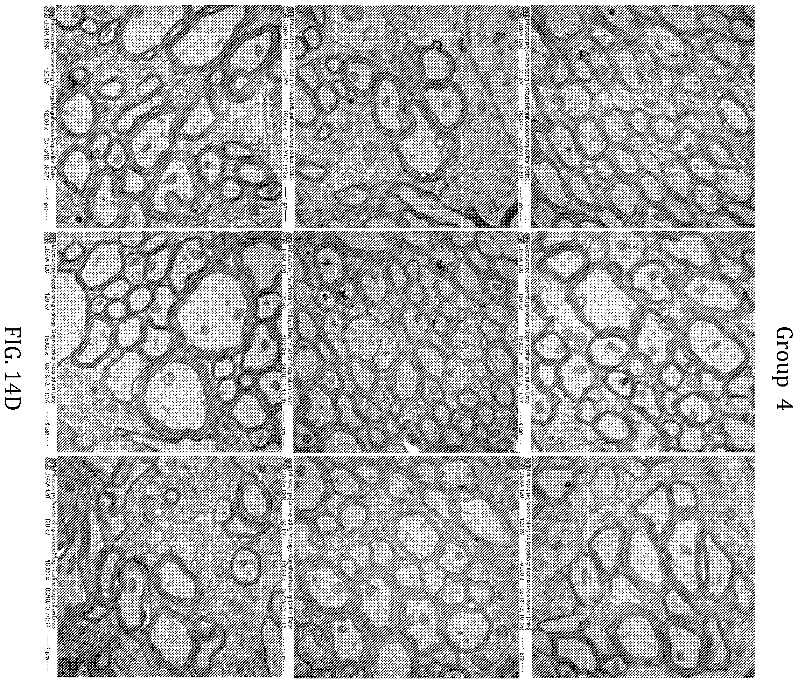

The corpus callosum region of the brain is heavily populated with axons and this region was the area of focus for the TEM studies. At least nine representative TEM images of the axon/myelin sheaths taken from the corpus callosum region are shown in FIGs. 14A-14D for a single mouse from each of Groups 1-4, respectively. At least nine TEM images are provided in each of FIGs. 14A-14D to show the observed axon/myelin typical variations within the corpus callosum for each mouse brain. The results shown in FIGs. 13A- 13C and 14A-14D from this study suggest that CNM-Au8 nanocrystalline suspensions favorably affected the amount of myelin damage and/or myelin repair in mouse brains in the observed regions between bregma-0.82mm to bregma-1.82mm (e.g., compare FIG. 14B, Group 2 to FIG. 14D, Group 4); and of significant importance, CNM-Au8 nanosuspensions did not appear to have an adverse effect on the amount of myelin present when provided alone with normal feed (e.g., see FIG. 14C-Group 3).

### Materials and Methods

#### *Animal Preparation and Induction of Demyelination*

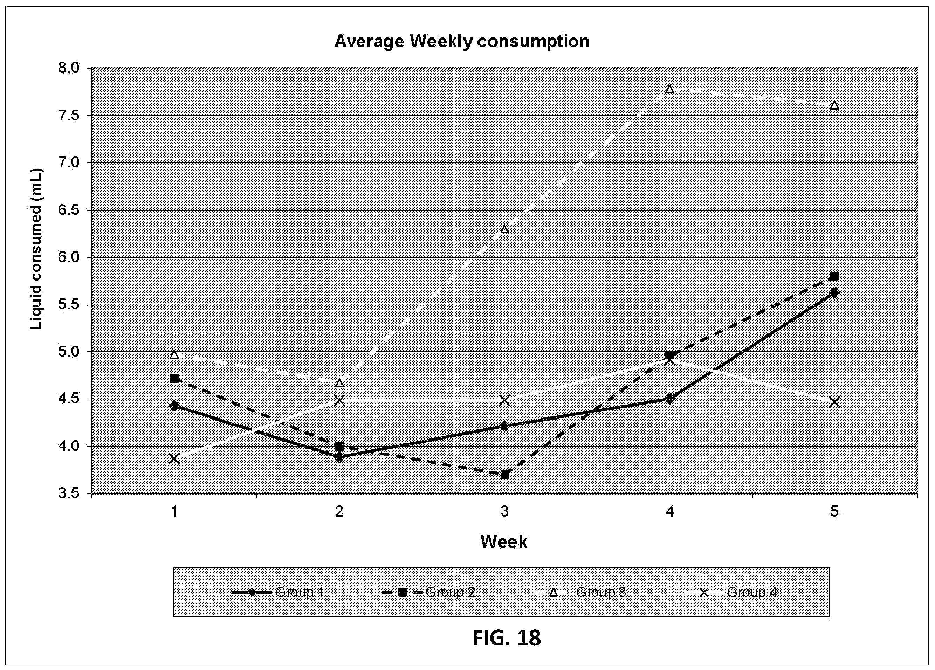

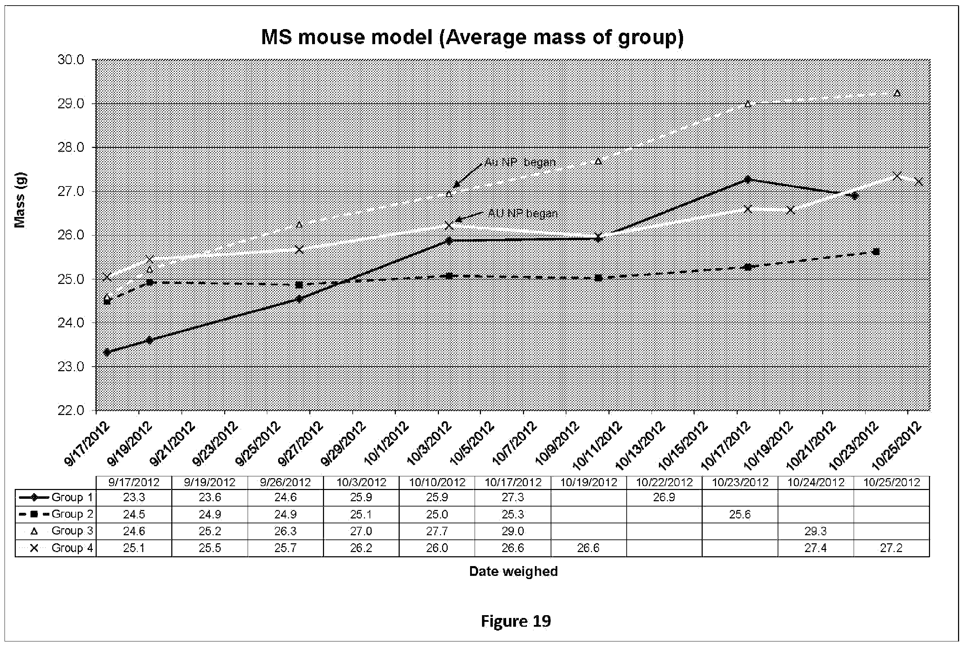

Male C57BL6 mice were obtained from Harlan Labs. All mice were observed 7-11 days prior to beginning the 5 week study. The mice underwent routine cage maintenance once a week and were monitored for behavioral changes and weighed once a week both before and during the 5 week study. FIG. 19 shows the average weight gain during the study, per mouse, for each of Groups 1-4. The mice in all groups were permitted to eat and drink as much, or as little, as desired. Specifically, all food, water and CNM-Au8 (51ppm of gold) treatment suspensions were provided *ad libitum.* The amount of water and CNM-Au8 treatment suspension consumed was recorded daily. The average amount of liquid consumed each day for the mice in each of Groups 1-4 is shown in FIG. 18. Fresh water and fresh CNM-Au8 nanosuspensions were provided daily.

Demyelination was induced by feeding the 8-week-old male C57BL6 mice feed pellets containing 0.2% cuprizone (bis-cyclohexanone oxaldihydrazone) (herein referred to as "Cuprizone Feed"), obtained from Harlan Labs (TD 06172) and pre-mixed into standard feed pellets. The control diet feed pellets were changed weekly, while the Cuprizone Feed was changed every 48 hours. Cuprizone Feed was provided for a five-week time period for the mice in Groups 2 and 4, in accordance with Table 2. A group size of four mice was used for each of the four groups, with only 2 mice being housed per cage due to the aggressive nature of these male mice.

In this pilot study, mice were anesthetized using sodium pentobartital (80 mg/kg) by injecting sodium pentobartital into the peritoneum with a 27 gauge, 0.5 inch needle. To minimize brain ischemia, the perfusion steps began immediately after each mouse was unconscious. Two different sets of perfusion buffers were used, depending on whether the mouse brain was subjected to immunostaining or TEM photomicroscopy. Perfusion associated with immunostaining utilized a total of 50 ml cold saline (0.9% NaCl in $dH_2O$), followed by a total of 150 ml of fixative (4% paraformaldehyde ("PFA")) at 4°C, sequentially passed into the mouse circulation via a peristaltic pump. Perfusion associated with transmission electron photomicroscopy similarly utilized 50 ml cold saline, followed by a total of 150 ml of fixative (3.5% PFA, 1.5% Glutaraldehyde in 0.1M Cacodylate). The peristaltic pump delivered each liquid at a rate of about 8-10 ml/min. Care was taken to avoid the formation of any air bubbles in the peristaltic pump tubing throughout the perfusion step.

To achieve sufficient perfusion, the right atrium of each mouse heart was cut open with small scissors. A butterfly needle was then inserted into the apex of the left ventricle and the pump started pumping the aforementioned liquids, while the right ventricle part of each heart was carefully held with tweezers. The brains from three mice in each group were prepared for paraffin block mounting (for immunostaining) while one mouse brain from each group was prepared for TEM photomicroscopy. Once each mouse brain was removed, each was post-fixed in the previously prepared cold (4°C) buffers, respectively, and either paraffin-embedded for immunostaining or resin-embedded for TEM photomicroscopy, in accordance with the literature.[4]

Table 2

| Cuprizone Pilot Study Matrix | | | | | | |
|---|---|---|---|---|---|---|
| Group 1 | ...... H$_2$0 ...... | | | | | Drinking |
| | 1 | 2 | 3 | 4 | 5 | |
| | < < < Normal Chow > > > | | | | | Diet |
| Group 2 | ......... H$_2$0 .......... | | | | | Drinking |
| | 1 | 2 | 3 | 4 | 5 | |
| | - - - Cuprizone Fee ding - - - - - | | | | | Diet |

(continued)

| Group 3 | ... H$_2$0 ... | | ... CNM-Au8 (51 ppm) ... | | | Drinking |
|---|---|---|---|---|---|---|
| | **1** | **2** | **3** | **4** | **5** | |
| | < < < Normal Chow > > > | | | | | Diet |

| Group 4 | ... H$_2$0 .... | | .... CN M-Au8 (51 ppm) ... | | | Drinking |
|---|---|---|---|---|---|---|
| | **1** | **2** | **3** | **4** | **5** | |
| | - - - - Cuprizone Fee ding - - - - - | | | | | Diet |

*Immunohistochemistry*

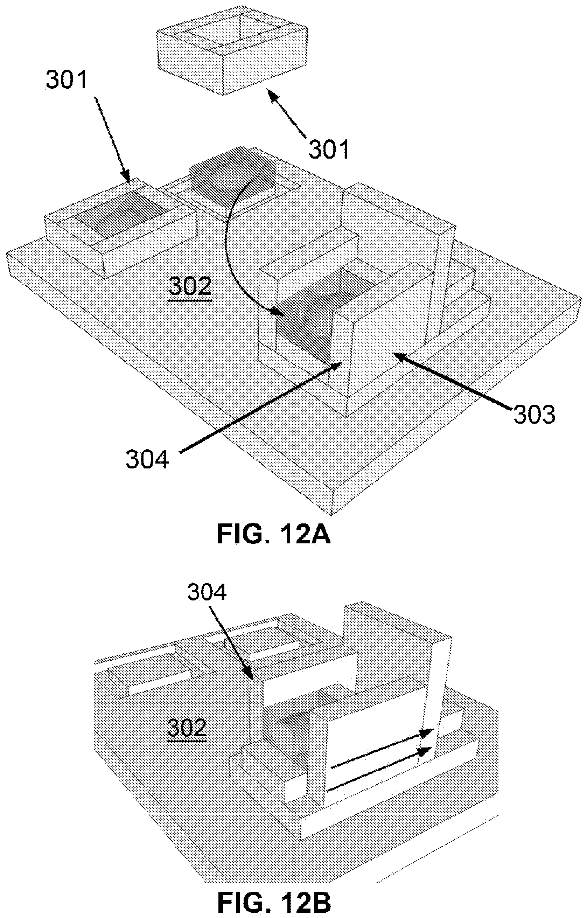

For immunostaining of PLP, 7 µm thick serial coronal brain sections between bregma-0.82 mm and bregma-1.82 mm (according to mouse atlas by Paxinos and Franklin[3]) were prepared using a custom holder, shown in FIGs 12A and 12B, and then were analyzed.

Specifically, each mouse brain was first embedded in an agar block. About 40 grams of agar powder (Tryptic Soy Agar, REF 236950, BD) was mixed thoroughly with about 1 liter of purified water, the mixture was then heated with frequent agitation and boiled for about 1 minute to completely dissolve the powder. A mold 301 was seated into the base 302 as shown in FIG. 12A. The mouse brain was then placed into the seated mold 301. When the temperature of the heated agar/water mixture cooled down to just above room temperature, the seated mold 301 was filled. About 2-3 minutes later, the mold 301 was lifted from the base 302 and the agar block containing brain was formed.

The agar block was then placed into the holder 303 and the portion of the brain corresponding to the head of each mouse was positioned such that it was facing the cutting edge 304. As shown in FIG. 12B, the holder 303 was then moved toward the position of the brain corresponding to the tail of the mouse and was located at a position which corresponded to bregma-0.82, and the brain was sectioned along the cutting edge 304 by a blade. The holder 303 was then moved about 1mm toward tail portion of the brain and the cutting edge 304 was then positioned at bregma -1.82. The brain block was then cut again at the cutting edge 304 by the same blade resulting in a 1mm thick slice between bregma-0.82 and -1.82.

According to the methods referenced previously[3], paraffin-embedded sections were dewaxed, rehydrated, while housed in a glass container partially filled with 10 mM citrate buffer (pH 6.0), and then microwave-heated in a conventional 1.65 KW household microwave until the buffer began to boil. Brain sections were then quenched with 0.3% H$_2$O$_2$, blocked for about 1 hr. in PBS containing 3% normal horse serum and 0.1% Triton X-100. The brain sections were then incubated at 4°C overnight in contact with the primary antibody against PLP, namely mouse IgG, (from AbD Serotec) at a dilution factor of about 1:500. Mouse IgG was chosen as the primary antibody because there is almost no IgG present in a mouse brain except for the dura portion of the brain.

After washing with washing buffer (PBS buffer, pH 7.4), coronal brain sections were further incubated with biotinylated anti-mouse IgG secondary antibody (purchased from Vector Laboratories) for about 1 hour, followed by exposure to peroxidase-coupled avidin-biotin complex (ABC Kit, Vector Laboratories) for about 30 minutes. Then, a material which is known to react with peroxidase-coupled avidin-biotin complex, referred to as diamino-3,3'benzidine ("DAB", Vector Laboratories), was contacted with the brain sections so that each of the sections changed color to somewhere between a light brown color to a dark brown color, depending on the amount of reaction which occurred between the peroxidase-coupled avidin-biotin complex and the DAB. A darker brown color corresponded to more myelin being present. FIG. 13B shows a representative myelin stained coronal brain section for a mouse in each of Groups 1-4. In order to determine the total areal size of each brain section observed (i.e., the total cross-sectional area of all brain matter present on each slide) additional serial brain slide specimens, located adjacent to the brown stained sections, were stained with both PLP antibody (as discussed above) and also stained with hematoxylin, which turned the brain sections a blue color (in addition to the already present brown coloring). FIG. 13C shows representative myelin+ hematoxylin stained brain sections for one mouse in each of Groups 1-4.

Specifically, to quantify the amount of immunopositive PLP in the coronal portion of each mouse brain, coronal sections (i.e., between bregma-0.82mm and 1.82mm) were examined. Rather than subjectively assigning a number associated with the degree of shading visually observed (i.e., light brown to dark brown), a unique method developed by the investigators was used.

First, a specially adapted Cannon Scanner (output resolution of 2400 dpi) scanned each of the brown stained coronal brain sections shown in FIG. 13B. Each pixel in the scan was then evaluated and automatically (by Photoshop) assigned a value between 1 and 255, with "255" corresponding to the lightest shade and "1" corresponding to the darkest shade. The data were then exported to Excel. The total number of pixels assigned to a number between 1 and 255 were then tabulated to achieve a histogram. The data for each histogram was then analyzed and a quantitative weighted average for the amount of "color" or "shade" in each myelin-stained coronal slide was determined. This quantitative number (appropriately corrected for background shading) resulted in the ability to make a direct comparison of the amount of color for an equal area in each myelin-stained coronal slide. To account for background input into the color or shade determination, an adjacent serial coronal section was used as a negative control. Specifically, the adjacent serial coronal section was stained without use of the primary antibody IgG.

Further, in order to make a meaningful scientific comparison of the amount of myelin present (i.e., color or shade intensity), which may correlate with certain aspects of preventing demyelination and/or promoting remyelination, in the coronal sections of the brain examined for each mouse in each mouse group, it was also necessary to determine the total amount (i.e., cross-sectional area) of brain matter present in each of the coronal sections. Thus, the total brain matter area on each coronal slide, shown in FIG. 13C, needed to be determined so that the total amount of color/brain matter area could be quantified and normalized. The amount of color per unit area was then used to compare directly the relative amount of myelin present. Accordingly, hematoxylin was used to stain all of the brain matter in another immediately adjacent serial coronal section. These brain sections were similarly quantified and the total "amount of color" (i.e., lightness or darkness) determined in the first coronal slide, was compared to the total cross-sectional area of brain matter present (represented by both blue coloring and light or dark brown coloring intensity above a minimum threshold amount), determined in a juxtaposed or serial coronal slide to determine the total amount of color or shade/unit area of brain matter.

As stated above, there were 4 groups of mice, and, for staining purposes, each group had 3 mice. Because staining intensity can vary as a function of environmental conditions which may vary when the staining steps are performed over a very large number of samples, great care was taken to normalize the staining or color intensity variations so that experimental results would not be skewed. The following steps well known and established steps were performed substantially in accordance with the literature.

Briefly, once the amount of color or shade per unit area of brain matter was determined, a negative control (corresponding the color or shade intensity which results without use of the aforementioned primary antibody), was subtracted from each result.

Moreover, for quantification, three separate sets of staining were designed and utilized. Each set contained four batches of the same staining characteristics. Each batch contained one sample from each group and one negative control. Staining of each sample was repeated four times to result in four batches of staining. Finally three samples from Group 1 and negative control were stained in a fifth batch.

The relative density of color or shade in each batch was first presented as a relative percentage to the corresponding Group1 sample in each batch. In the three samples from Group 1, normalization factors were expressed as a relative ratio of sample1 according to the staining in the fifth batch.

Further, the relative density to Group 1- Sample 1 was calculated again to by multiplying the relative density in each batch by their corresponding (and calculated) normalization factors.

Finally in each sample the Average of Relative Densities to Group 1- Sample 1 from four batches was calculated and determined to be the "Relative PLP density" which was then plotted as a bar graph (see Fig. 13A). All results of the myelin staining are shown in FIGs. 13A-13C.

*Preparation of Mouse Brain Sections for Transmission Electron Microscopy*

After perfusion, the samples were post-fixed in the aforementioned fixative for 4-6 hours at 4°C, then washed with cacodylate buffer (0.1M, pH 7.4) three times and stored in the same buffer at 4°C for 2-3 days.

A coronal slide was cut from the section of the brain between bregma-0.82 mm and - 1.82 mm by using the custom holder/procedure shown in FIG. 12.

Specifically, each mouse brain was first embedded in an agar block. About 40 grams of agar powder (Tryptic Soy Agar, REF 236950, BD) was mixed thoroughly with about 1 liter of purified water, the mixture was then heated with frequent agitation and boiled for about 1 minute to completely dissolve the powder. A mold 301 was seated into the base 302 as shown in FIG. 12A. The mouse brain was then placed into the seated mold 301. When the temperature of the heated agar/water mixture cooled down to just above room temperature, the seated mold 301 was filled. About 2-3 minutes later, the mold 301 was lifted from the base 302 and the agar block containing brain was formed.

The agar block was then placed into the holder 303 and the portion of the brain corresponding to the head of each mouse was positioned such that it was facing the cutting edge 304. As shown in FIG. 12B, the holder 303 was then moved toward the position of the brain corresponding to the tail of the mouse and was located at a position which corresponded to bregma-0.82, and the brain was sectioned along the cutting edge 304 by a blade. The holder 303 was then moved about 1mm toward tail portion of the brain and the cutting edge 304 was then positioned at bregma -1.82. The brain block was then cut again at the cutting edge 304 by the same blade resulting in a 1mm thick slice between bregma-0.82 and -1.82.

The slide tissues were post-fixed in 1.5% Potassium ferocyanide and 1% Osmium tetroxide in Cacodylate buffer for about 40 minutes at 4°C. After washing in Cacodylate buffer 3 times, the tissue blocks were again post-fixed in 1% Osmium tetroxide in Cacodylate buffer for about 1 hour at 4°C and followed by washing three times in $dH_2O$. The blocks were finally post-fixed in 1% Uranyl acetate in $dH_2O$ for about 40min, at room temperature. Dehydration steps then followed by immersing the blocks in 30% ethanol for about 5 minutes, 50% ethanol for about 5 minutes, 70% ethanol for about 5 minutes, twice, 80% ethanol for about 5 minutes, twice, 95% ethanol for about 10 minutes, twice, 100% ethanol three times each for about 10 minutes, 20 minutes and 30 minutes, propylene oxide for about 5 minutes, twice, propylene oxide plus resin (1:1 ratio) for about 60 minutes, and finally placed in resin overnight at room temperature. Samples were then incubated with resin at 37°C for about 1 hour, then with resin plus DMP catalyst at 37°C for about another hour and finally embedded in resin plus DMP catalyst at about 60°C for about 48 hours.

The slide tissue was cut in the middle sagittal plane of brain and ultrathin sections were cut along the surface where the middle sagittal plane is located. Sections measuring about 90nm thick were obtained using an Ultramicrotome (Reichert Jung Ultracut, Capovani Brothers Inc.; Scotic, NY) and photomicrographs were obtained with a transmission electron microscope (TEM, Zeiss Libra 120).

***G-ratio Measurement and Quantification***

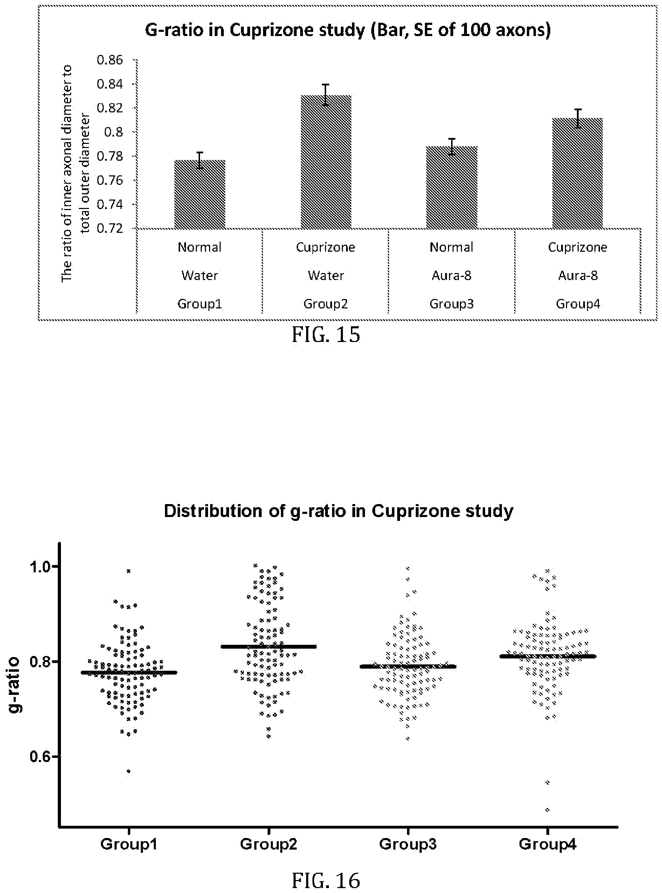

Using provided TEM software (Zeiss Libra 120), the cross-sectional areas of both neural axons and the total areas (i.e., cross-sectional areas of the axons and myelin sheaths combined), of 100 randomly selected axons in each of the four groups were measured; and then by utilizing specially adapted software, the inner and outer diameters were estimated (i.e., the observed cross-sectional areas of the axon/myelin sheath coatings were assumed to be concentric circles). G-ratios were calculated by dividing the calculated axon diameter by the calculated total outer diameter of the axons and myelin sheaths added together. The distribution of G-ratios is shown in FIG. 16 as a scatter plot utilizing GraphPad software.

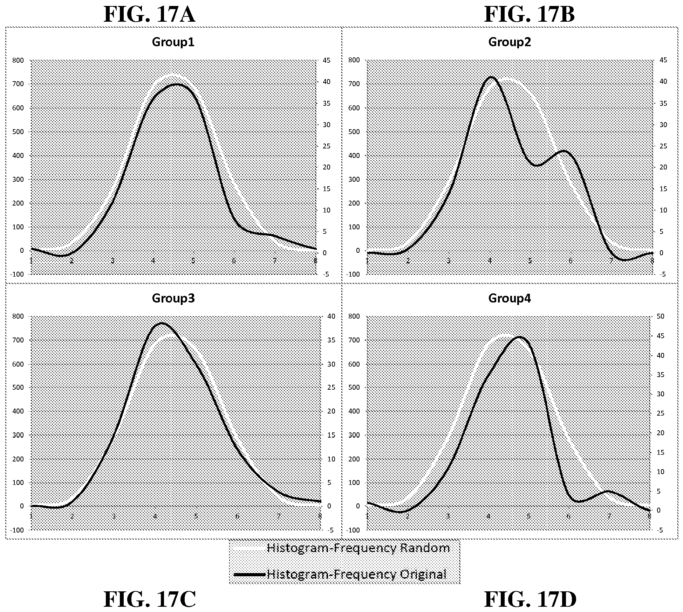

To present the data even more clearly, normal distribution curves of the G-ratios were plotted in Excel. Specifically, FIGs. 17A-17D show histograms for each of Groups 1-4. A set of random numbers was generated according to the average and standard deviation and a histogram named "Histogram-Frequency Random" was created; then the real data were used to plot another histogram named "Histogram-Frequency Original." The differences between the Random distribution curve and the Original distribution curve were then compared in each group.

***Quantification of PLP Immunostaining***

As shown in FIGs. 13A-13D, after 5 weeks of Cuprizone Feed, the Group 2 mice showed a marked loss of myelin relative to the myelin present in, for example, Groups 1, 3 and 4, thus suggesting that the conditions set forth in Table 2 for Group 2 were successful to cause demyelination of at least some of the coronal axons. A specific comparison between the amount of myelin present in Group 1 mice (water and control diet feed) and Group 2 mice (water and Cuprizone Feed) showed statistically significant myelin loss, $p < 0.01$. Further, Group 4 mice that consumed Cuprizone Feed for all 5 weeks, and received treatment with CNM-Au8 (51ppm) *ad libitum* for only 3 of the 5 weeks, showed more myelin present, suggesting myelin preservation and/or remyelination (compare Group 2 vs. Group 4, $p < 0.005$). Reference is also made to the amount of myelin present as shown, for example, in the TEM images in FIG. 14D, discussed elsewhere herein.

***Myelin Sheath Observations from TEM Photomicroscopy Studies***

FIGs. 14A-14D show representative TEM photomicrographs of cross-sectional areas of representative portions the corpus callosum regions for four different mice, namely, regions from one mouse from each of Groups 1-4. The matrix conditions for Groups 1, 2, 3 and 4 set forth in Table 2 seemed to result in data consistent with what one would hope for in a cuprizone demyelination study, namely, measurable loss of myelin in Group 2, in a timeframe which permits a determination if a candidate treatment (such as a CNM-Au8 gold nanosuspension) may show any beneficial therapeutic or prophylactic results, such as preventing or slowing demyelination and/or promoting remyelination.

The representative corpus callosum brain tissue cross-sectional samples in these four mice were originally observed by TEM at about 16,000x magnification (and scale bars are present on each photomicrograph representing the actual magnification). Hundreds of areas within the corpus callosum of each mouse were examined to arrive at a representative set of TEM photomicrographs. Thus, representative images of the cross-sectional areas taken from the corpus callousm of the four mice are shown in FIGs. 14A-14D (i.e., 9-10 images are shown in each figure). By utilizing only the naked eye, the thickness of the myelin sheaths and the characteristics of the axons were very similar between two groups that received control diet feed (i.e., control (Group1) and CNM-Au8 -only treatment (Group 3)). In contrast, however, the mouse group which received Cuprizone Feed and water (Group 2), clearly showed less total myelin present (e.g., suggesting damage to and/or demyelination of the shown axons) in portions of the observed cross-sections in the corpus callosum. Consistent with the literature, the myelin degradation caused by the Cuprizone Feed was found to have non-uniform effects (i.e., was non-homogenous) on the myelin/axons in the corpus callosum cross-sectional areas viewed. Specifically, in the corpus callosum cross-sections observed in this study, it appeared that somewhere around less than 40% of the cross-sectional areas viewed exhibited some amount of myelin damage, while the remaining cross-sectional areas appears to be similar to control (i.e., similar to Group 1). It should be noted that this is considered to be typical and in agreement with the cuprizone mouse model studies reported elsewhere in the literature.

The TEM photomicrographs of the corpus callosum cross-sectional area of the Group 4 mouse is of great interest. This mouse received Cuprizone Feed for all 5 weeks of the study and CNM-Au8 suspension *ad libitum* treatment for weeks 3-5 of the study. The TEM photomicrographs in FIG. 14D show that there were few, if any, demyelinated axons and that the total amount of myelin present was similar to the total amount of myelin present in control (Group 1). These observations correspond to the total amount of myelin present, as captured by the immunostaining results of the stained coronal brain sections for the three mice in each of Groups 1-4, as set forth in FIGs. 13A-13D. Specifically, the relative amount of PLP staining shown in FIGs. 13A-13D for the coronal sections of the three mice in each of mouse Groups 1, 3 and 4 are higher (i.e., corresponding to more myelin present) than the relative amount of PLP staining for the three mice in Group 2 that consumed Cuprizone Feed and water.

**G-ratio Measurement and Distribution of G-ratios**

G-ratio measurement and quantification are widely utilized as a functional and structural index of the relative amount of myelin coating present on axons (i.e., axonal myelination). The higher the G-ratio, the thinner the myelin is relative to the axon diameter; and conversely, the lower the g-ratio, the thicker the myelin sheath is relative to the axon. Thus, typically, and within norms, the lower the reported G-ratio, the better. Specifically, it has been reported that average G-ratio ranges for myelinated axons for the corpus callosum region of the brain is within the range of 0.75 to 0.815.

G-ratios measured in this study are reported in FIG. 15. The highest reported G-ratio occurs in Group 2, namely those mice that consumed Cuprizone Feed and water. The reported G-ratio for the Group 2 mice was higher than the reported G-ratios of the other Groups. The lowest reported G-ratio is for the Group 1 mice, namely, those that were fed control diet feed and water.

Of interest, the G-ratio comparison between the Group 1 mice (water and Cuprizone Feed) and the Group 3 mice (CNM-Au8 suspension *ad libitum* and control diet feed) showed very little difference, consistent with the TEM images in FIG. 14A and FIG. 14C, respectively. These additional data also suggest that CNM-Au8 suspensions did not have any measureable negative side effects regarding the amount of myelin present.

To understand further the reported G-ratios, data scatter plots for each of Groups 1-4 were generated. As shown in FIG. 16, the Group 2 mice (e.g., the higher G-ratio; which may correspond to less healthy or negatively modified axon function) exhibited the highest data scatter compared to the three other mouse groups. The data scatter in the remaining three mouse groups was very similar; with there being no effective difference observed between Group 1 and Group 3.

To quantify the G-ratio data even further, the data were expressed differently in FIGs. 17A-17D. Specifically, bell shaped curves were generated and plotted to show the continuous probability distribution of the G-ratio data in each of the four mouse groups. The four plots in FIGs. 17A-17D each include a curve labeled "Histogram-Frequency Random" which was generated from the G-ratio data "average" and the standard deviation of the G-ratio data for that group (created effectively as an internal control). In addition, the four plots in FIGs. 17A-17D each also include a curve labeled "Histogram-Frequency Original" which was generated from the actual G-ratio data.

The data plotted in FIGs. 17A-17D show that the "Histogram-Frequency Random" plot and the "Histogram-Frequency Original" plot are very similar for each of Groups 1, 3 and 4. In contrast, the mice that consumed the Cuprizone Feed (i.e., Group 2) show two large peaks associated with the original G-ratio data. Moreover, the "Histogram-Frequency Original" curve is quite different from the "Histogram-Frequency Random" distribution curve. Of interest, the CNM-Au8 treatment suspension provided *ad libitum* to the mice in Group 4 appeared to minimize the differences in the curves, relative to the mice adversely affected by the Cuprizone Feed. For the data associated with the Group 4 mice, the "Original" distribution curve is basically similar to its "Random" distribution curve, with only a very small peak appearing at the higher ratio end of the curve.

***Conclusions***

The data suggest:

1. Mice that were given Cuprizone Feed and water for 5 weeks developed myelin loss or damage (e.g., demyelination)

EP 2 994 146 B1

that was sought by the investigators (i.e., Group 2 from Table 2).

2. Mice that were given control diet feed and CNM-Au8 suspension *ad libitum* (i.e., Group 3) did not show any abnormal behavior or measured myelin differences relative to mice that were given control diet feed and water (Group 1).

3. CNM-Au8 treatment suspensions provide *ad libitum* positively affected the amount of myelin present (e.g., reduced myelin damage and/or promoted remyelination) of the mice in Group 4 that were exposed to Cuprizone Feed for all 5 weeks and CNM-Au8 suspension (51ppm gold concentration) for the last 3 weeks of the study.

## EXAMPLE 3

### Cuprizone Demyelination Model- 2 Week/5 Week -105 Mouse Study

### Summary

The goal of this 105 mouse study was to determine if "CNM-Au8" nanosuspensions, provided to the mice: (1) either as an *ad libitum* treatment from water bottles at a gold concentration of about 50 ppm (as the only drinking liquid for the mice for the last 3 weeks or all of the 5 weeks in the study); or (2) by gavage treatment (for the last 3 weeks or all of the 5 weeks in the study) at a gold concentration of about 1000 ppm (and given once a day, by gavage, based on the weight of each mouse at a volume of about 10 mL of CNM-Au8 nanosuspension/kg of mouse body weight, "10mL/kg"), might act as a therapeutic effective amount or a prophylactic effective amount and thus influence the amount of myelin damage present in the corpus callosum and/or promote remyelination of at least some axons in the corpus callosum. As in Example 2, the myelin damage was induced by the mice ingesting Cuprizone Feed.

A total of 105 C57BL6 male mice were separated into 7 groups (15 mice per group), as shown in Table 3. The mice were about 8 weeks old at the start of the study.

In an attempt to induce myelin damage (e.g., a negative reaction of the myelin and/or demyelination) six of the seven groups (i.e., Groups 2-7) were fed the same Cuprizone Feed discussed in Example 2. The seven mouse groups and the respective conditions to which the seven mouse groups were exposed are set forth briefly below, as well as being summarized in Table 3.

**Group 1.** The 15 mice in Group 1 consumed regular chow for all 5 weeks of the study (i.e., were not fed Cuprizone Feed) and also drank water for all 5 weeks of the study, and were then processed as described herein.

**Group 2.** The 15 mice in Group 2 were fed Cuprizone Feed for two weeks and drank water for the same two weeks of the study, and were then processed after two weeks, as described herein.

**Group 3.** The 15 mice in Group 3 were fed Cuprizone Feed for all 5 weeks and drank water for all 5 weeks of the study, and were then processed as described herein.

**Group 4.** The 15 mice in Group 4 were fed Cuprizone Feed for all 5 weeks of the study and were given by gavage, for all 5 weeks, once a day, a treatment volume of about 10 mL/kg of a concentrated CNM-Au8 suspension at a gold crystal concentration of about 1000 ppm, and were then processed as described herein to determine if the gold nanosuspension provided was a prophylactic effective amount.

**Group 5.** The 15 mice in Group 5 were fed Cuprizone Feed for all 5 weeks and drank water for the first 2 weeks of the study and were then given by gavage, for the next 3 weeks, once a day, a treatment volume of about 10 mL/kg of a concentrated CNM-Au8 suspension at a crystalline gold concentration of about 1000 ppm, and were then processed as described herein to determine if the gold nanosuspension provided was a therapeutic effective amount.

**Group 6.** The 15 mice in Group 6 were fed Cuprizone Feed for all 5 weeks of the study and drank *ad libitum* from water bottles a treatment CNM-Au8 suspension at a crystalline gold concentration of about 50 ppm for all 5 weeks of the study, and were then processed as described herein to determine if the gold nanosuspension provided worked as an effective treatment.

**Group 7.** The 15 mice in Group 7 were fed Cuprizone Feed for all 5 weeks of the study and drank water for the first 2 weeks of the study and then drank *ad libitum* from water bottles a treatment CNM-Au8 suspension at a gold concentration of about 50 ppm for the next 3 weeks of the study, and were then processed as described herein to determine if the gold nanosuspension provided worked as an effective treatment (e.g., acted as a therapeutic effective amount).

### Materials and Methods

#### *Animal Preparation and Induction of Myelin Damage*

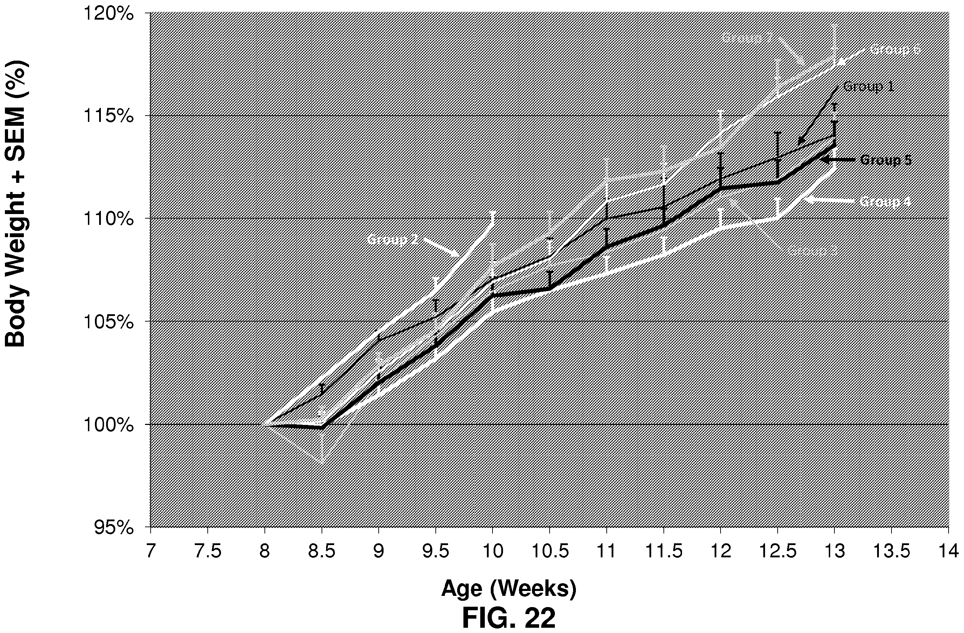

Male C57BL6 mice were obtained from Taconic Farms. All mice were acclimated between 2-4 weeks prior to

26

beginning the 2/5 week study. The mice underwent routine cage maintenance and were monitored for behavioral changes. Mice were weighed before the start of the study and then twice a week during the study. FIG. 22 shows the average weight gain per mouse for the mice in each of Groups 1-7.

The 15 mice in each of Groups 1-7 were permitted to eat and drink as much, or as little, as desired. Specifically, all food, water and CNM-Au8 (50 ppm concentration of gold) treatment suspensions were provided *ad libitum* or were provided by gavage (1000 ppm concentration of gold), as noted above. Fresh water and fresh CNM-Au8 suspensions were provided daily.

All mice were anesthetized using Avertin (250-400 mg/kg) by injecting Avertin into the peritoneum with a 27 gauge, 0.5 inch needle. To minimize brain ischemia, the perfusion steps began immediately after each mouse was unconscious. Perfusion buffers used utilized up to 50 ml cold saline (0.9%NaCl in $dH_2O$) until the liver became completely clear as observed for each mouse using loupes (magnifying glasses), followed by a total of about 150 -180 ml of fixative (3.5% PFA, 1.5% Glutaraldehyde in 0.1M Cacodylate). The peristaltic pump delivered each liquid at a rate of about 4-6 ml/min. Care was taken to avoid the formation of any air bubbles in the peristaltic pump tubing throughout the perfusion step.

To achieve sufficient perfusion, the right atrium of each mouse heart was cut open with small scissors. A needle was then inserted into the apex of the left ventricle and the pump started pumping the aforementioned liquids. After at least 150 mL of PFA (Glutaraldehyde) had passed, the peristaltic pump was stopped. Using scissors, the head was removed, a small cut was made into the skull, which was then chipped away until the brain could be easily removed. Once each mouse brain was removed, each brain was post-fixed in the previously prepared cold (4°C) buffers, respectively, and resin-embedded for TEM photomicroscopy, in accordance with the literature.[4]

The raw materials for perfusion were obtained from the following sources:

(1) Sodium cacodylate trihydrate (for EM): Sigma Aldrich, Cat#: C0250-100G
(2) Paraformaldehyde EM Grade, Prill Purified, 1kg: Ted Pella, Cat#: 18501
(3) Glutaraldehyde, 50% EM grade, 10 X 10ml: Ted Pella, Cat#: 18431
(4) Sterilization Filter Units: Fisher Scientific, Cat#: 09-740-2A

**Table 3**

| Group 1 | ................ $H_2O$ ................ | | | | |
|---|---|---|---|---|---|
| Mouse Nos. | **1** | **2** | **3** | **4** | **5** |
| 1-15 | < < < < Normal Diet > > > > | | | | |

| Group 2 | ..... $H_20$ ..... | | | | |
|---|---|---|---|---|---|
| Mouse Nos. | **1** | **2** | | | |
| 1-15 | - - Cup rizone Feed - | | | | |

| Group 3 | ................$H_20$ .. ........ ...... | | | | |
|---|---|---|---|---|---|
| Mouse Nos. | **1** | **2** | **3** | **4** | **5** |
| 1-15 | - - - - Cuprizone Feed - - - - | | | | |

| Group 4 | : : : CNM - Au8 Suspensio Suspension - Gavage (1000 PPM Gold; 10 mL/kg) : : : : | | | | |
|---|---|---|---|---|---|
| Mouse Nos. | **1** | **2** | **3** | **4** | **5** |
| 1-15 | - - - - Cuprizone Feed - - - - | | | | |

(continued)

| Group 5 | ..... $H_2O$ ..... | | CNM - Au8 Gold; 10mL/kg) Supension - Gava ge (1000 PPM | | |
|---|---|---|---|---|---|
| Mouse Nos. | 1 | 2 | 3 | 4 | 5 |
| 1-15 | - - - - Cuprizone Feed - - - - | | | | |

| Group 6 | : CNM - Au8 Suspension - Drinking Bottle ( 50 PPM Gold; ad libitum): : : | | | | |
|---|---|---|---|---|---|
| Mouse Nos. | 1 | 2 | 3 | 4 | 5 |
| 1-15 | - - - - Cuprizone Feed - - - - | | | | |

| Group 7 | $H_2O$ ..... | | CNM - Au8 Sus (50 PPM Gold; ad pension - Drinking ad libitum) Bottle | | |
|---|---|---|---|---|---|
| Mouse Nos. | 1 | 2 | 3 | 4 | 5 |
| 1-15 | - - - - Cuprizone Feed - - - - | | | | |

*Preparation of Mouse Brain Sections for Transmission Electron Microscopy*

After perfusion, the samples were post-fixed in the aforementioned fixative for 4-6 hours at 4°C, then washed with cacodylate buffer (0.1M, pH 7.4) three times and stored in the same buffer at 4°C for 2-3 days.

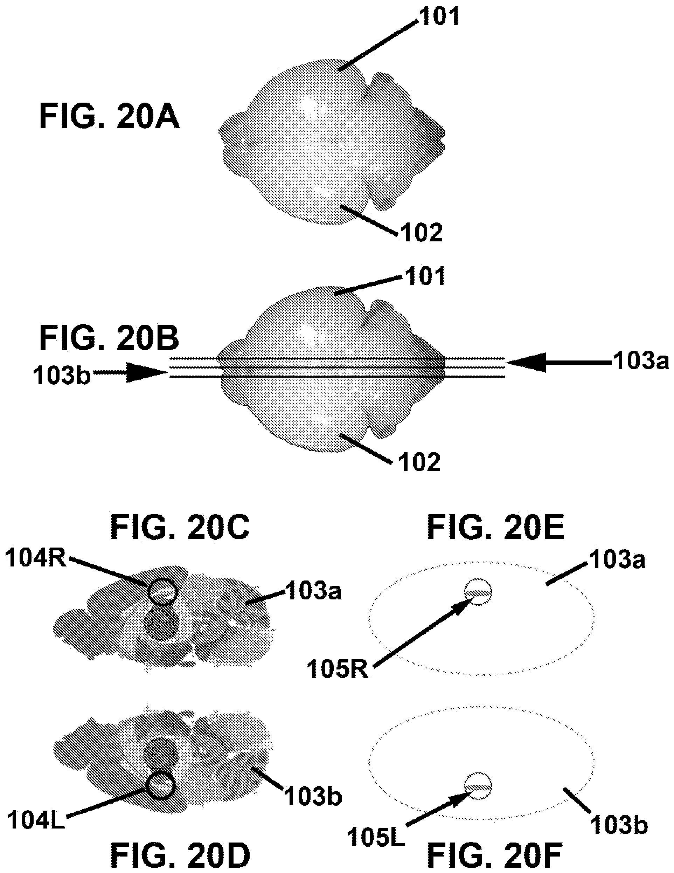

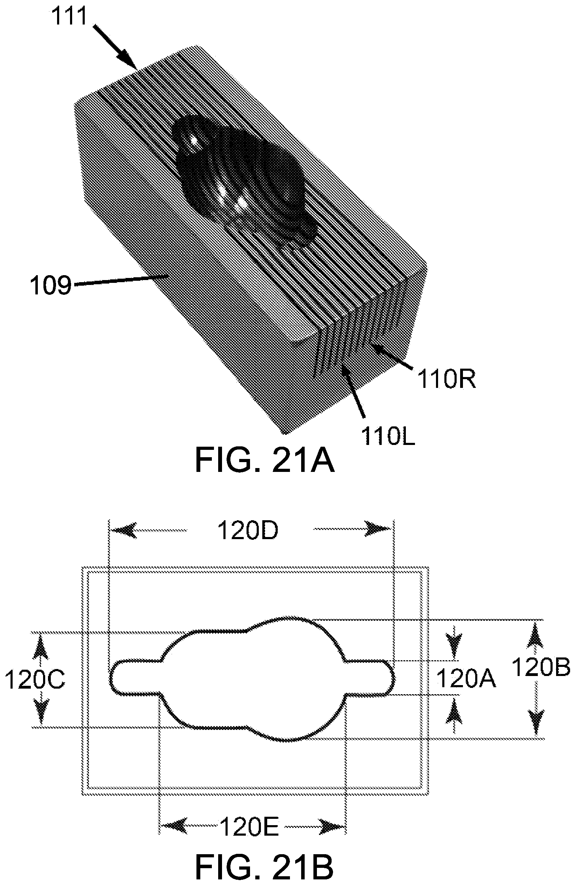

The brain was removed from cacodylate buffer, and placed onto tissue paper. A razor blade was inserted into the middle line of the brain sagittally. The brain, with the razor blade still positioned into the middle line, was placed into the sagittal mouse brain matrices 109, shown in FIG. 21A, and the razor blade was guided into the center groove 111 of a sagittal mouse brain matrices 109. The sagittal mouse brain matrices 109 consists of thirteen grooves that are spaced 1mm apart. Without moving the brain, a second blade was inserted into the groove, 110R, 2mm apart from the center groove on the right side, and a third blade was inserted into the groove, 110L, 2mm apart from the center groove on the left side. Two mirror slides of brain tissue 103a and 103b, as shown in FIGs. 20B, 20C and 20D were made. Cylindrical tissue blocks 104R and 104L with a diameter of 2mm were then cut by a Harris Uni-Core (Ted Pella, Prod # 15076) as shown in FIGs. 20C and 20D. The position of the cylindrical tissue block 104R and 104L were taken where the posterior portion (i.e., splenium portion) of the Corpus Callosum 105R and 105L run through the tissue slides 103a and 103b. The tissue block surface, which is on the middle sagittal plane of brain, was labelled and EM sections will be cut on this surface.

The block tissues were post-fixed in 1.5% Potassium ferocyanide and 1% Osmium tetroxide in Cacodylate buffer for 40minutes at 4°C. After washing in Cacodylate buffer three times, the tissue blocks were again post-fixed in 1% Osmium tetroxide in Cacodylate buffer for about 1 hour at 4°C and followed by washing three times in $dH_2O$. The blocks were finally post-fixed in 1% Uranyl acetate in $dH_2O$ for about 40min, at room temperature. Dehydration steps then followed by immersing the blocks in 30% ethanol for about 5 minutes, 50% ethanol for about 5 minutes, 70% ethanol for about 5 minutes twice, 80% ethanol for about 5 minutes twice, 95% ethanol for about 10 minutes twice, 100% ethanol three times each for about 10 minutes, 20 minutes and 30 minutes, propylene oxide for about 5 minutes twice, propylene oxide plus resin (1:1 ratio) for about 60 minutes, and finally room temperature resin overnight. Samples were then incubated with resin at 37°C for about 1 hour, then with resin plus DMP catalyst at about 37°C for another hour and finally embedded in resin plus DMP catalyst at about 60°C for about 48 hours.

Sections measuring about 90nm thick were obtained using an Ultramicrotome (Reichert Jung Ultracut, Capovani Brothers Inc.; Scotic, NY) and photomicrographs were obtained with a transmission electron microscope ("TEM", Zeiss Libra 120).

The 15 mice in Group 2 were terminated after 2 weeks of eating Cuprizone Feed and drinking only water in order to assess the amount and type of axonal myelin damage in the corpus callosum after 2 weeks of exposure to Cuprizone Feed; and the other 90 mice in the other six groups were all terminated after 5 weeks, as set forth in Table 3.

In each of the seven mouse groups, a predetermined area of the brain from each mouse was targeted for extraction. The corpus callosum region of the brain is heavily populated with axons that are sensitive to the cuprizone treatment and this region was the area of focus for all the TEM studies. Several different quantitative and qualitative evaluation techniques were then employed to observe and quantify many of the TEM images taken.

*I. Comparison between Corpus Callosum TEM Images Taken at 4,000x and 5,000x*

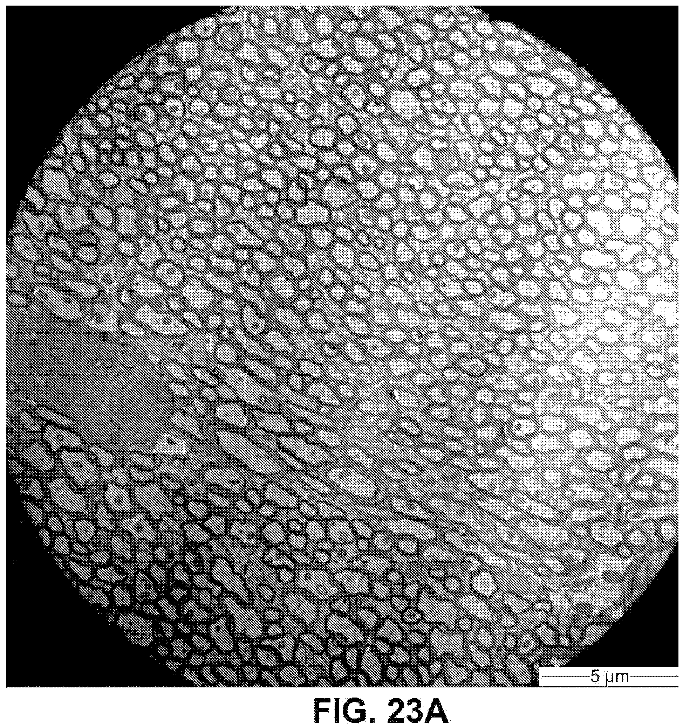

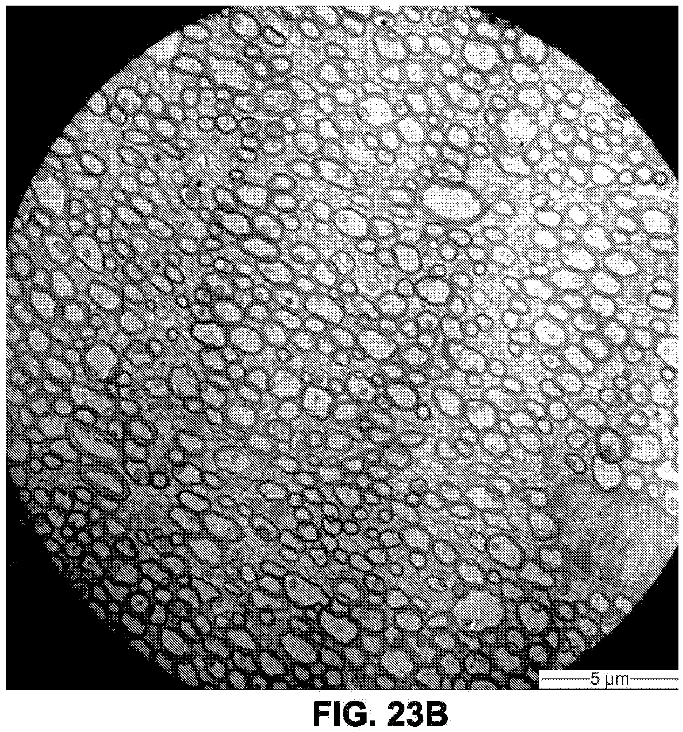

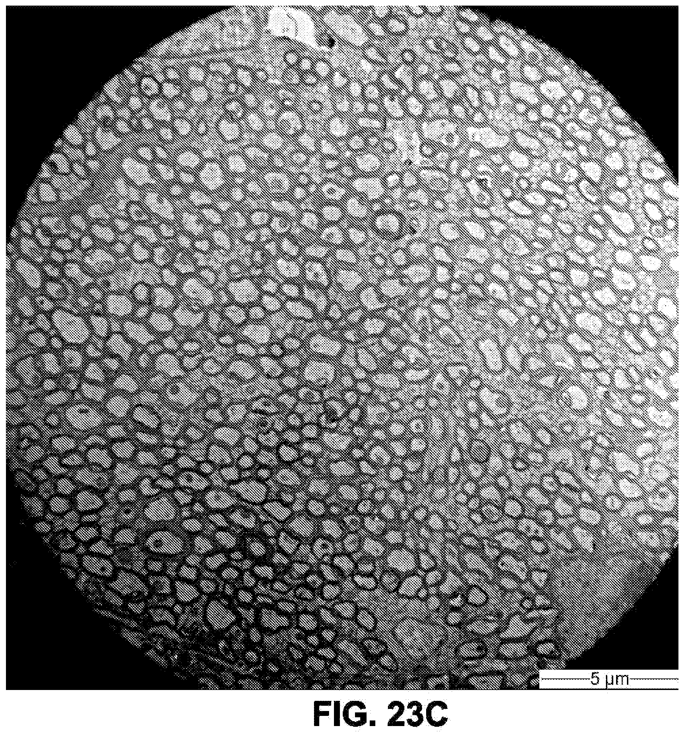

A first set of TEM images was taken at the lowest magnification, originally taken at 4,000x - 5,000x, and the TEM set appears as FIGs. 23-29. Each of these images represents a small portion of the entire corpus callosum region of each mouse brain. Further, these images were not randomly selected, but rather, were chosen because they correspond to the region(s) of the corpus callosum that showed the most extensive damage to the myelin.

It is again noted that the toxic cuprizone model does not result in uniform myelin damage across the entire corpus callosum, so great care was taken by skilled operators to choose those portions exhibiting the greatest amount of damage due to the Cuprizone Feed.

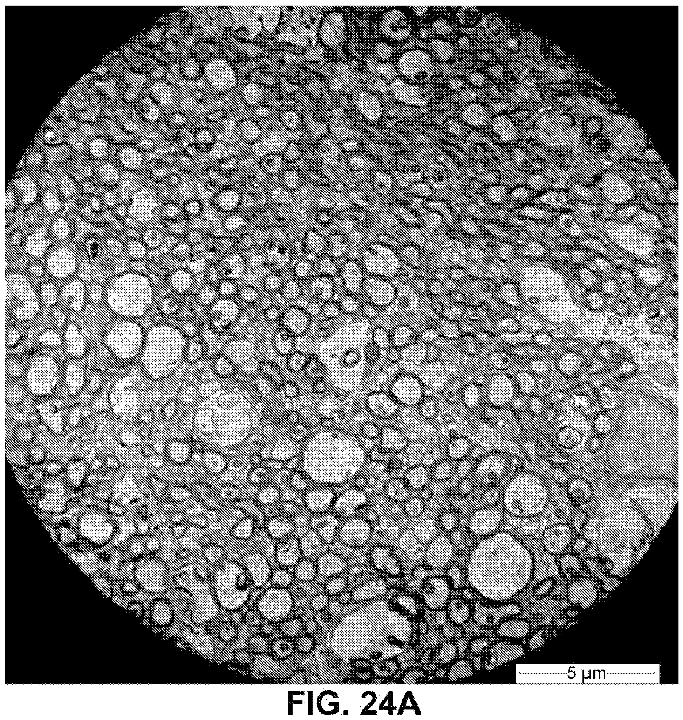

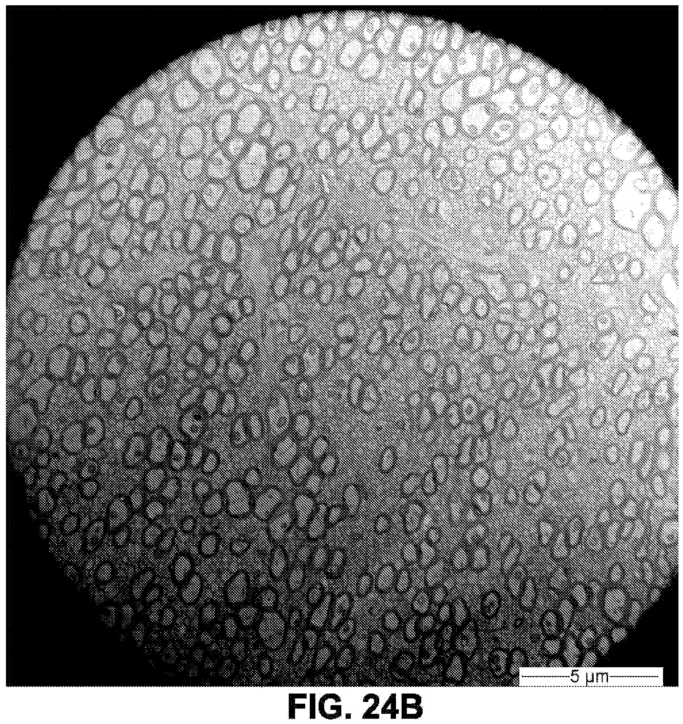

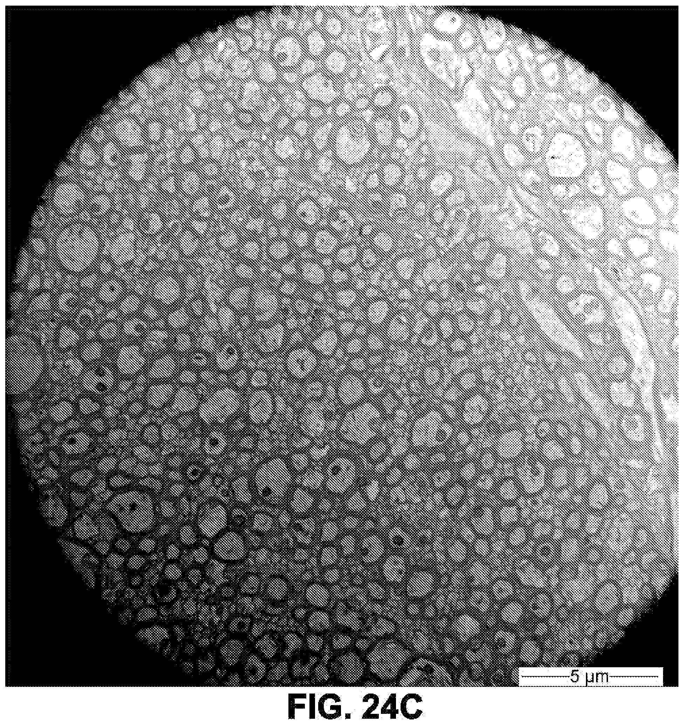

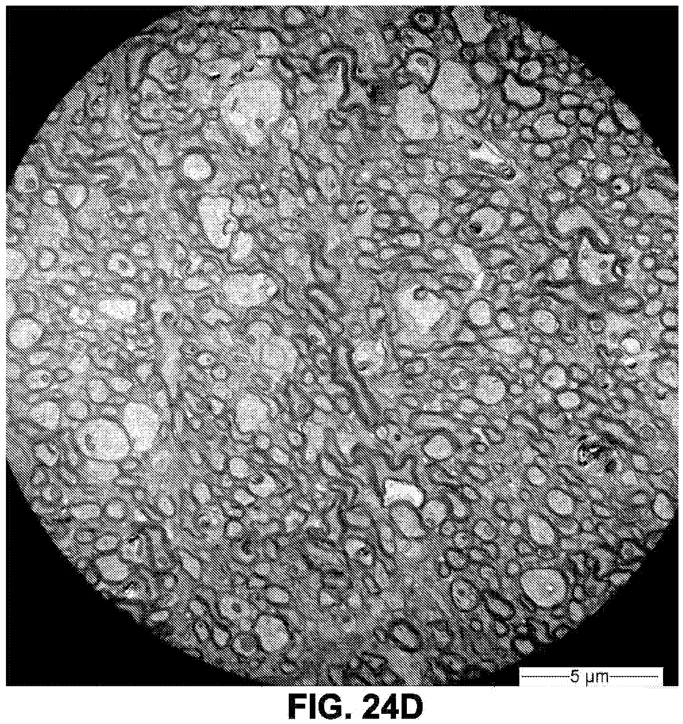

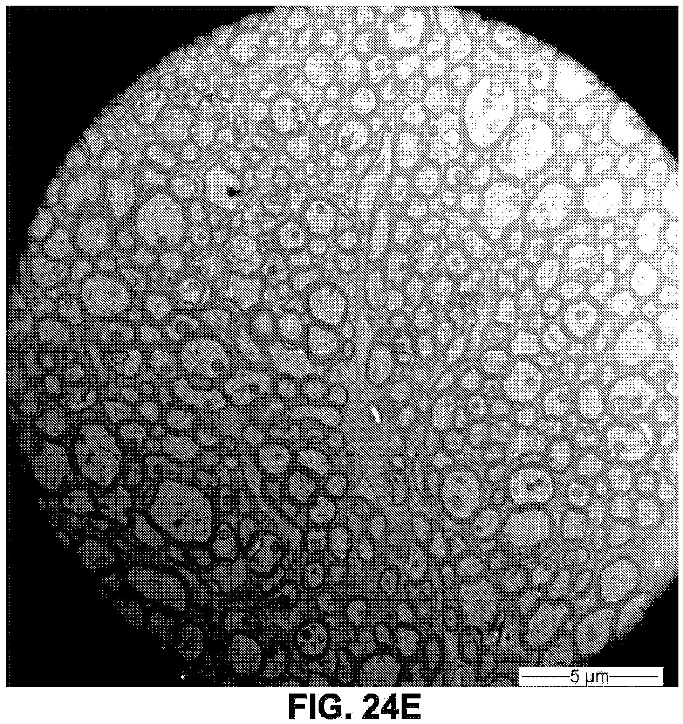

FIGs. 23A, 23B and 23C, all originally taken at 4,000x, correspond to mouse brains from the Group 1 mice. The Figure 23 TEM photomicrographs show, relative to all the other TEM photomicrographs in FIGs. 24-29, the most amount of myelin present on the corpus callosum axons. No areas of extensive demyelination or dysmyelination could be identified anywhere within the corpus callosum regions of these Group 1 mice.

TEM photomicrographs corresponding to mice from Group 2, all originally taken at 4,000x, are shown in FIGs. 24A-24E. These representative TEM images show areas of less myelin present relative to FIGs. 23A - 23C (i.e., the Control Group 1). It should be noted that in viewing the mouse brains associated with Group 2, that there were areas in the Group 2 mouse brains that showed several areas of less myelin being present in the corpus callosum; and such areas were not observed in the Control (Group 1) images represented by FIGs. 23A-23C.

It should also be noted that a number of axons shown in the FIGs. 24A-24E photomicrographs appear to have a larger diameter than any axons observed and photographed in the Control Group 1. The observed non-normal thicker myelin on some axons are likely a reaction to the toxic Cuprizone Feed. Specific reference is made to FIGs. 24A, 24D and 24E.

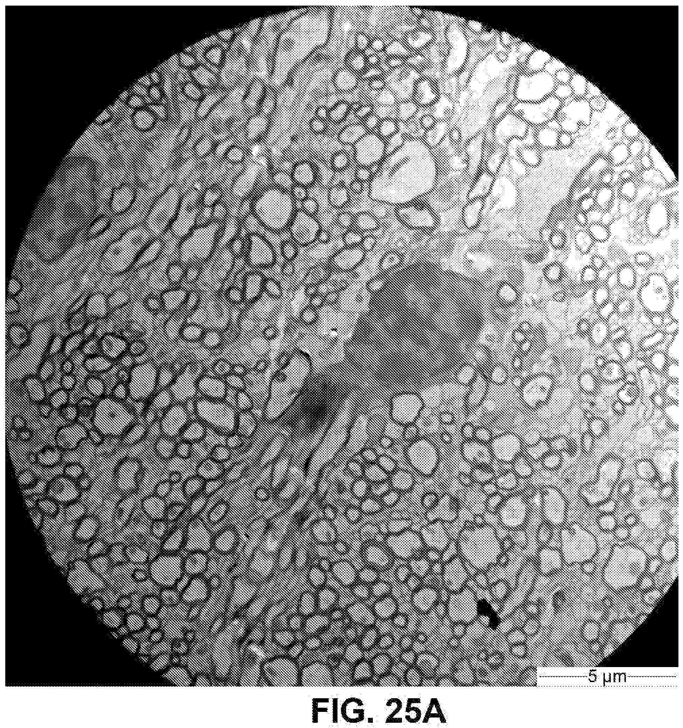

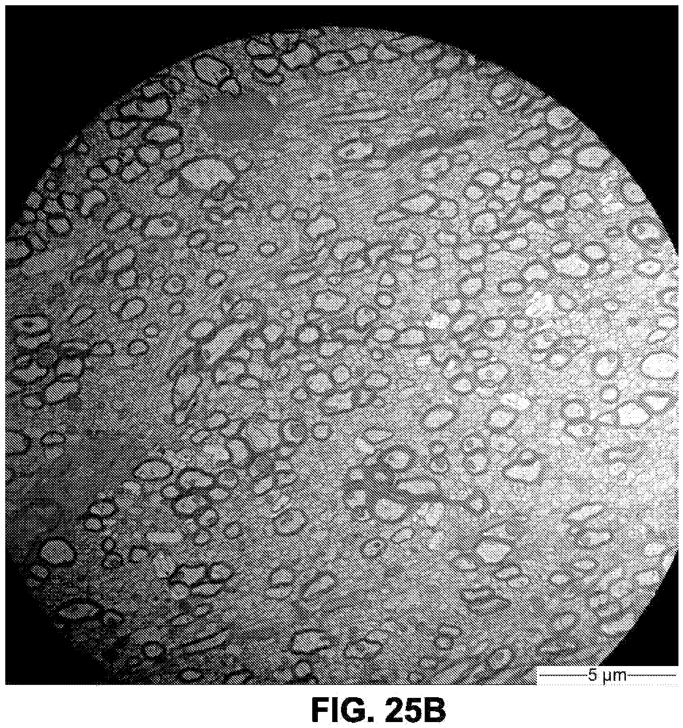

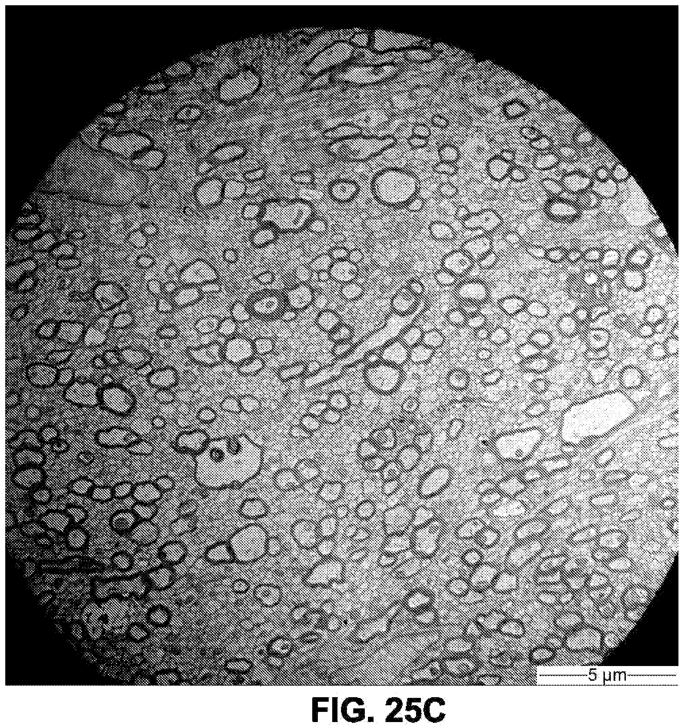

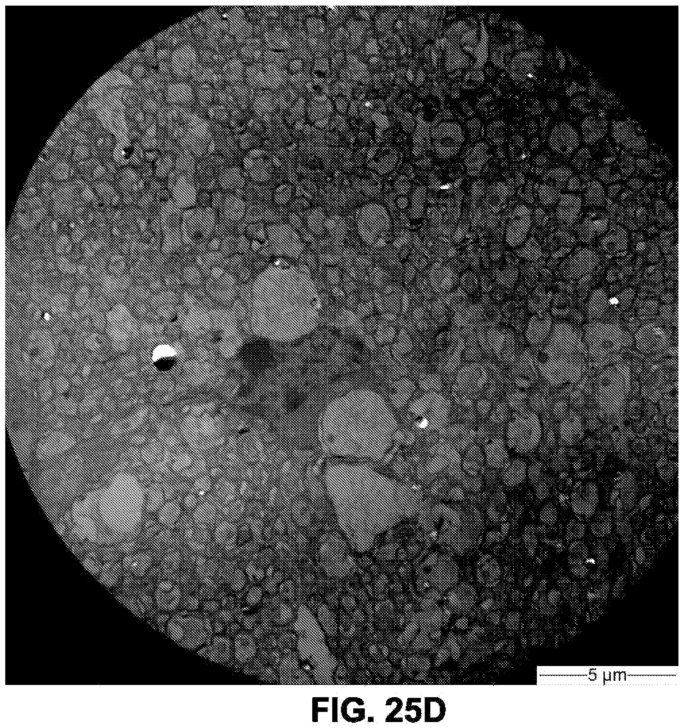

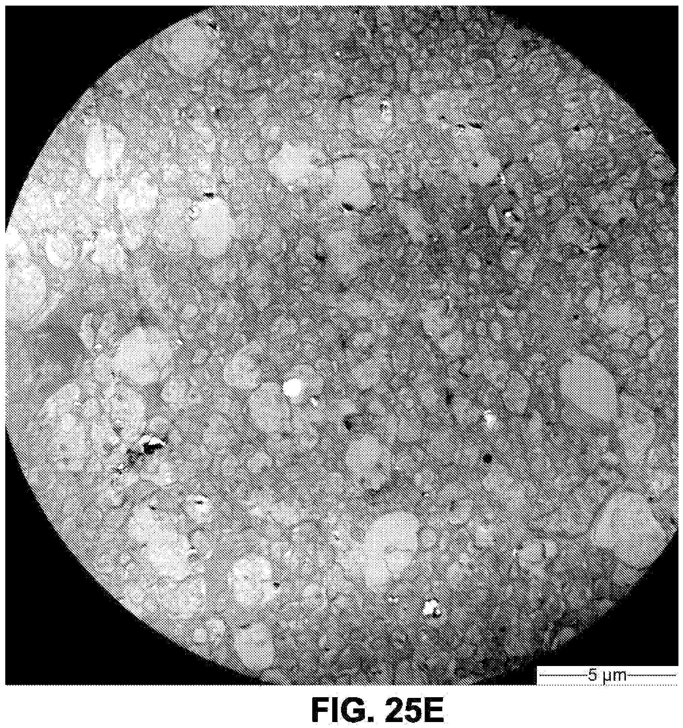

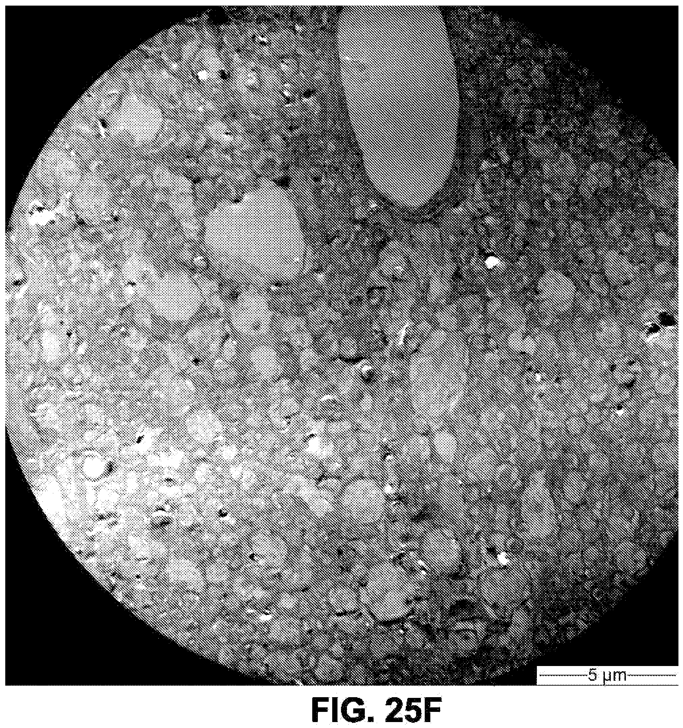

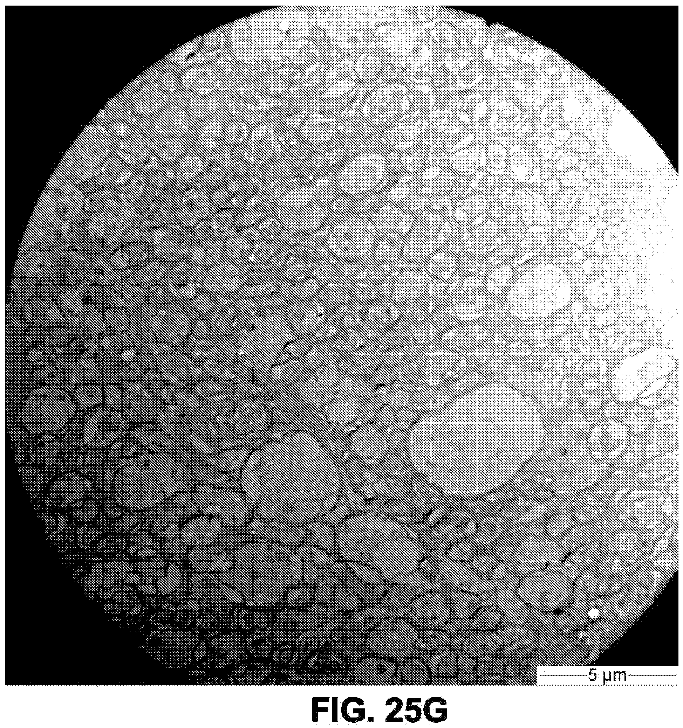

TEM photomicrographs corresponding to brains of mice in Group 3 (i.e., the mice that were given Cuprizone Feed for 5 weeks) are shown in FIGs. 25A-25G. These representative TEM images, all originally taken at 4,000x, show areas of the corpus callosum where even less myelin is present relative to the Group 2 mice which consumed Cuprizone Feed for only 2 weeks. Further, it appears that there are even more axons having a larger diameter and thinner myelin sheaths than any axons observed and photomicrographed in the mice from the Control Group 1. Specific reference is made to FIG. 25D, FIG. 25E, FIG. 25F and FIG. 25G. Myelin damage is an established finding during weeks 2-6 of cuprizone induced demyelination[13] and axonal spheroids such as observed herein have been previously reported.[14] Further, the large observed axonal swellings may be a reaction to the loss of myelin.

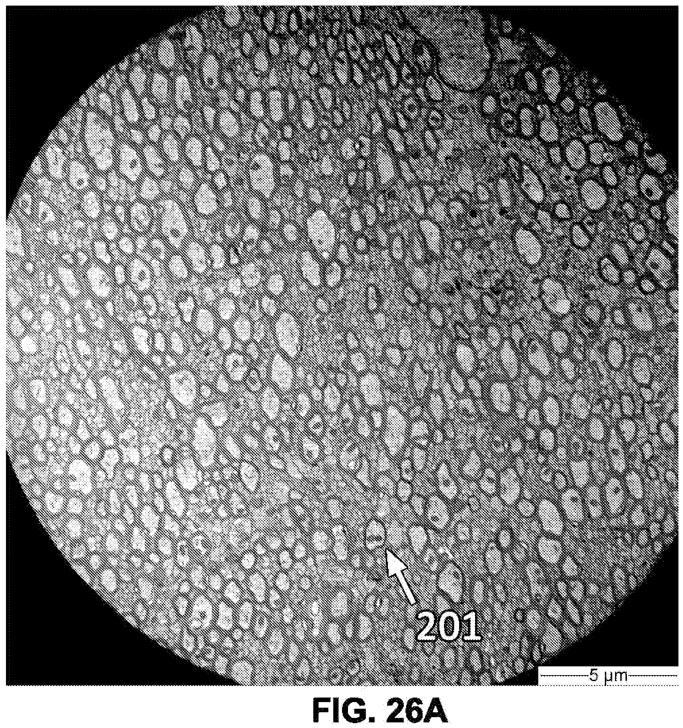

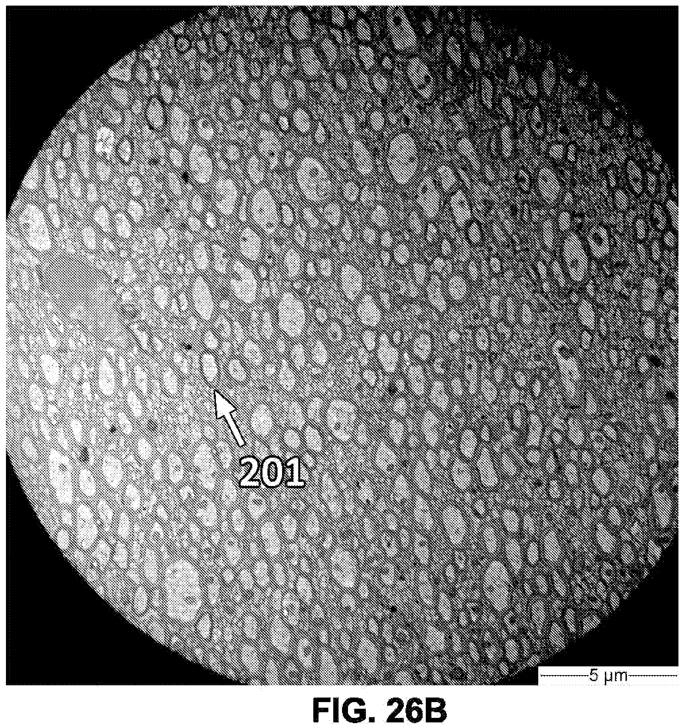

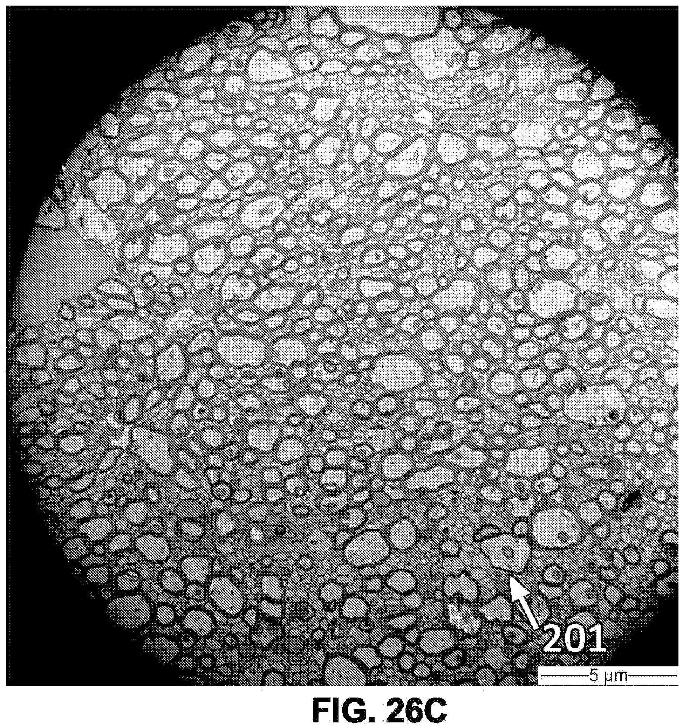

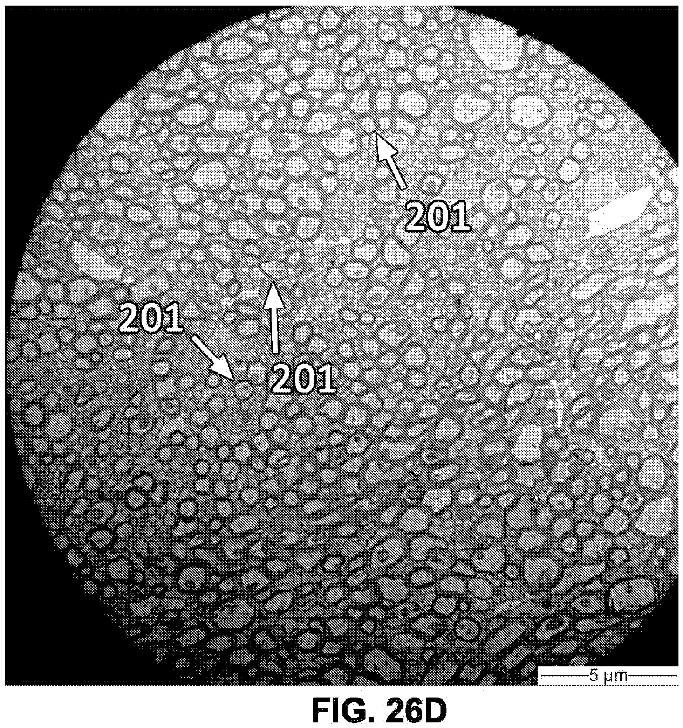

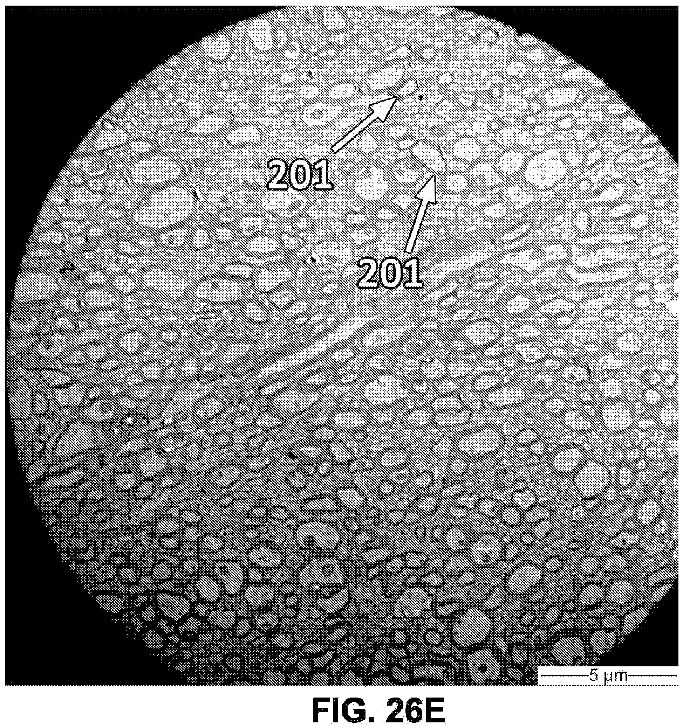

The TEM images corresponding to brains of mice from the prophylactic treatment Group 4 are shown in FIGs. 26A-26E, all images were originally taken at 4,000x. The mice in Group 4 were given Cuprizone Feed for 5 weeks and were gavaged once a day with 1000 ppm (1000 $\mu$g/ml) gold concentration present in CNM-Au8 nanosuspensions, in an amount of 10 ml of nanosuspension per kilogram of mouse weight (i.e., 10 ml/Kg). No areas of myelin damage, like those shown in FIGs. 25A-25G (i.e., Group 3), could be found in the Group 4 TEM images. In fact, the TEM images from Group 4 were somewhat similar to the TEM images from Control Group 1 (see FIGs. 23A-23C for comparison).

Further, the white arrows 201, present in each of FIGs. 26A-26E, correspond to axons that, in accordance with the literature, demonstrate the characteristics consistent with remyelination[8]. Specifically, these marked axons 201 show a thin and dark compact myelin sheath relative to other axons of similar or greater cross-sectional areas.

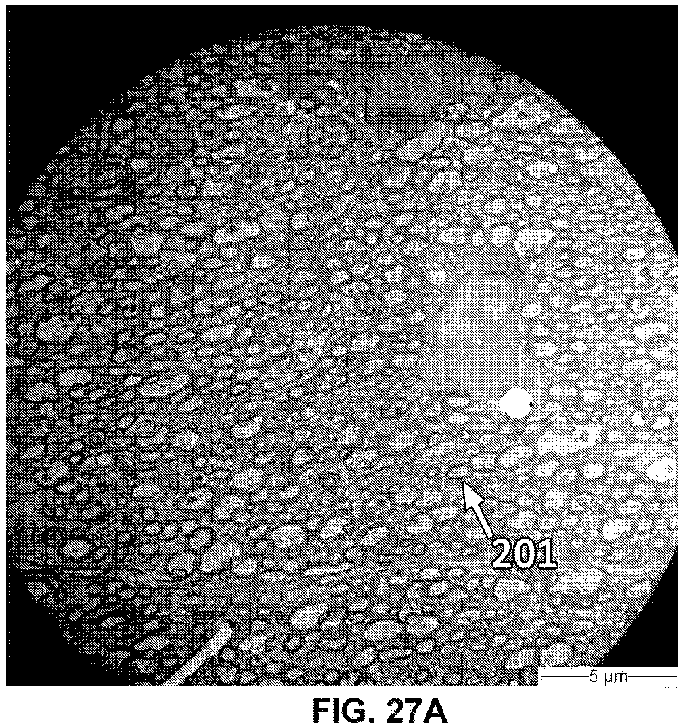

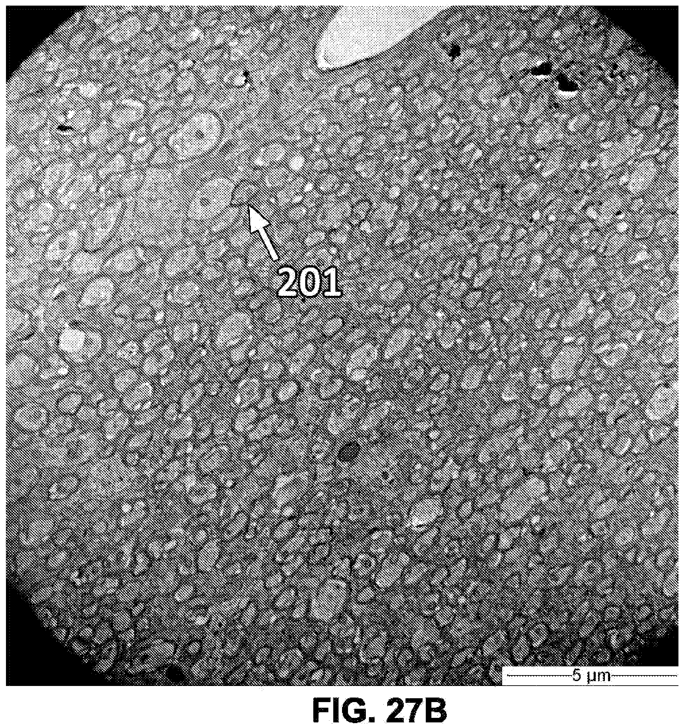

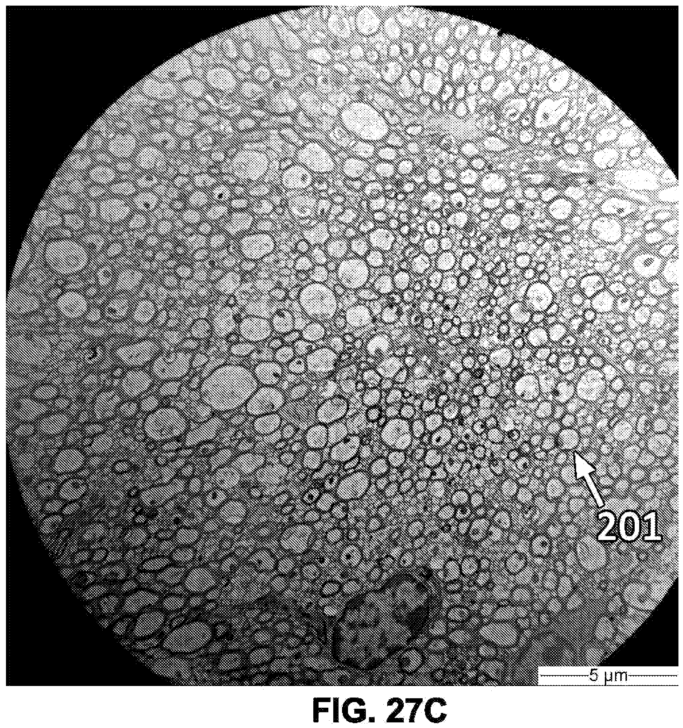

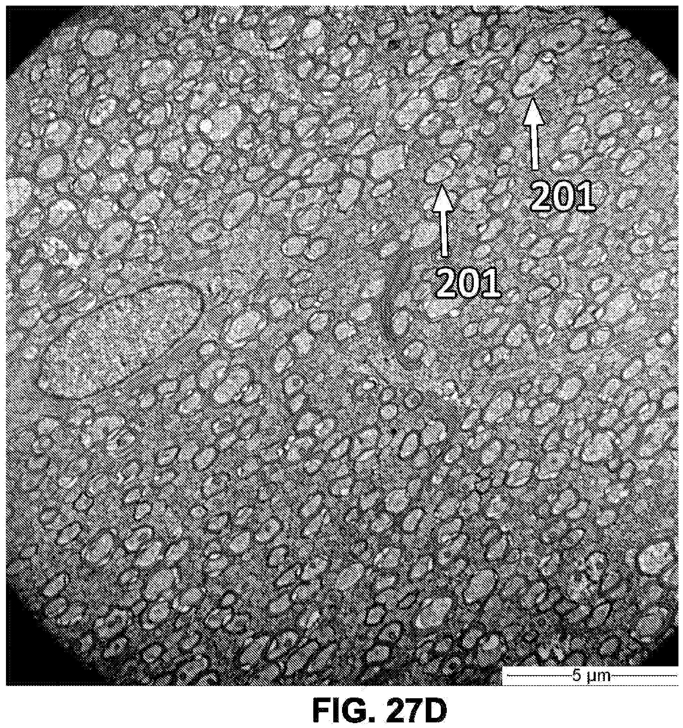

The TEM images corresponding to brains of mice from Group 5 mice are shown in FIGs. 27A-27D. These TEM images were originally taken at both 4,000x and 5,000x, as noted on the scale bars on the TEM images. These images, like those in FIGs. 26A-26E, also do not have any areas of extensive myelin damage like those demyelinated areas of the Group 3 mice (e.g., there are markedly reduced amounts of areas exhibiting extensive myelin damage or demyelination in the Group 5 mice). The Group 5 mice were fed Cuprizone Feed for 5 weeks and were given CNM-Au8 nanosuspensions by gavage, once per day, at a concentration of 1000 ppm (1000 $\mu$g/ml) and in an amount of 10 ml/Kg for weeks 3-5 of the study as a therapeutic treatment. Clearly, FIGs. 27A-27D show that the gavage of the aforementioned CNM-Au8 nanosuspensions had a therapeutic effect (e.g., a benefit) on the mice of Group 5, relative to the mice of Group 3.

Further, the white arrows 201, present in each of FIGs. 27A-27D, correspond to axons that, in accordance with the literature are believed to be remyelinated[8]. Specifically, these marked axons 201 show a thin and dark compact myelin sheath relative to other axons of similar or greater cross-sectional areas.

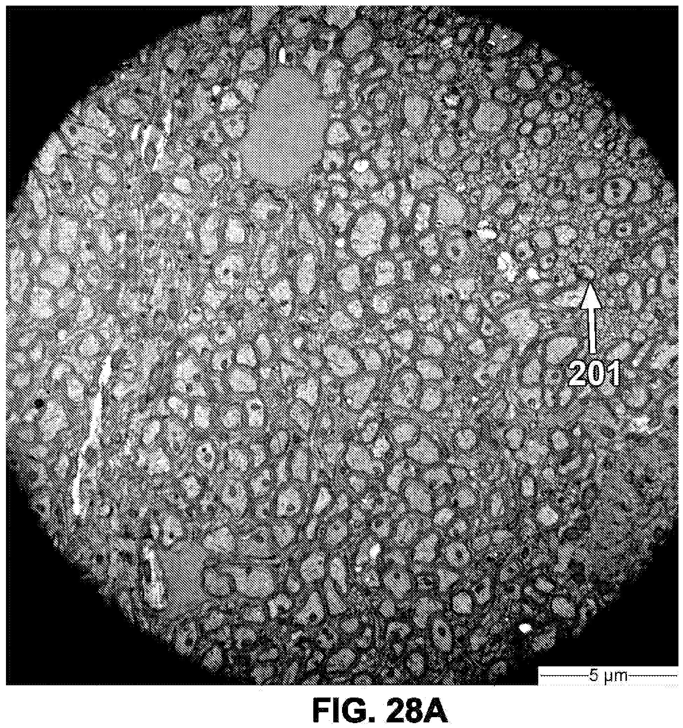

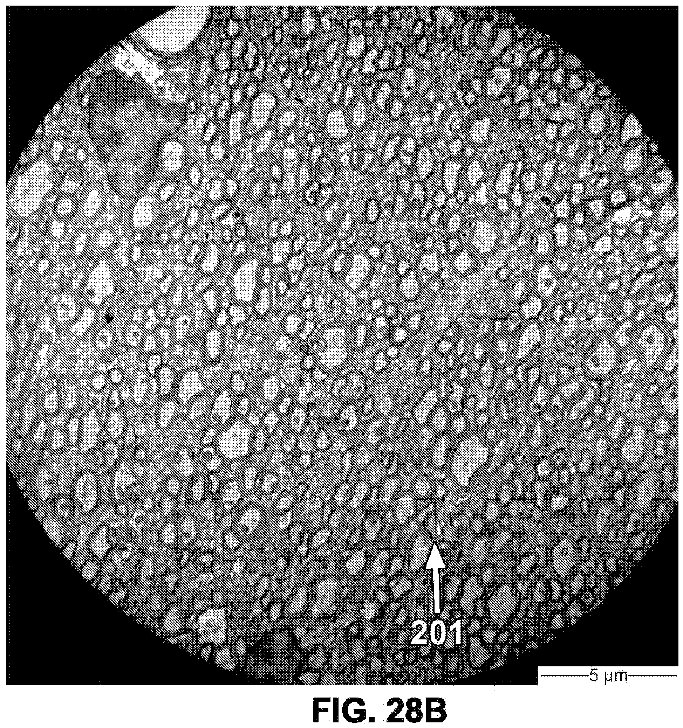

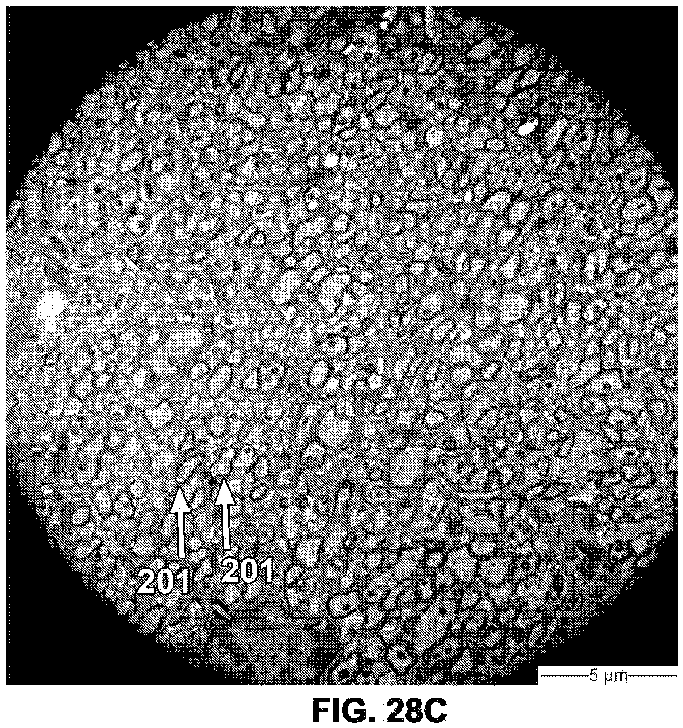

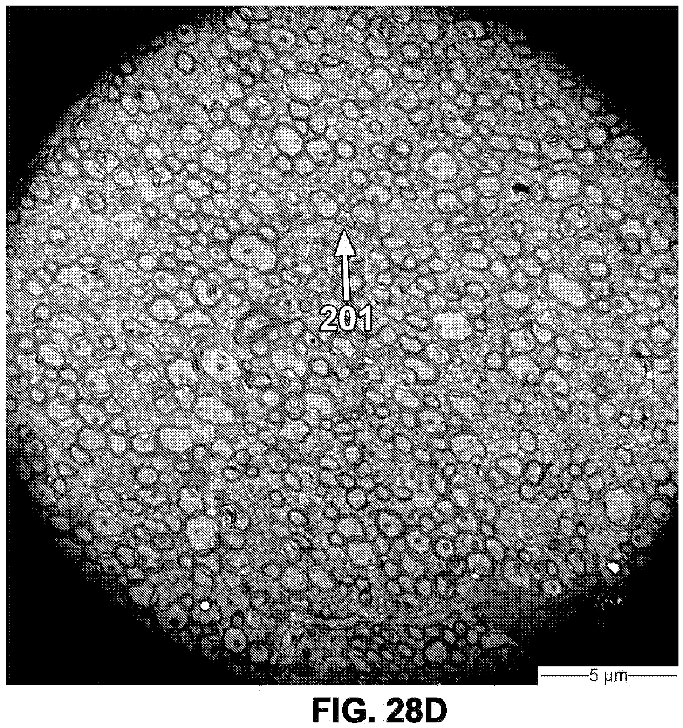

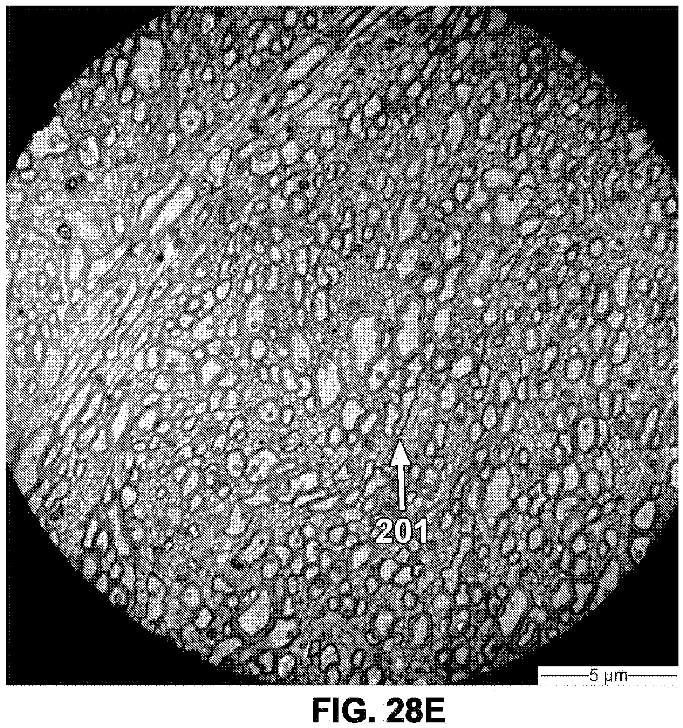

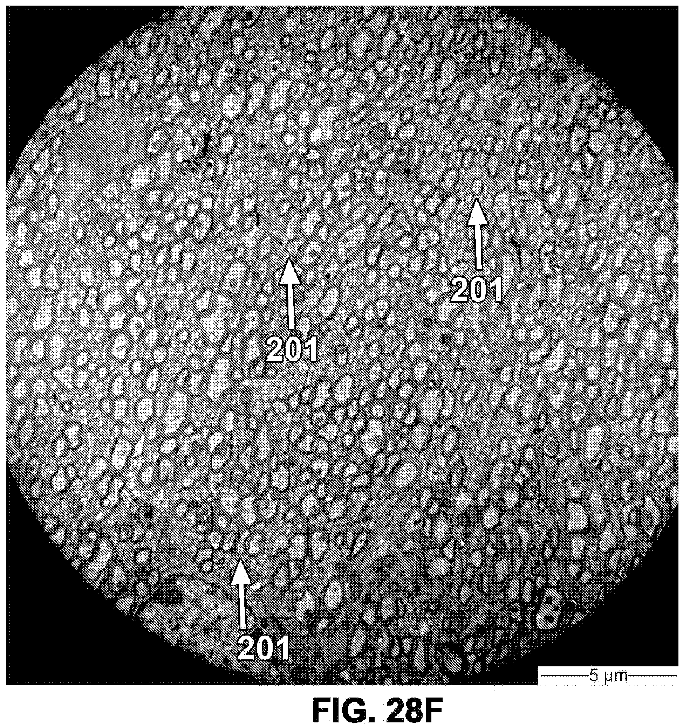

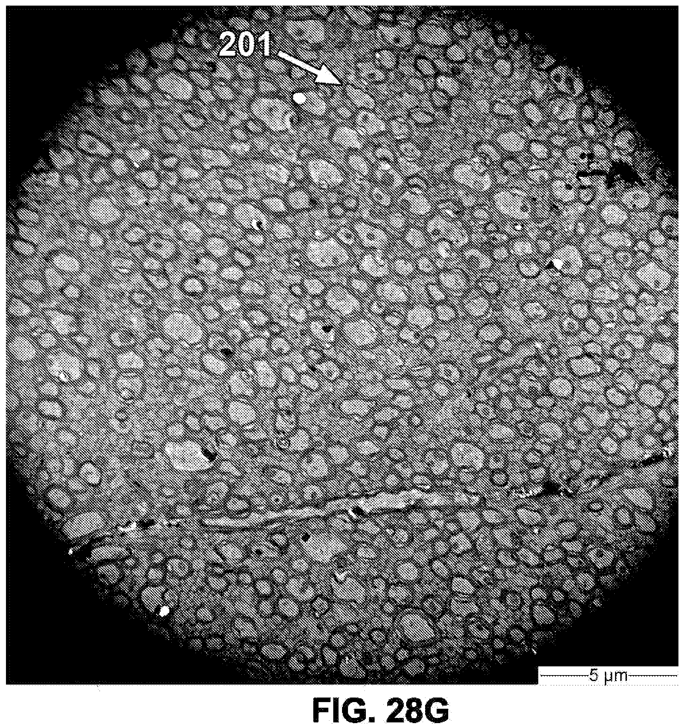

The TEM images corresponding to the brains of mice from Group 6 mice are shown in FIGs. 28A-28G. These TEM images were also originally taken at 4,000x, as noted on the scale bars on the images. These images, like those in FIGs. 26A-26E, also show markedly reduced amounts of areas or regions exhibiting extensive myelin damage or demyelination similar to those undesirable areas observed in the Group 3 mice. The Group 6 mice were fed Cuprizone Feed for 5 weeks and were given CNM-Au8 prophylactic nanosuspensions *ad libitum,* at a concentration of 50 ppm gold (50 $\mu$g/ml) for all 5 weeks of the study as a treatment. Clearly, the *ad libitum* exposure of CNM-Au8 nanosuspensions

at 50 μg/ml, had either or both of a prophylactic and/or therapeutic effect on the myelin for the mice of Group 6, relative to the myelin observed for the mice of Group 3.

Further, the white arrows 201, present in each of FIGs. 28A-28G, correspond to axons that, in accordance with the literature are believed to be remyelinated.[8] Specifically, these marked axons show a thin and dark compact myelin sheath relative to other axons of similar or greater cross-sectional areas.

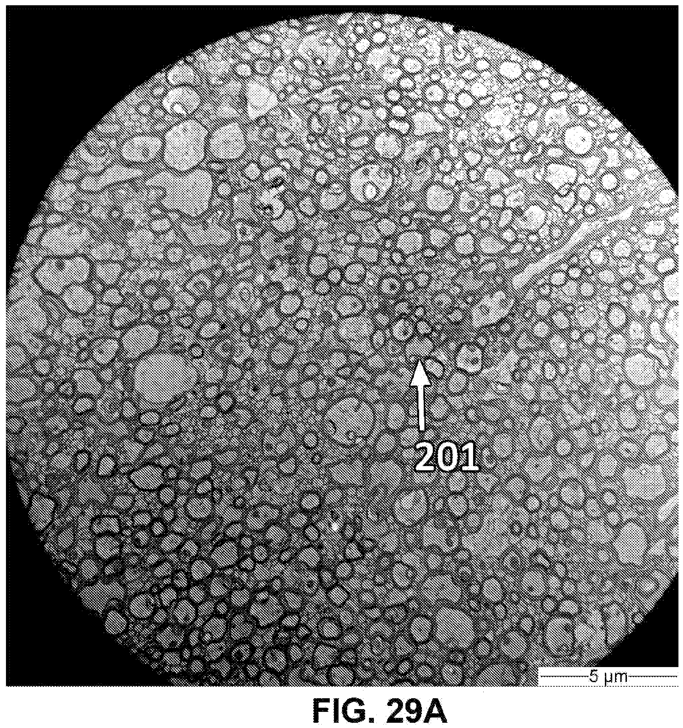

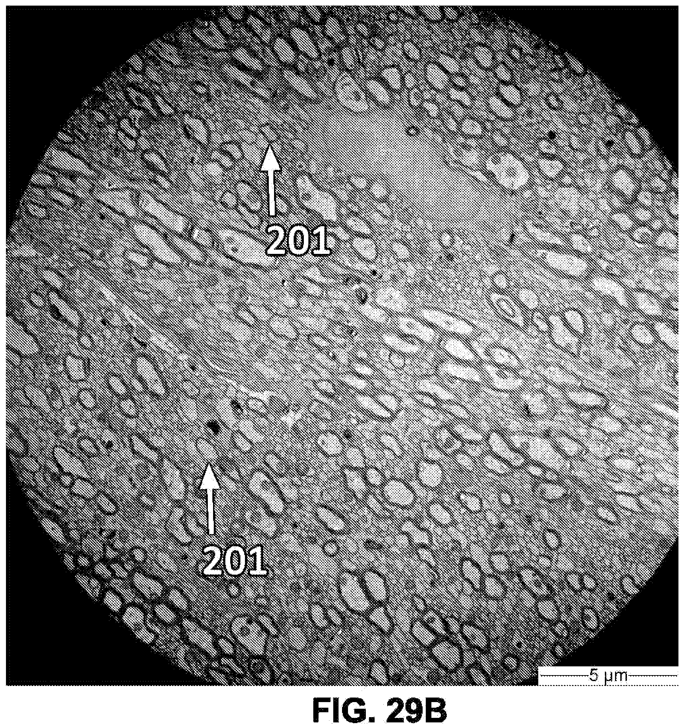

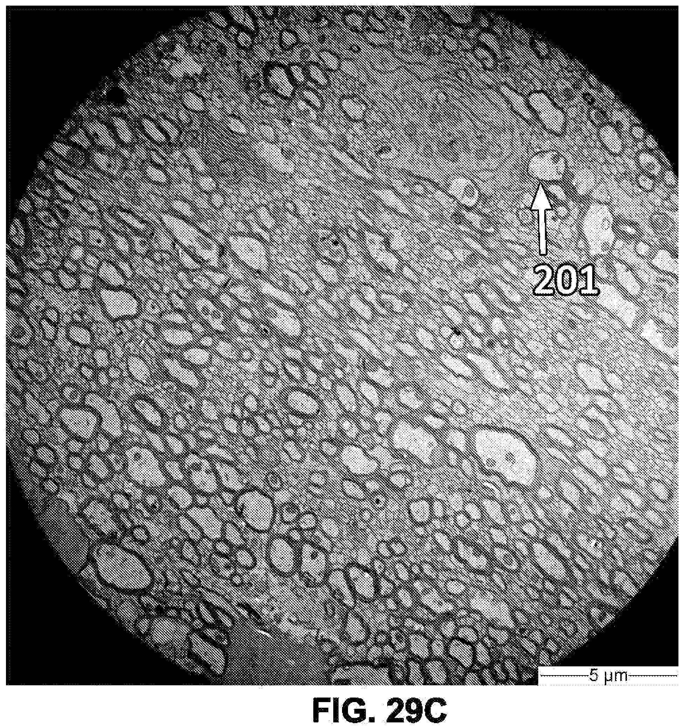

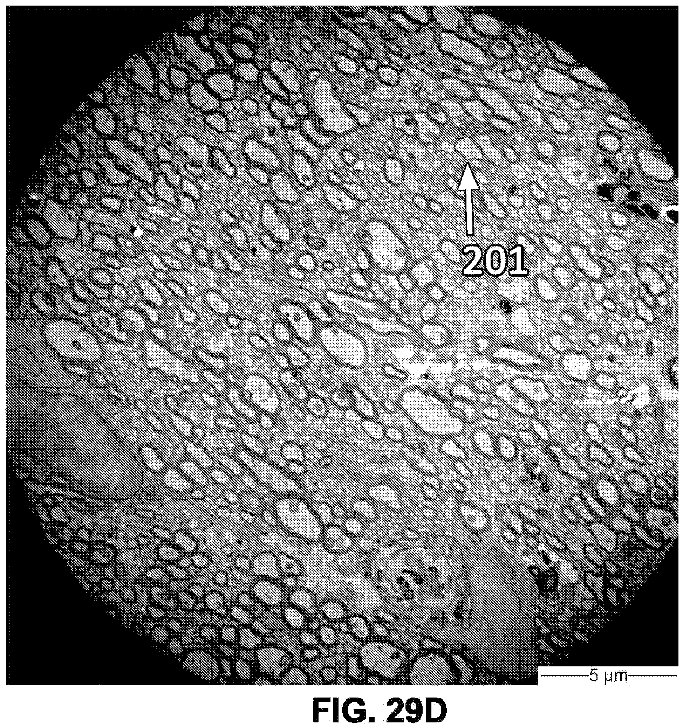

The TEM images corresponding to Group 7 mice are shown in FIGs. 29A-29D. These TEM images were originally taken at 4,000x, as noted on the scale bars on the images. These images, like those in FIGs. 26A-26E, also show markedly reduced amounts of areas exhibiting extensive myelin damage or demyelination similar to those undesirable areas in the Group 3 mice. The Group 7 mice were fed Cuprizone Feed for 5 weeks and were given CNM-Au8 treatment nanosuspensions *ad libitum,* at a concentration of 50 ppm gold (50 μg/ml) for weeks 3-5 of the study as a treatment. Clearly, the *ad libitum* exposure of CNM-Au8 nanosuspensions at 50 μg/ml, had either or both of a prophylactic and/or therapeutic effect on the mice of Group 7, relative to the mice of Group 3.

Further, the white arrows 201 present in each of FIGs. 29A-29D, correspond to axons that, in accordance with the literature are believed to be remyelinated.[8] Specifically, these marked axons show a thin and dark compact myelin sheath relative to other axons of similar or greater cross-sectioned areas.

These data suggest that Cuprizone Feed resulted in some demyelinated axons in the corpus callosum regions of mouse brains and that CNM-Au8 gold nanocrystal suspensions were an effective treatment (both therapeutic and prophylactic) for mammals to reduce the amount of demyelination of axons and/or result in remyelination of axons.

## II. Quantification of Number of Demyelinated Axons/Unit Area

Another method for determining if there are any positive treatment effects of CNM-Au8 gold nanocrystal suspensions on the amount of myelin damage due to Cuprizone Feed is to count the number of demyelinated axons, and/or clearly damaged myelin present on axons, and compare the number of demyelinated axons in each of mouse Groups 1-7. This approach is facilitated by observing a series of similar magnification (i.e., originally taken at 16,000x) TEM images taken randomly from the corpus callosum region of the mouse brains from mice in each of Groups 1-7. The study details for Groups 1-7 are set forth in Table 3, as previously discussed. This methodology seeks to distinguish demyelinated axons from unmyelinated axons and then compare the number of demyelinated axons (per unit area) in each mouse group.

Briefly, in this approach, the smallest fully myelinated axon (i.e., a fully myelinated axon with the smallest cross-sectional area) is identified in each TEM image with a white star numbered 203. This smallest fully myelinated axon 203 serves as a starting "Reference Axon" in each individual TEM photomicrograph. Then, all non-myelinated axons and/or demyelinated axons and/or axons that contain clearly damaged myelin that are of about the same cross-sectional area, or of a larger cross-sectional area, than the Reference Axon 203 are classified as being "demyelinated", and are then identified with an appropriate mark. The marked axons are then counted individually and added together, as discussed in more detail below.

The Reference Axon 203 size may change somewhat in each TEM photomicrograph because different portions of the non-homogenous corpus callosum can look somewhat different from each other. Once an average number of "demyelinated" axons has been determined for the Control Group 1, then that average number serves as a type of "background noise" and that average number is subtracted from the Weighted Average" number of demyelinated axons in each of Groups 2-7. It is believed that by using a Reference Axon approach, a more accurate understanding of the local corpus callosum neighborhood can be obtained resulting in a more accurate counting and representation of damaged or demyelinated axons.

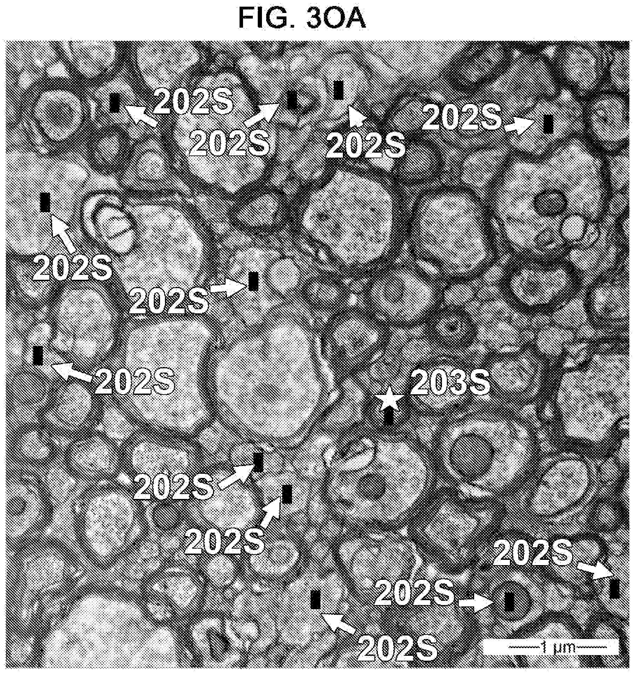

Specifically, FIG. 30A shows a representative TEM photomicrograph, originally taken at 16,000x, randomly taken from the corpus callosum region of a mouse from the Control Group 1. In this TEM photomicrograph, the smallest cross sectional area of a fully myelinated axon is represented by a white star and is numbered 203S. This Reference Axon 203S, identified by a skilled operator, becomes the Reference Axon in this FIG. 30A TEM photomicrograph.

Once this smallest, fully myelinated axon has been chosen by the skilled operator as the Reference Axon 203S in this TEM image, then the same skilled operator uses a touch screen computer screen, which displays the actual TEM image, and with a stylus, touches the screen and a rectangular black box is imposed on the portion of the screen (and thus imposed on the TEM photomicrograph) corresponding to the Reference Axon 203S. Once the Reference Axon 203S is identified, then every other axon that is judged by the skilled operator: (1) to be of the same cross-sectional area as the Reference Axon 203S and is not myelinated (i.e., is either lacking clearly defined myelin, or the myelin is clearly damaged), or (2) is of a larger cross-sectional area than the Reference Axon 203S and is not myelinated (i.e., is either lacking clearly defined myelin, or the myelin is clearly damaged) is marked with another black rectangular box (shown as black box in FIG. 30A) by touching the touch-screen display in the same manner. When the operator is finished marking all such (1) and (2) axons, a software program automatically counts all rectangular boxes on each photomicrograph (i.e., corresponding to all axons (1) and (2) marked by the skilled operator and judged by the skilled operator as being damaged). FIG. 30A also has white arrows numbered 202S imposed thereon pointing to each of the black boxes. These white arrows 202S have been added to assist the reader in identifying which axons (1) and (2) have been marked with the black boxes (i.e., those that have been identified by the skilled operator to be damaged). However, the white arrows are added manually after all the automatic totaling (e.g., counting of the number of damaged or demyelinated axons (1) and (2)) has occurred.

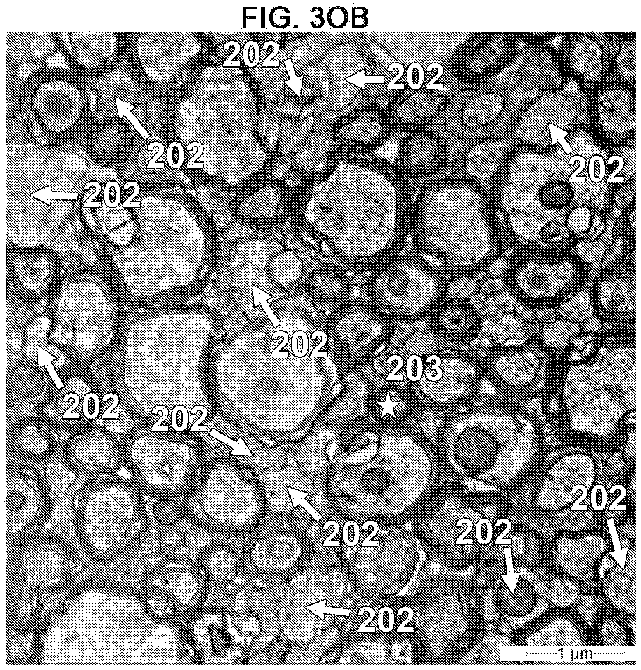

FIG. 30B is the same TEM photomicrograph shown in FIG. 30A, with the white star identifying the Reference Axon 203 and the white arrows 202 indicating those axons that once contained a black box, however, the black boxes have been removed and only the white arrows 202 remain. In all of the remaining TEM photomicrographs shown in FIG. 30B - FIG. 36B, all of the black boxes have been removed. However, it should be understood that the white arrows 202, while manually added later, correspond to those axons (1) and (2) judged by the skilled operator to be damaged, as determined by reference to a different Reference Axon 203 in each TEM photomicrograph.

Thus, FIG. 30B shows a representative cross section of the corpus callosum of a mouse in Control Group 1. FIG. 30B shows a single white star 203 as the Reference Axon and 12 white arrows 202 corresponding to those demyelinated axons (1) and (2), as determined by the skilled operator to be damaged.

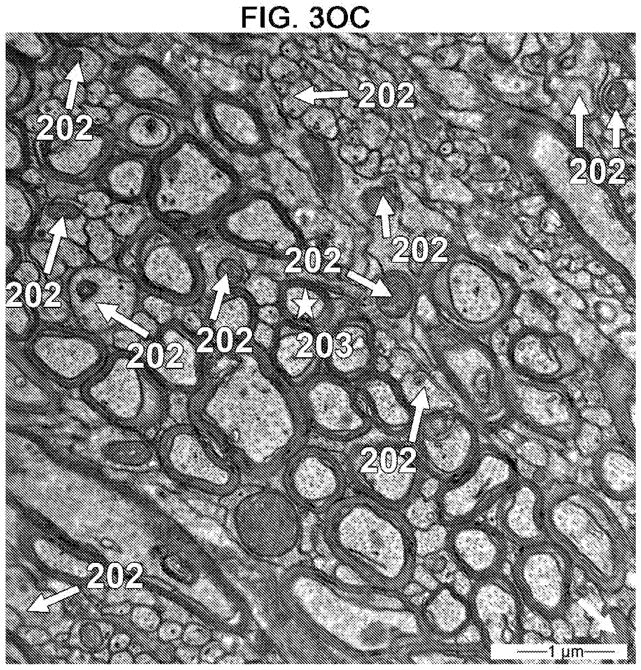

Similarly, FIG. 30C corresponds to another representative cross section of the corpus callosum of a mouse in Control Group 1. In this case, the Reference Axon 203 is also denoted in the same way with a white star 203 and each of those demyelinated axons (1) and (2) are also designated with a white arrow numbered 202. FIG. 30C shows 11 demyelinated axons.

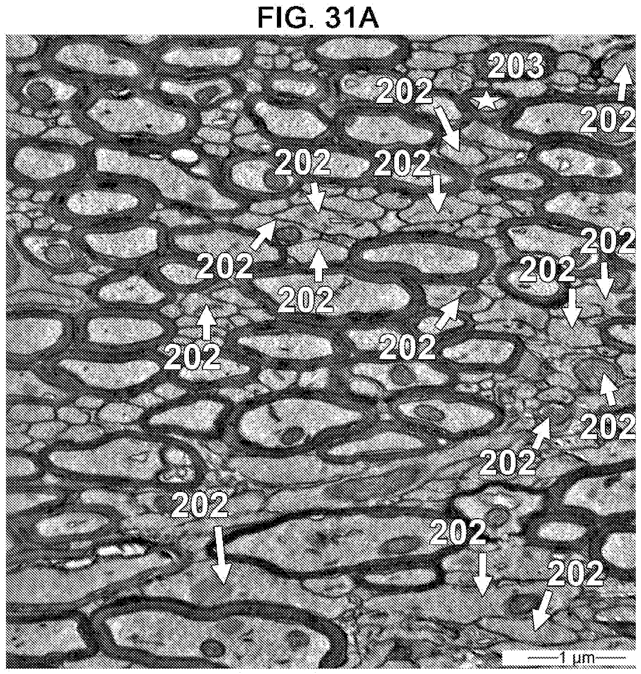

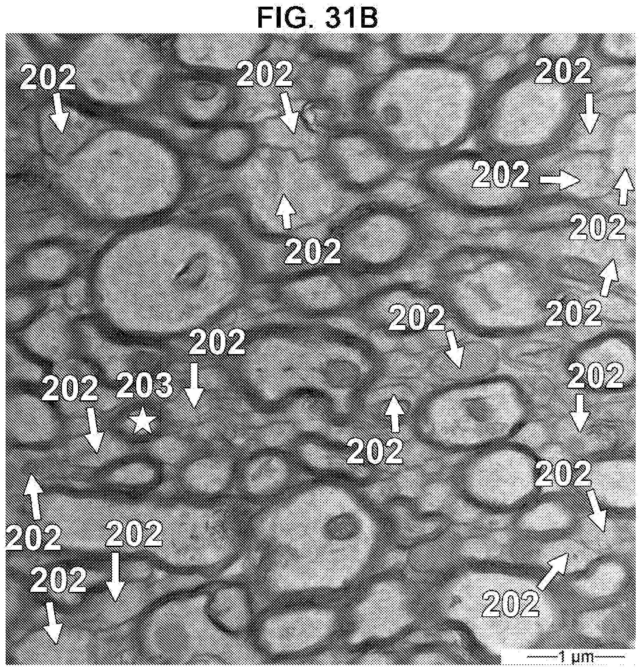

Likewise, FIGs. 31A and 31B show two representative TEM photomicrographs, also taken at 16,000X, taken from representative mice in Group 2, and also identifying a similar Reference Axon 203 in each TEM image with a white star and 15 demyelinated axons 202 in FIG. 31A; and 17 demyelinated axons 202 in FIG. 31B.

In a similar manner, mice from each of the Groups 3-7 are also represented by two similar representative TEM photomicrographs of the corpus callosum taken from mice in each of these groups.

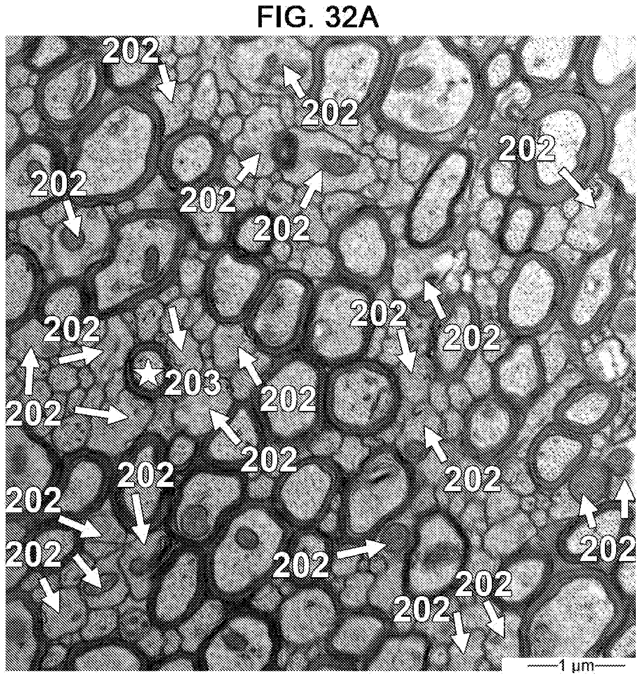

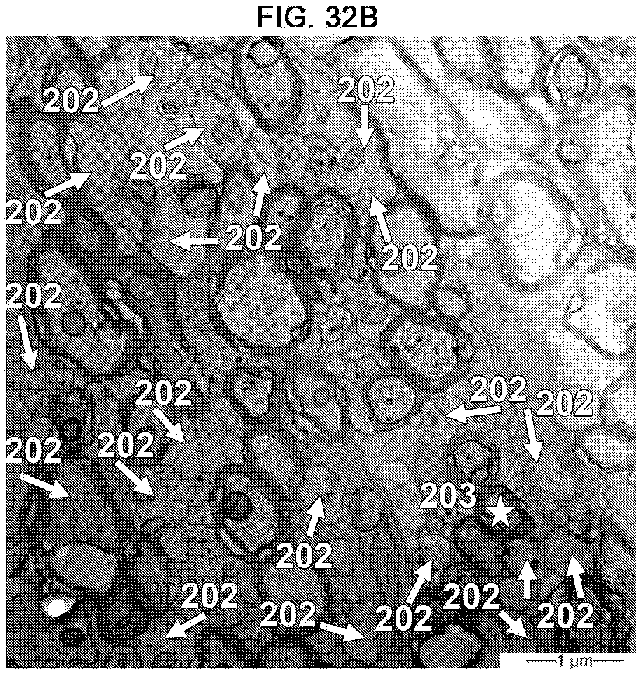

FIGs. 32A and 32B correspond to representative TEM images of the corpus callosum of mice from Group 3, showing the Reference Axon 203 and 23 demyelinated axons 202; and 20 demyelinated axons 202, respectively.

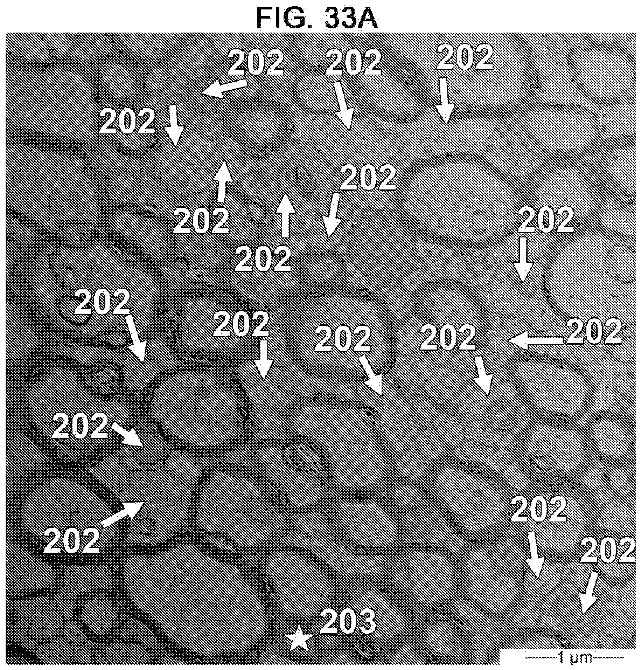

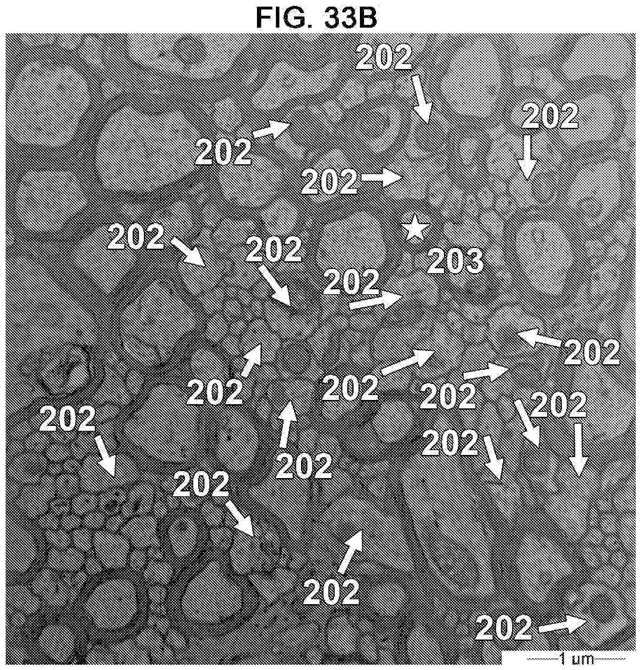

FIGs. 33A and 33B correspond to representative TEM images of the corpus callosum of mice from Group 4, showing the Reference Axon 203 and 17 demyelinated axons 202; and 19 demyelinated axons 202, respectively.

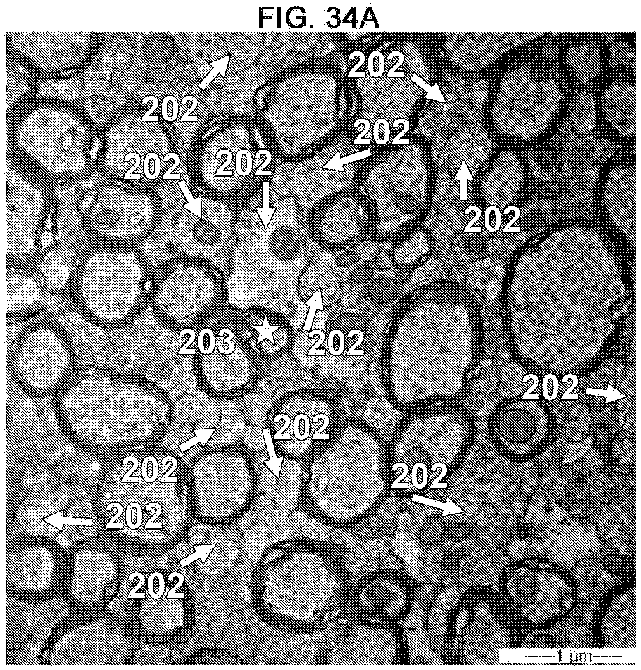

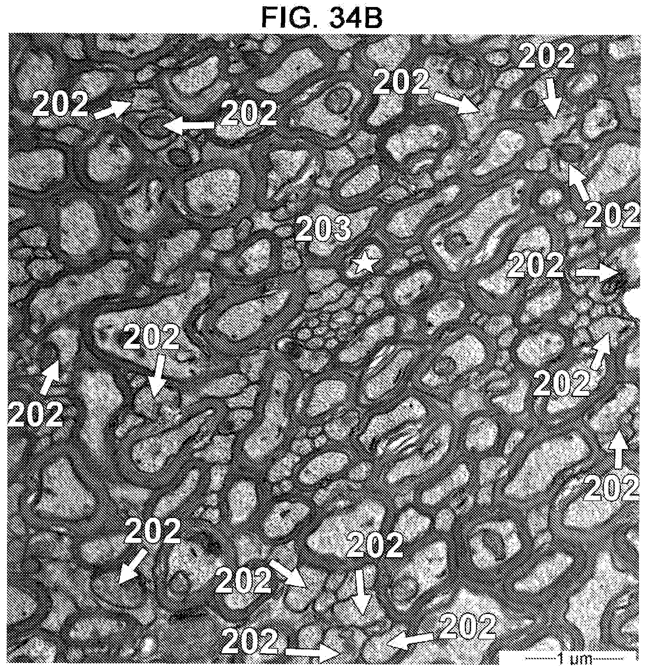

FIGs. 34A and 34B correspond to representative TEM images of the corpus callosum of mice from Group 5, showing the Reference Axon 203 and 13 demyelinated axons 202; and 15 demyelinated axons 202, respectively.

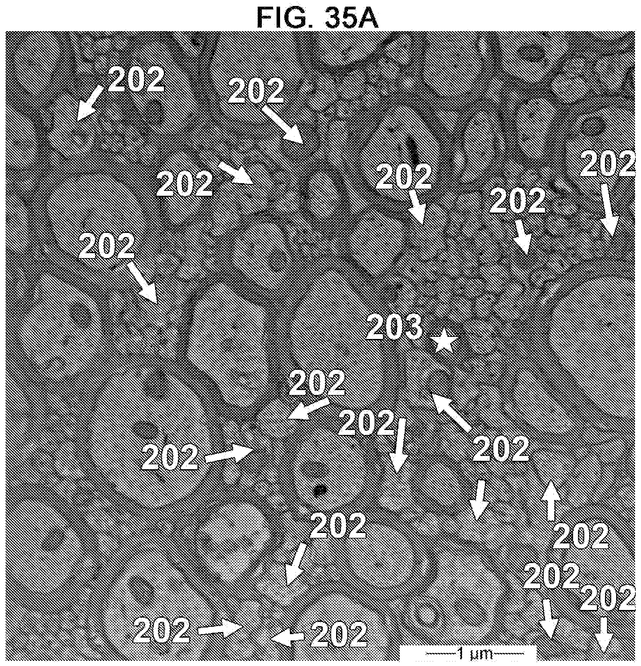

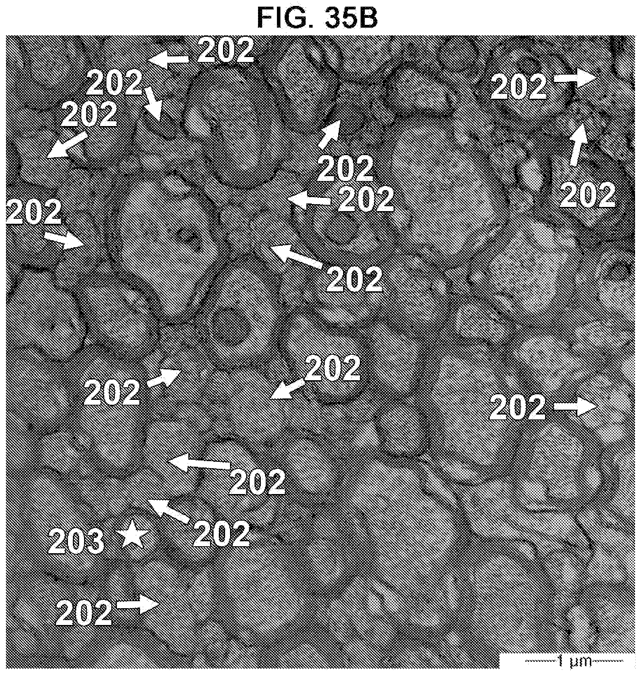

FIGs. 35A and 35B correspond to representative TEM images of the corpus callosum of mice from Group 6, showing the Reference Axon 203 and 18 demyelinated axons 202; and 15 demyelinated axons 202, respectively.

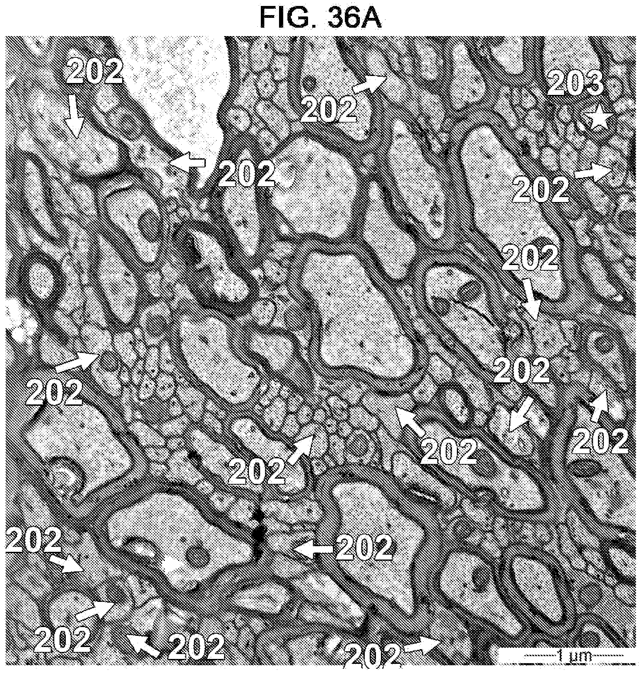

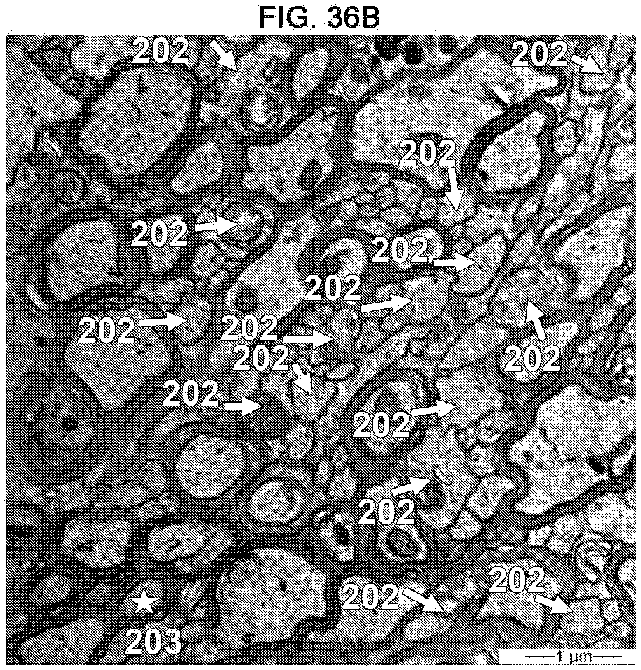

FIGs. 36A and 36B correspond to representative TEM images of the corpus callosum of mice from Group 7, showing the Reference Axon 203 and 15 demyelinated axons 202; and 14 demyelinated axons 202, respectively.

**Table 4**

| Group Number | Total Number of Photomicrographs Examined | Total Number of Demyelinated Axons Counted | Weighted Average of Counted Demyelinated Axons Per Photomicrograph | Adjusted Weighted Average of Demyelinated Axons Per Photomicrograph |
|---|---|---|---|---|
| 1 | 45 | 419 | 10 | 0 |
| 2 | 40 | 451 | 16 | 6 |
| 3 | 44 | 742 | 21 | 11 |
| 4 | 34 | 630 | 19 | 9 |
| 5 | 40 | 364 | 17 | 7 |
| 6 | 40 | 588 | 15 | 5 |
| 7 | 70 | 1007 | 15 | 5 |

Table 4 shows in summary form, the total number of TEM photomicrographs similar, to those representative photomicrographs shown in FIGs. 30-36, that were examined in a similar manner. In this regard, for example, 45 total TEM photomicrographs were examined for mice in Group 1. Further, of those 45 TEM photomicrographs examined for Group 1, a total of 419 demyelinated axons were counted. The fourth column in Table 4 lists the "Weighted Average of Counted Demyelinated Axons Per Photomicrograph" and lists that there were "10" for Group 1. It should be noted that the weighted average was achieved as follows.

Within each of Groups 1-7, representative corpus callosum samples from each mouse were photographed in multiple locations. For each sample of corpus callosum, the "Total Number of Demyelinated Axons Counted" in all the photomicrographs was summed and the average number of demyelinated axons per photomicrograph for each sample was determined. (results not shown). Due to variability in some perfusion steps, some corpus callosum samples had a larger number of better quality photomicrographs that could be used for counting. Therefore, the average number of demyelinated axons for each sample of corpus callosum was assigned a weight in accordance with the quality of that sample's photomicrograph set. The weights were determined as follows. For each sample of corpus callosum from a mouse group, the number of demyelinated axons identified for that sample was divided by the total number of demyelinated axons identified for that group. This is the sample weight. Each sample weight was multiplied by the sample average of demyelinated axons per micrograph. These weighted sample averages were summed over each group and reported as the "Weighted Average Counted Demyelinated Axons per Photomicrograph" in Table 4.

The final column in Table 4 lists the "Adjusted Weighted Average of Demyelinated Axons per Photomicrograph". Those numbers were determined by subtracting "10" from the previous column, with "10" effectively corresponding to "background noise" in the photomicrographs.

Accordingly, the highest number for the "Adjusted Weighted Average of Demyelinated Axons per Photomicrograph" occurs in Group 3, whereas the lowest number for the "Adjusted Weighted Average of Demyelinated Axons per Photomicrograph" occurs in Group 1 (i.e., the Control Group).

In sum, 313 total TEM photomicrographs of representative portions of the corpus callosum were reviewed for a varying number of mice in each of Groups 1-7, resulting in a total number of demyelinated axons counted of about 4,200. Table 2 reports the weighted average of demyelinated axons counted for each mouse group.

It should be understood that the determination of the total number of "demyelinated" axons per TEM photomicrograph was performed in a manner that was intended to be as objective as possible. In this regard, randomly selected portions of the corpus callosum were separately photomicrographed. Those photomicrographs (all originally taken at 16,000X) that provided a clear enough distinction between axons were then candidate photomicrographs for counting "demyelinated"" axons. It is noted that some of the mouse brains did not undergo complete perfusion during the sample preparation steps which caused some of the TEM images to be blurry or contain unacceptable artifacts. Once all of the randomly selected photomicrographs that were, for example, too blurry to read, and/or or contained too many artifacts were eliminated, then every remaining TEM photomicrograph was analyzed, as discussed above, and is summarized in Table 4.

Thus, each of the CNM-Au8 gold nanocrystal suspensions used for the mice in each of Groups 4-7, resulted in (i) an "Adjusted Weighted Average of Demyelinated Axons Per Photomicrograph" to be less than the "Adjusted Weighted Average of Demyelinated Axons Per Photomicrograph" of the mice in Group 3; and (ii) an "Adjusted Weighted Average of Demyelinated Axons Per Photomicrograph" to be more than the "Adjusted Weighted Average of Demyelinated Axons Per Photomicrograph" of the mice in Control Group 1.

These data suggest that Cuprizone Feed resulted in some demyelinated axons in the corpus callosum regions of mouse brains and that CNM-Au8 gold nanocrystal suspensions were an effective treatment (both therapeutic and prophylactic) for mammals to reduce the amount of demyelination of axons.

### III. Remyelination of Axons Shown in Images Taken at 16,000x and 40,000x

Another objective method for determining if there are any positive treatment effects of CNM-Au8 gold nanocrystal suspensions on the amount of myelin damage due to Cuprizone Feed is to determine if any remyelination can be observed in the corpus callosum regions of brains of mice in each of Groups 1-7. The study details for Groups 1-7 are set forth in Table 3, as previously discussed.

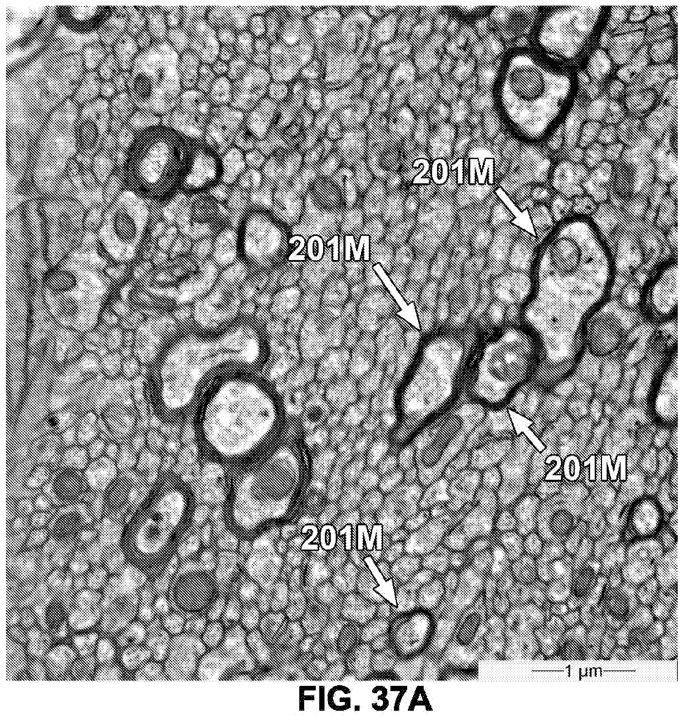

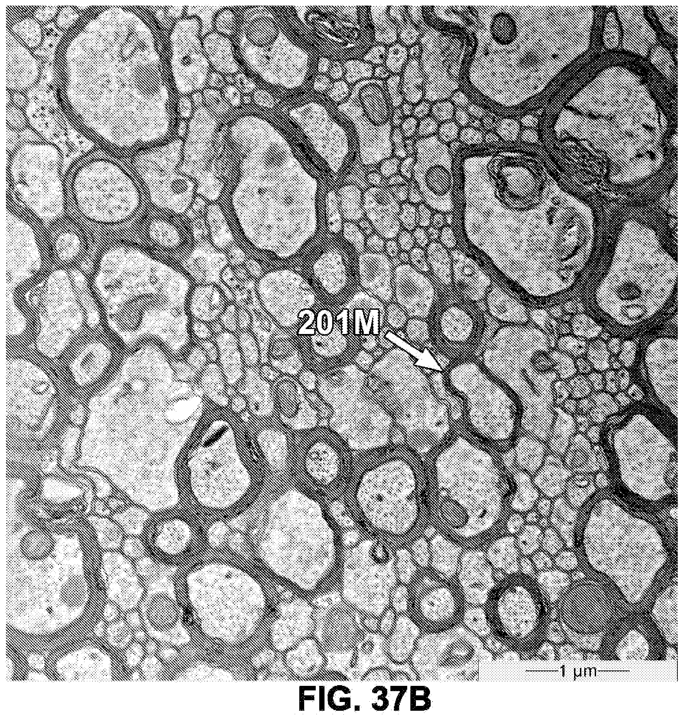

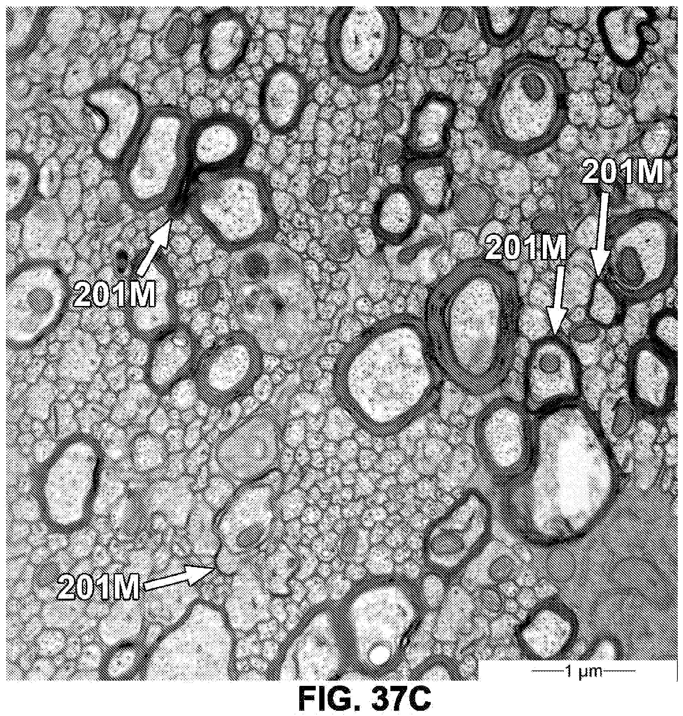

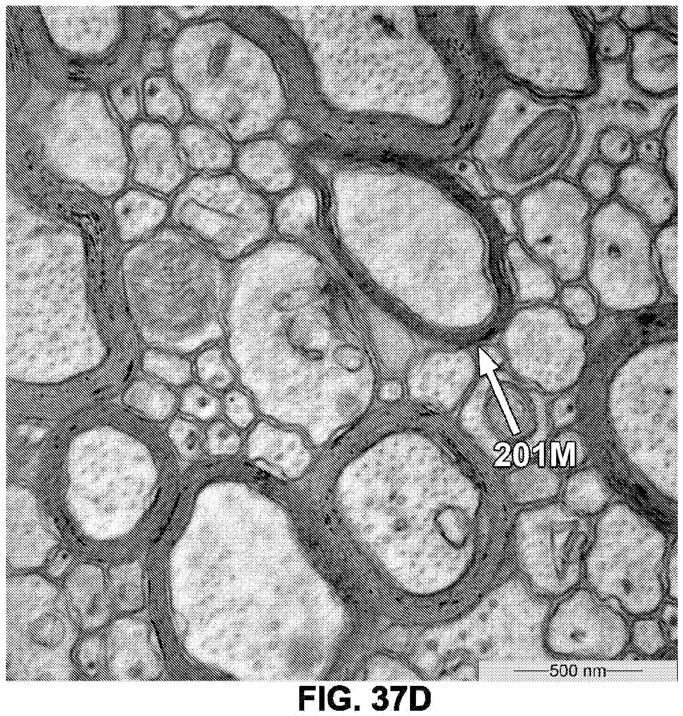

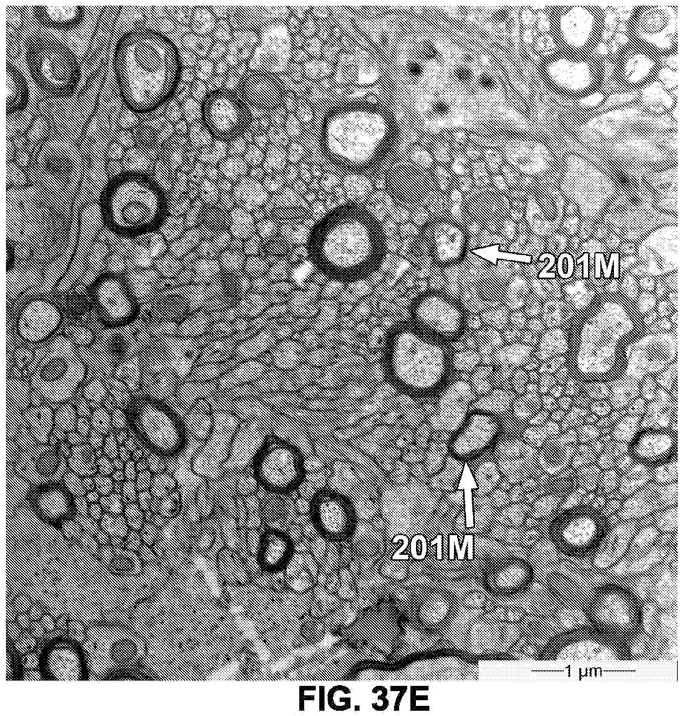

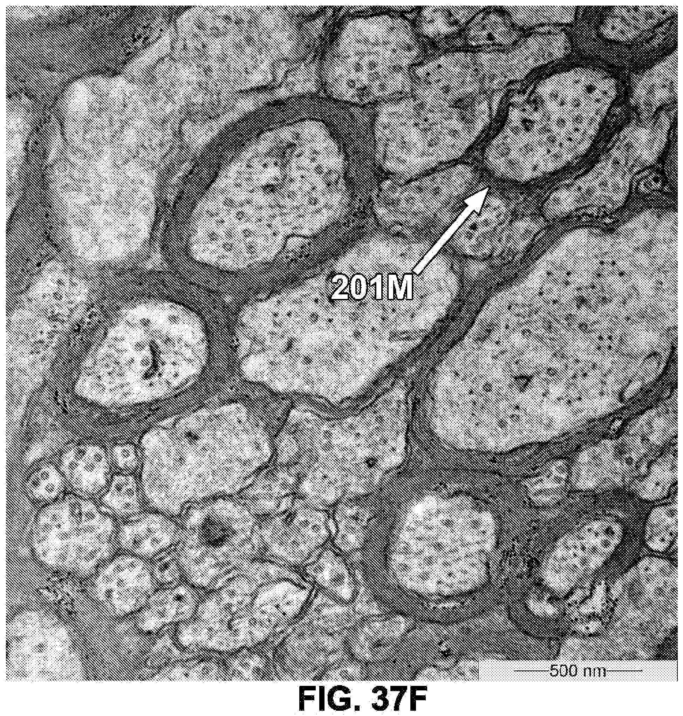

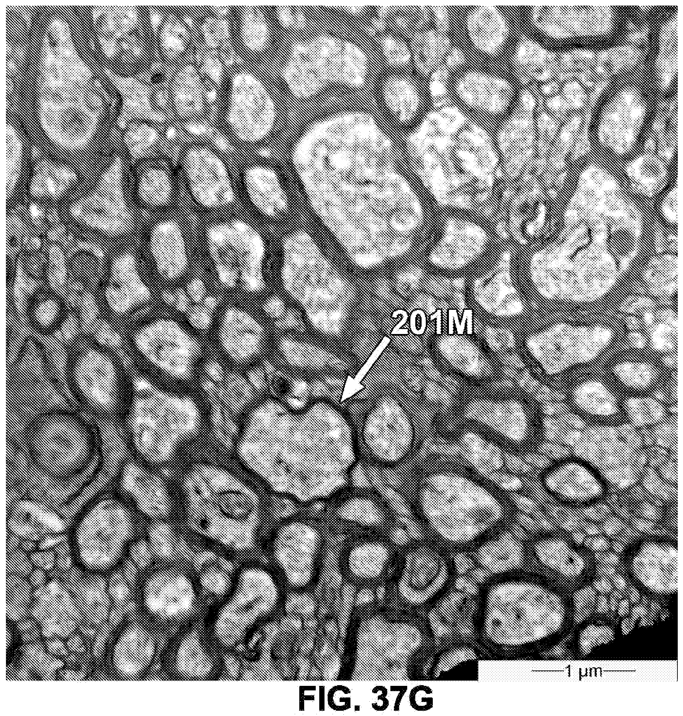

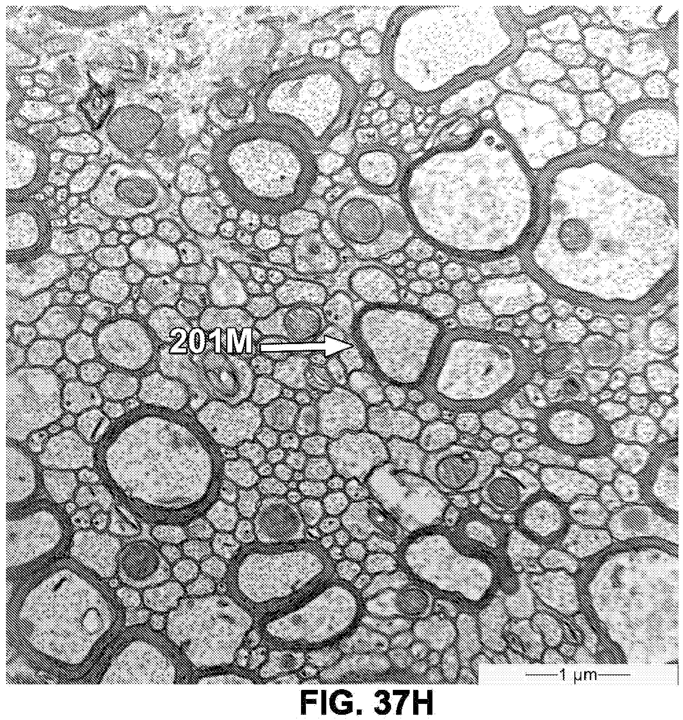

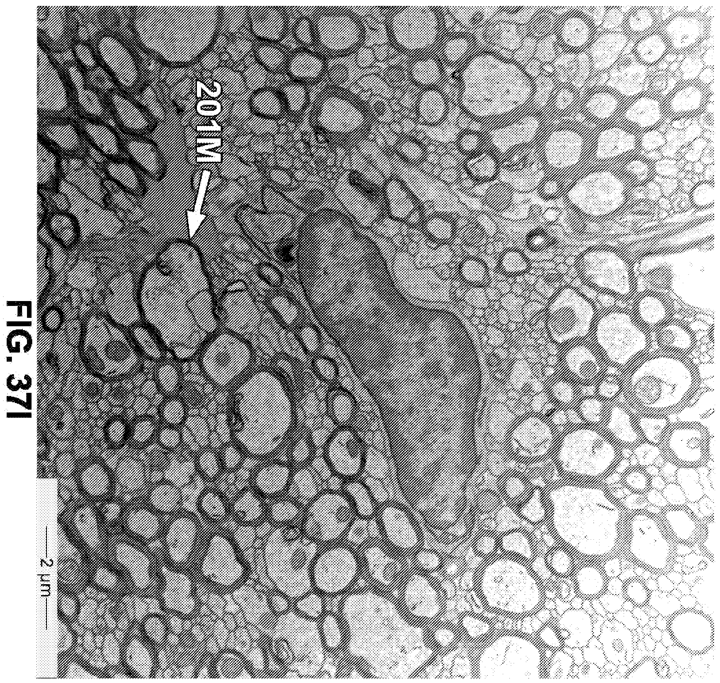

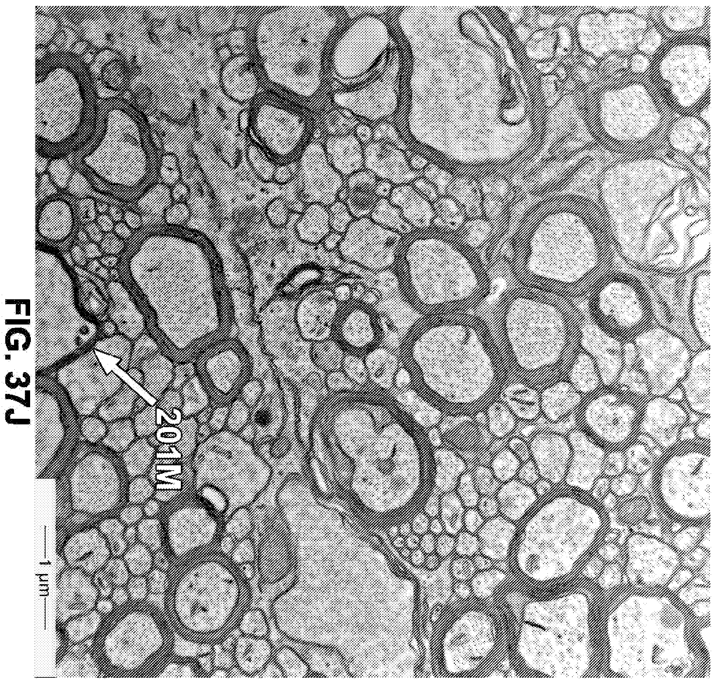

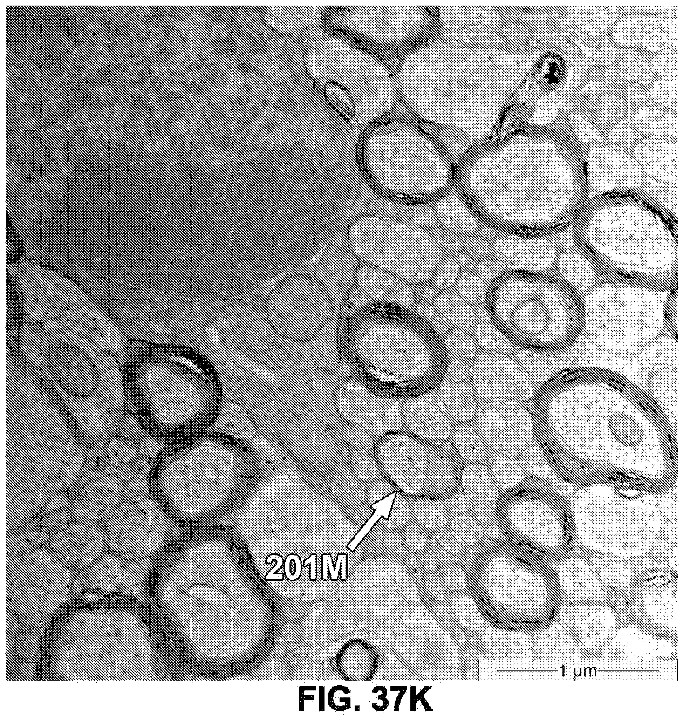

In this regard, FIGs. 37-40 show representative TEM photomicrographs taken at either 16,000x or 40,000x, as noted by the scale bar on each photomicrograph, of representative regions of the corpus callosum of mice in Groups 4-7. It is noted that the representative TEM images show only prophylactic treatment groups 4 and 6, and therapeutic treatment groups 5 and 7, because axons similar to those axons designated "201M" on the TEM images were not observed in the corpus callosum portions of mice in Groups 1-3. Specifically, the arrows 201M point toward thin, dark and compact myelinated areas which, in accordance with the literature are believed to be remyelinated axons[8]. Similar thin, dark and compact regions on axons were not found in representative photomicrographs corresponding to mice in Groups 1-3.

FIGs. 37A-37K correspond to TEM images from mice in Group 4, taken at both 16,000x and 40,000x. These FIGs. show a number of arrows 201M. These arrows 201M are directed toward what the literature regards as remyelinated axons[8]. It should be understood that the darker myelin regions are not artifacts of the sample preparation or TEM imaging process because nearby or neighborhood axons can be used as reference points and these neighborhood axons do not have darker myelin regions.

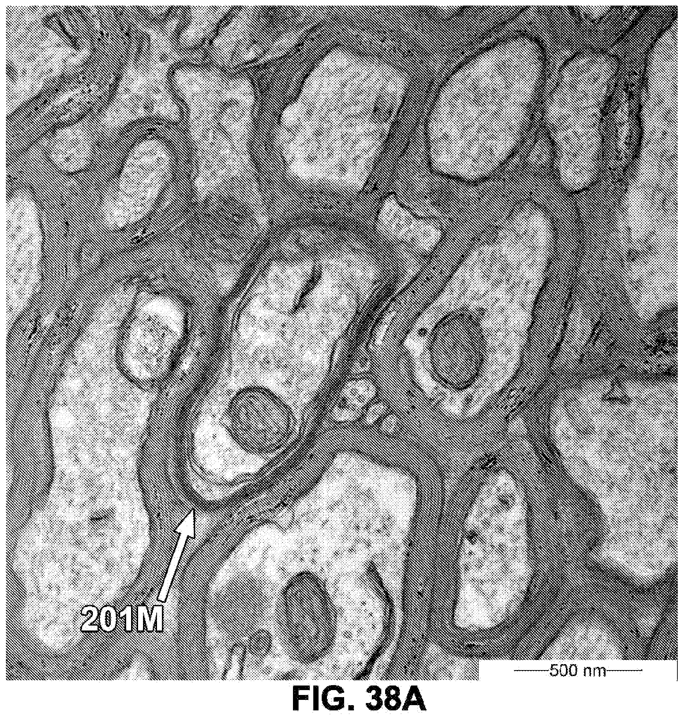

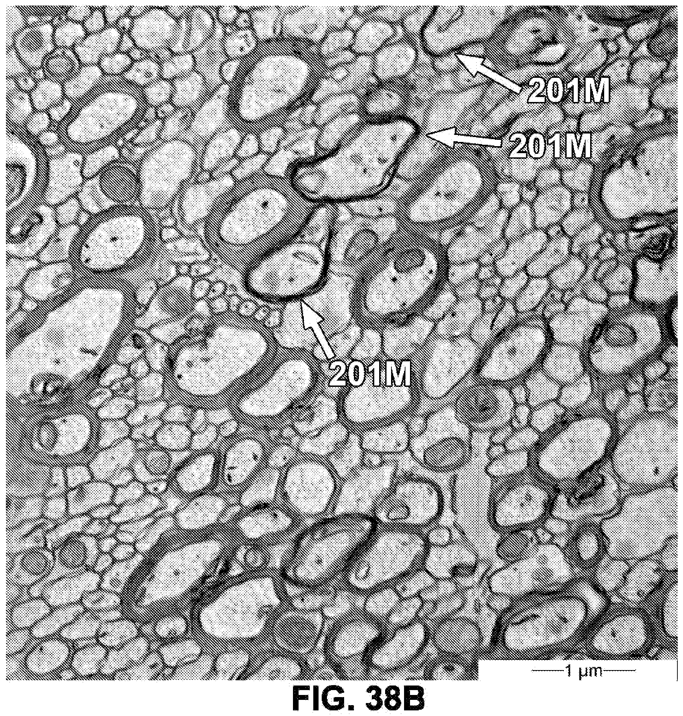

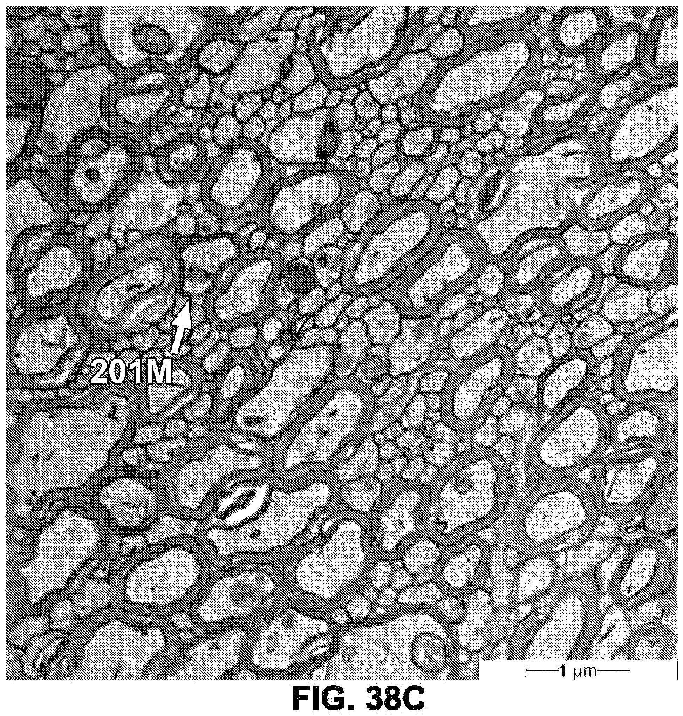

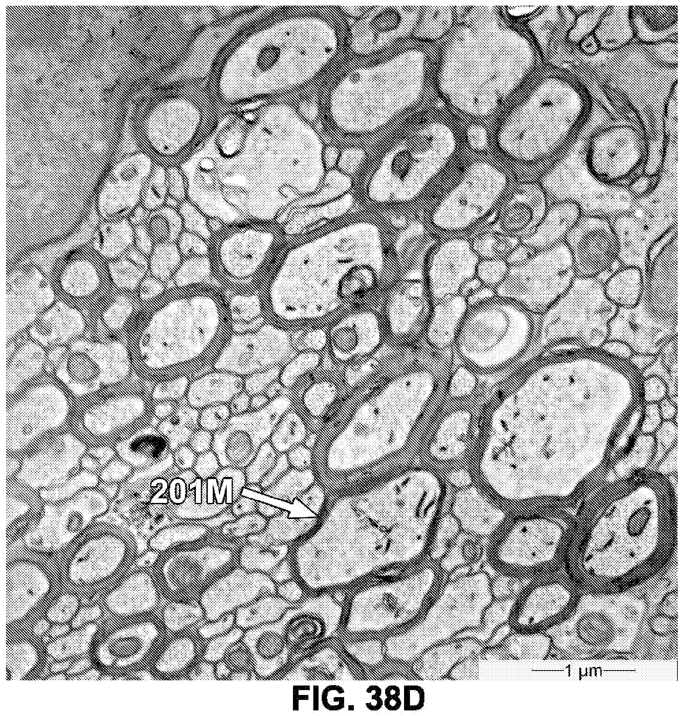

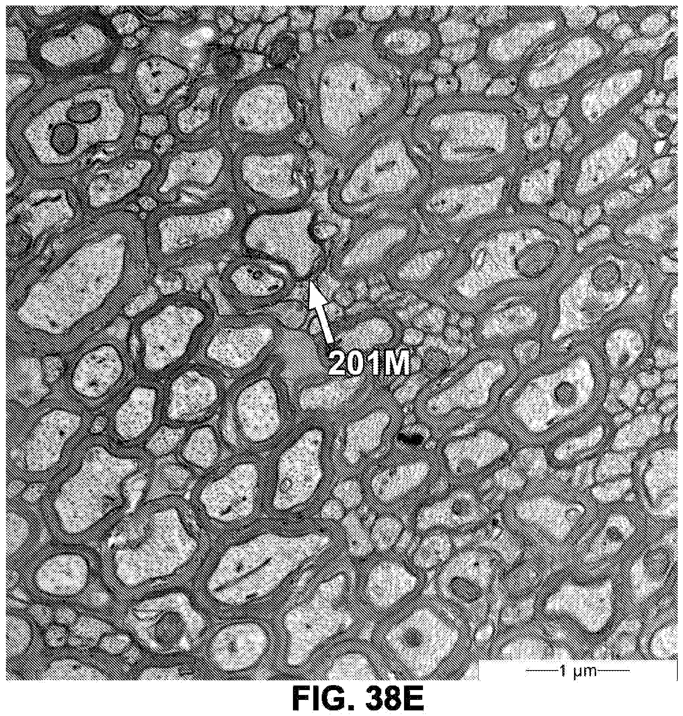

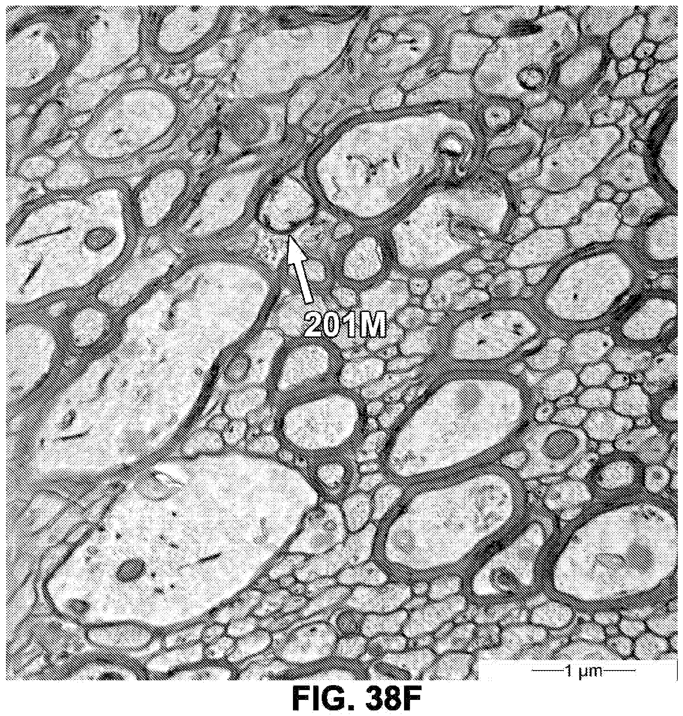

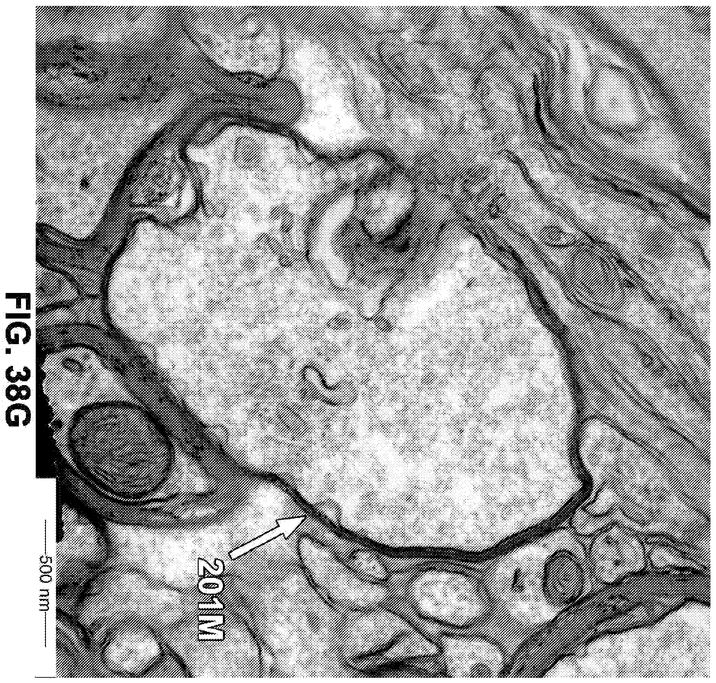

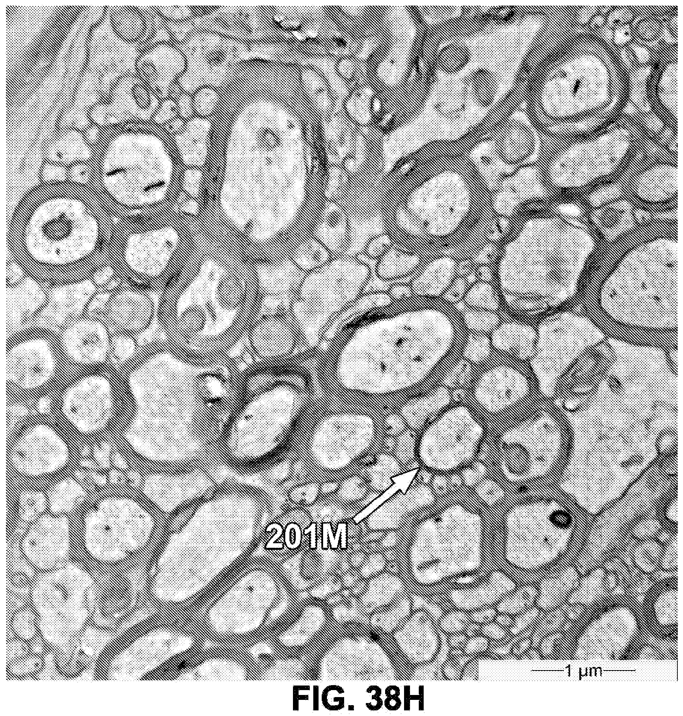

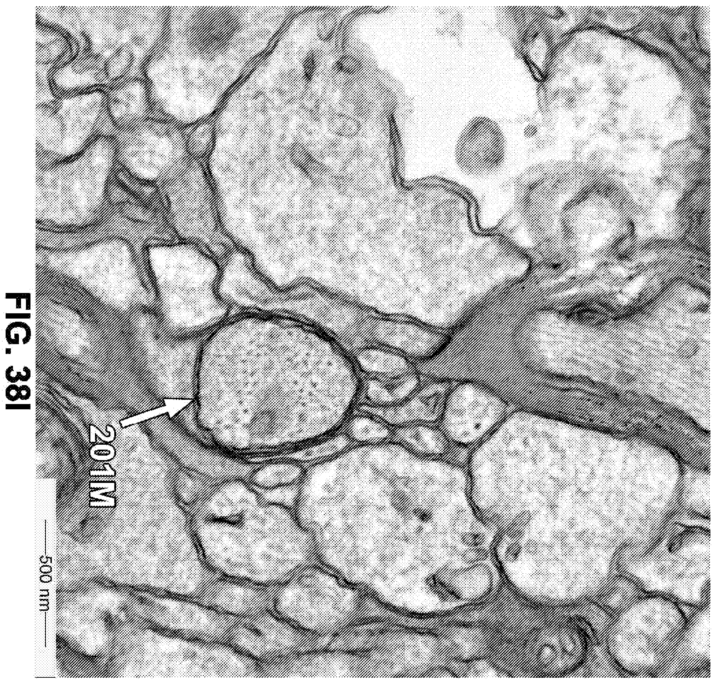

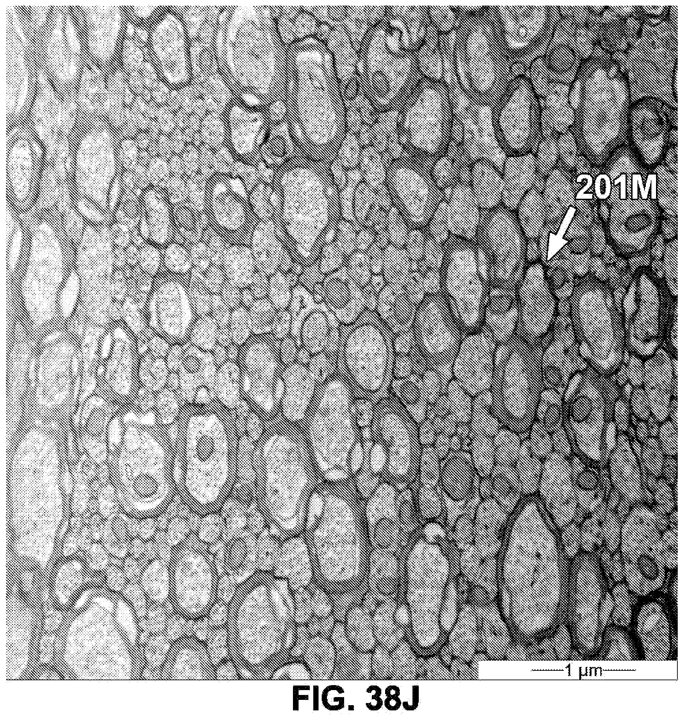

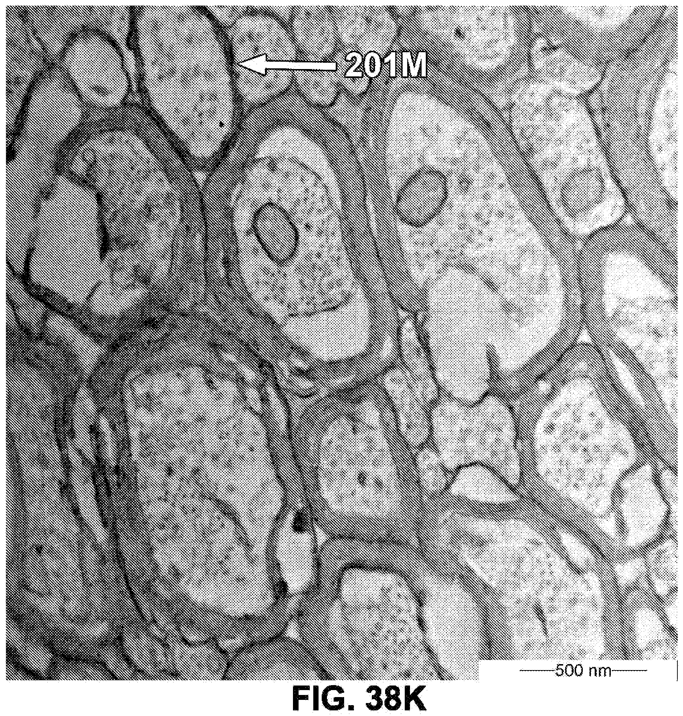

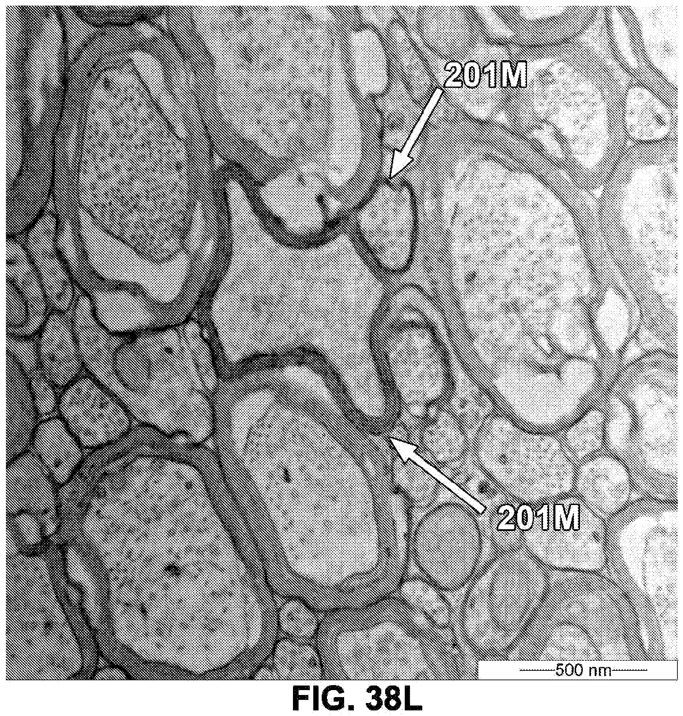

FIGs. 38A-38L correspond to TEM images from mice in Group 5, taken at both 16,000x and 40,000x. The FIGs. show a number of arrows 201M. These arrows 201M are directed toward what the literature regards as remyelinated axons[8]. It should be understood that the darker myelin regions are not artifacts of the sample preparation or TEM imaging process because nearby or neighborhood axons can be used as reference points and these neighborhood axons do not have similar darker myelin regions.

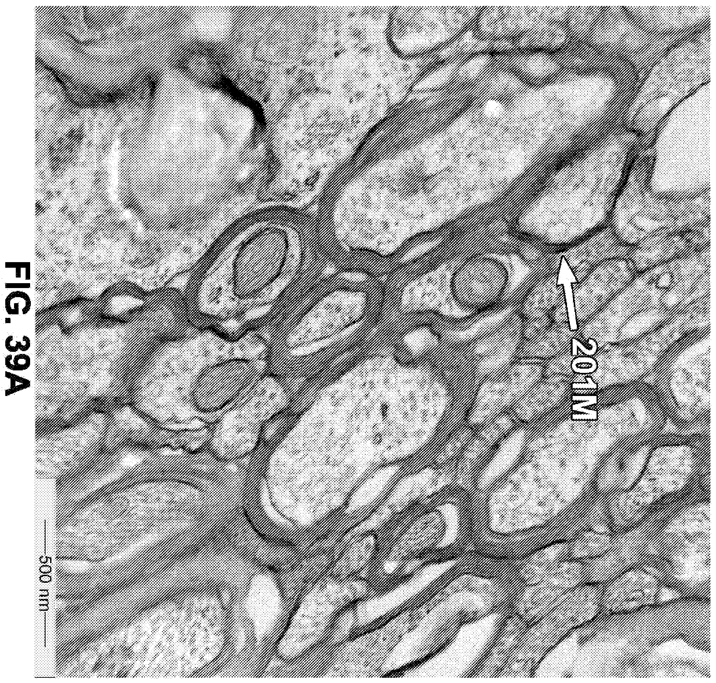

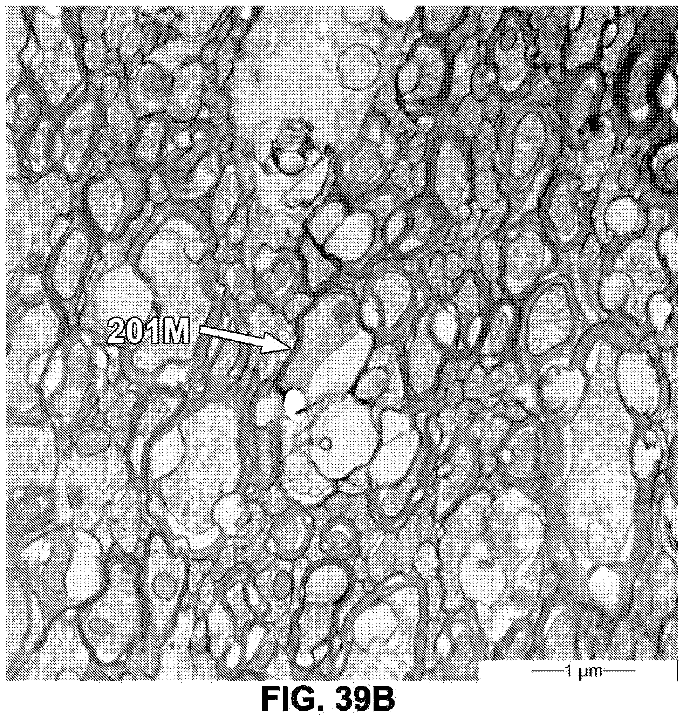

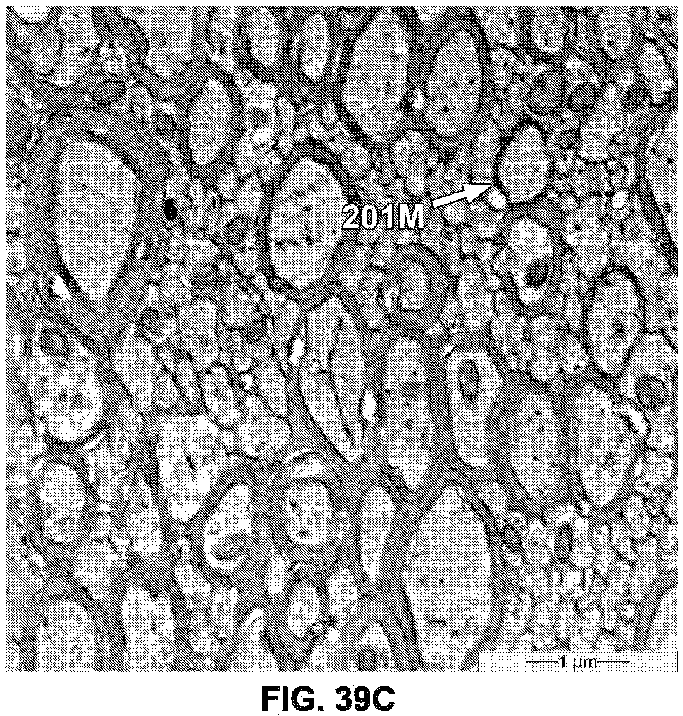

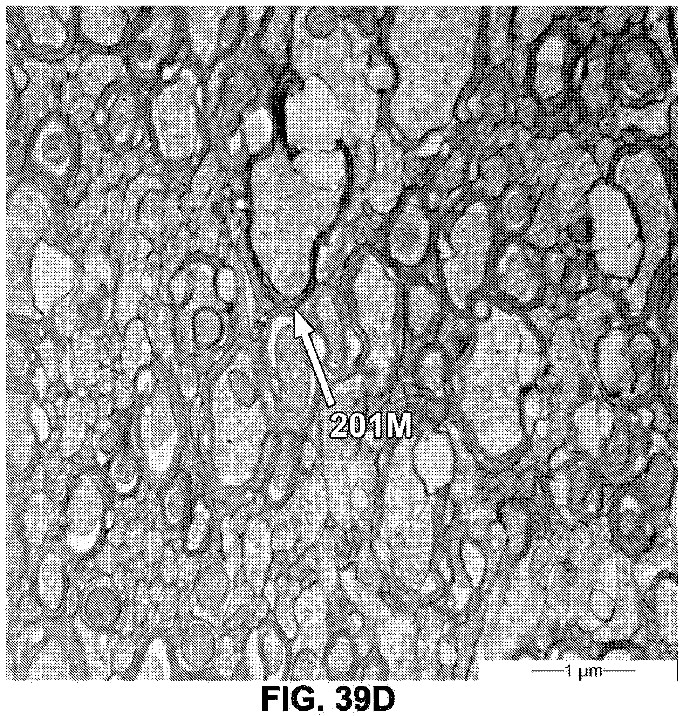

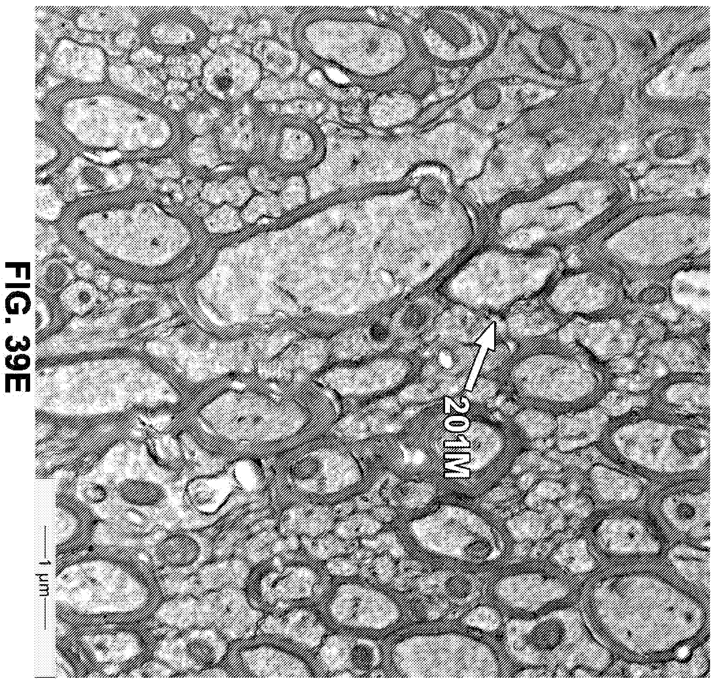

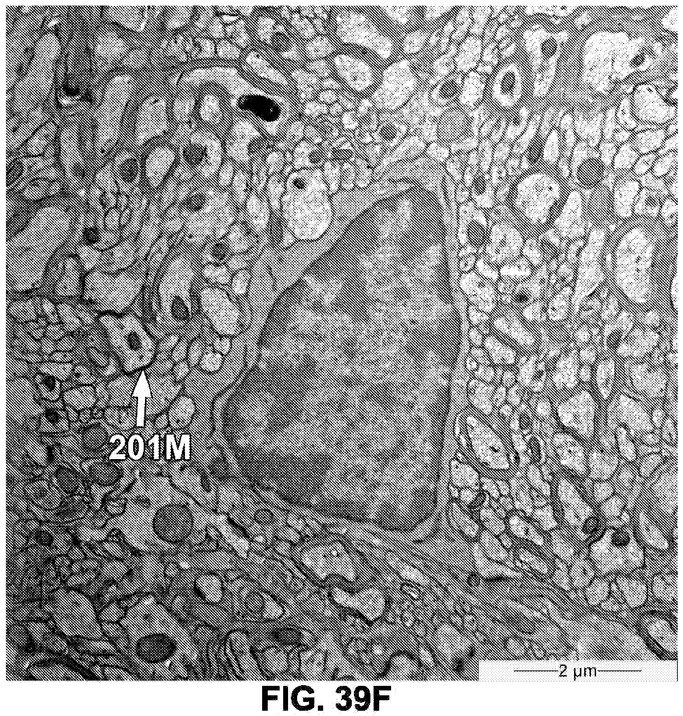

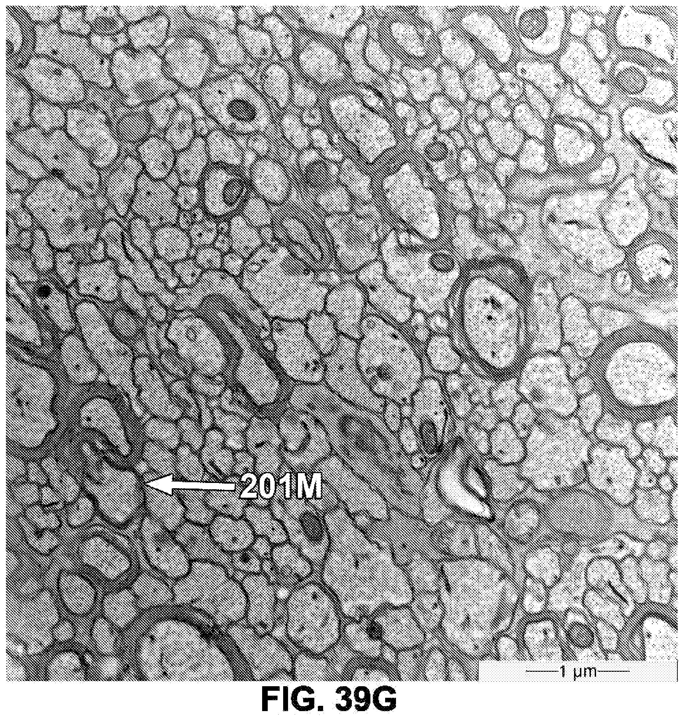

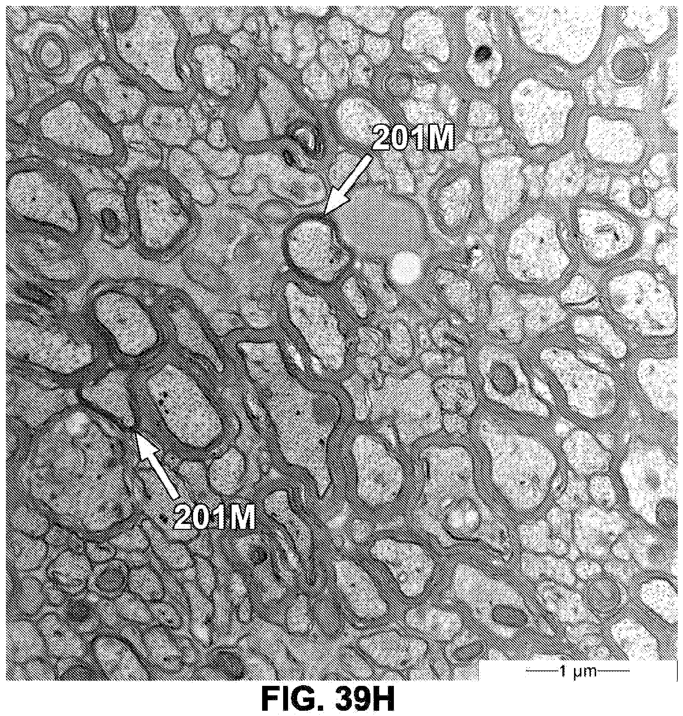

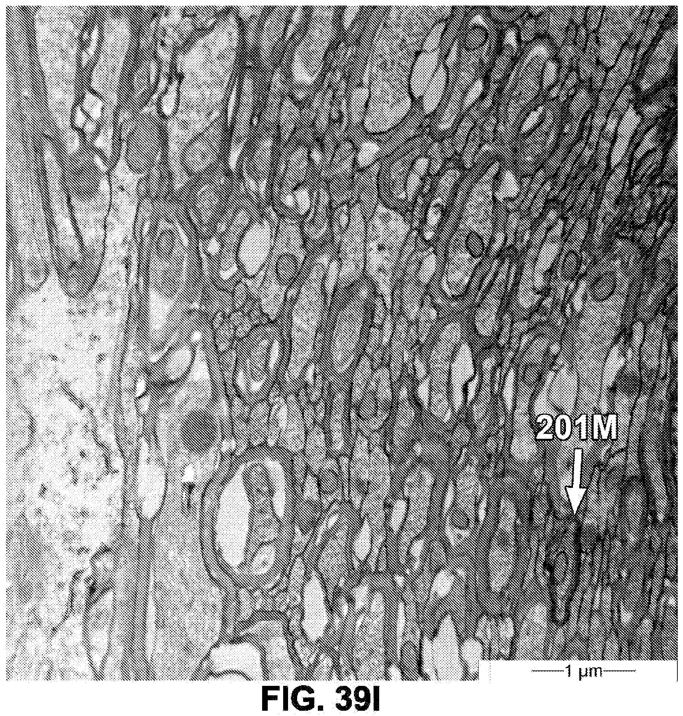

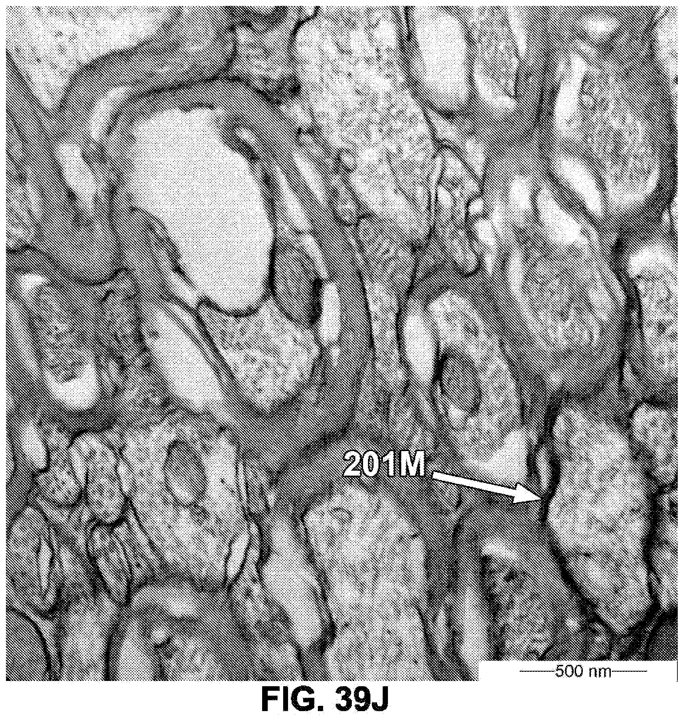

FIGs. 39A-39J correspond to TEM images from Group 6, taken at both 16,000x and 40,000x. These FIGs. show a number of arrows 201M. These arrows 201M are directed toward what the literature regards as remyelinated axons[8]. It should be understood that the darker myelin regions are not artifacts of the sample preparation or TEM imaging process because nearby or neighborhood axons can be used as reference points and these neighborhood axons do not have similar darker myelin regions.

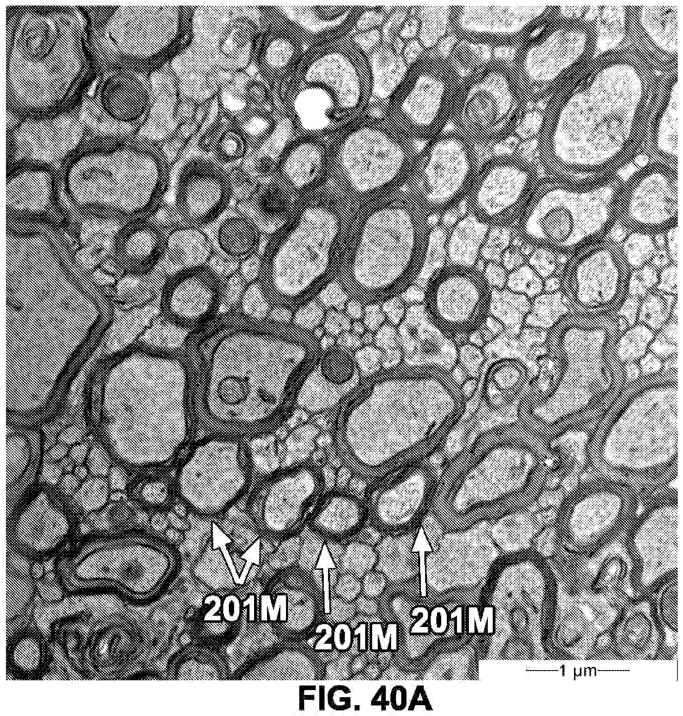

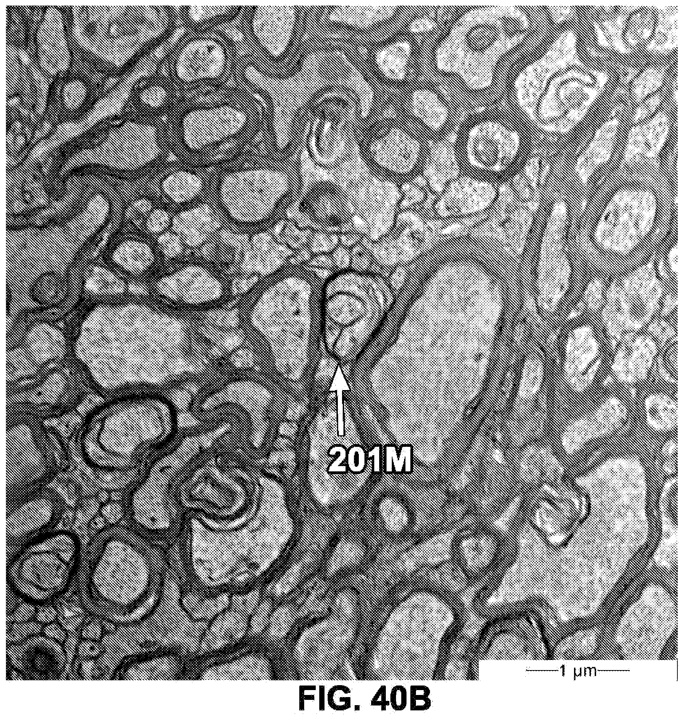

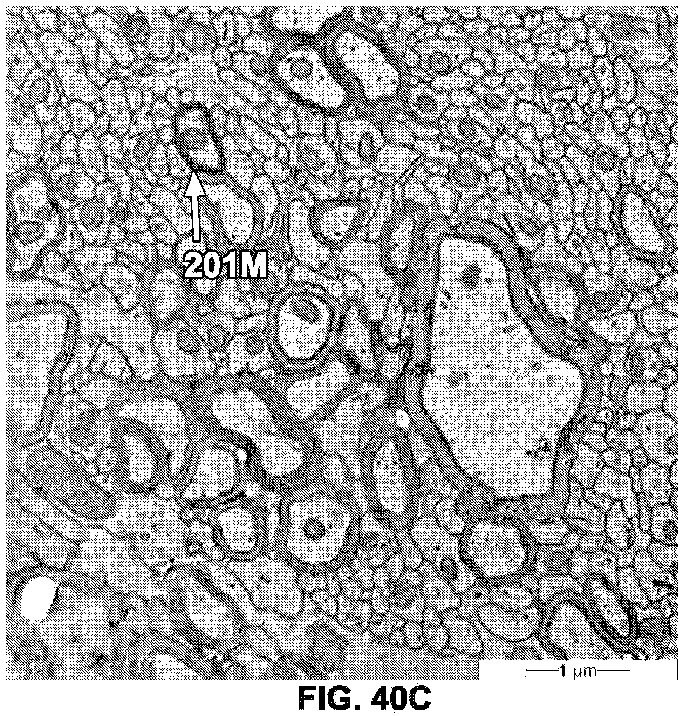

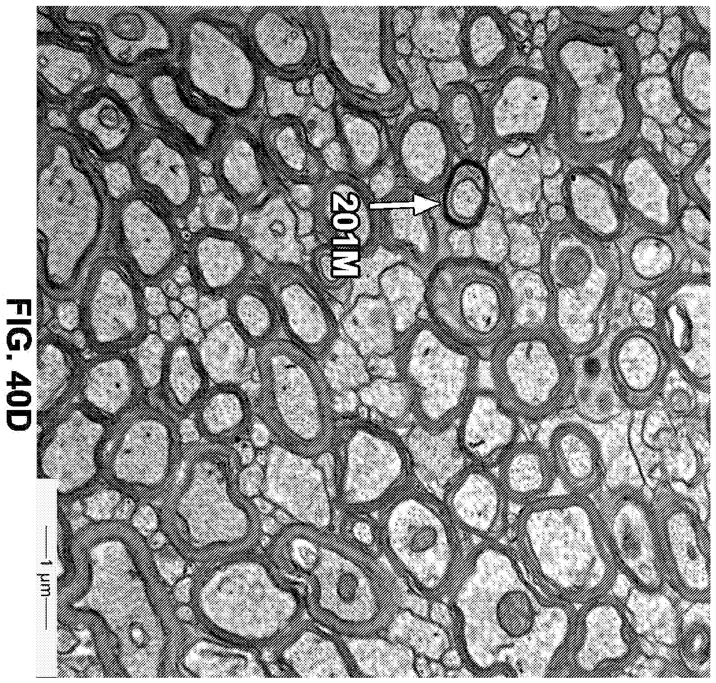

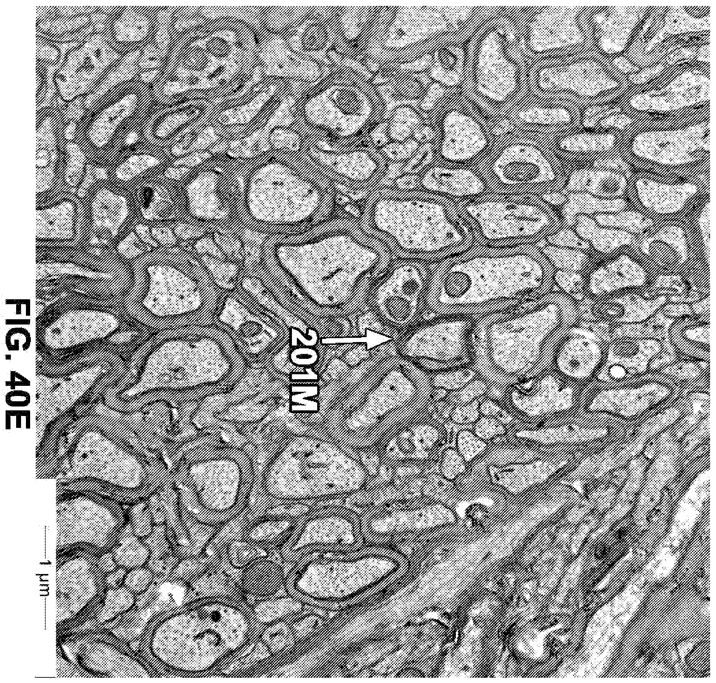

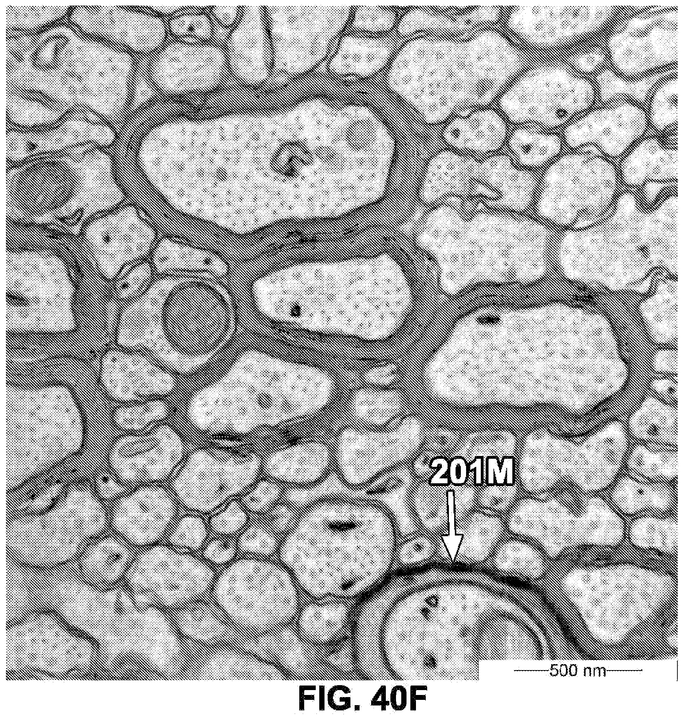

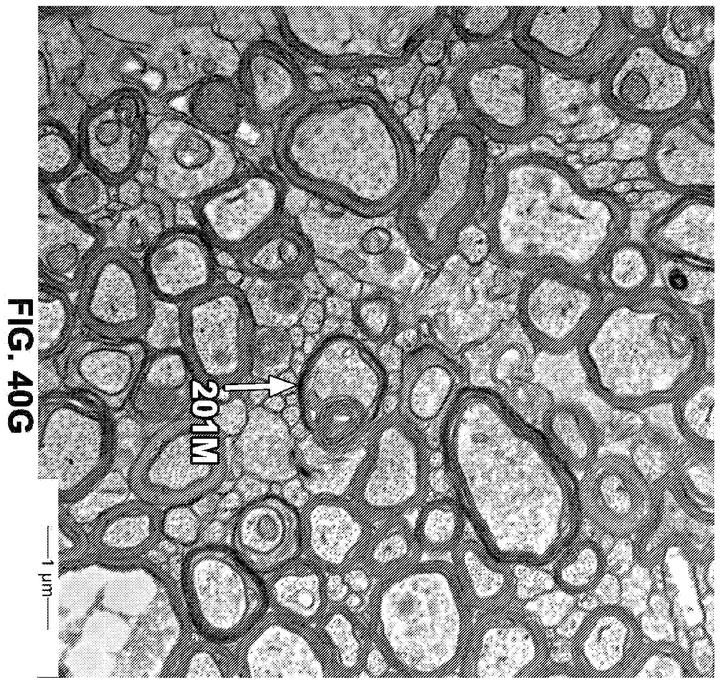

FIGs. 40A-40G correspond to TEM images from Group 7, taken at both 16,000x and 40,000x. These FIGs. Also show a number of arrows 201M. These arrows 201M are directed toward what the literature regards as remyelinated axons[8]. It should be understood that the darker myelin regions are not artifacts of the sample preparation or TEM imaging process because nearby or neighborhood axons can be used as reference points and these neighborhood axons do not have similar darker myelin regions.

The presence of the remyelinated axons, as indicted by the arrows 201M, suggest that CNM-Au8 gold nanocrystal suspensions were an effective treatment for mammals (both therapeutic and prophylactic) to achieve remyelinated axons (e.g., increasing the amount of remyelinated axons relative to axons in similar corpus callosum regions observed in mice in Groups 1-3).

### IV. G-ratio Measurement of Myelin on Axons and Quantification

Another objective method for determining if there are any positive treatment effects of CNM-Au8 gold nanocrystal suspensions on the amount of myelin damage due to Cuprizone Feed is to calculate and compare G-ratios of myelin on axons in the corpus callosum regions of brains of mice in each of Groups 1-7, in accordance with the literature[5,6]. G-ratio calculations are another recognized means for estimating differing pathologic effects.

Specifically, G-ratio measurement and quantification are widely utilized as a functional and structural index of the relative amount of myelin coating present on axons (i.e., axonal myelination). The higher the G-ratio, the thinner the myelin is relative to the axon diameter; and conversely, the lower the g-ratio, the thicker the myelin sheath is relative to the axon. Thus, typically, and within norms, the lower the reported G-ratio, the better. Specifically, it has been reported that average G-ratio ranges for myelinated axons for the corpus callosum region of the brain is within the range of 0.75 to 0.81[5].

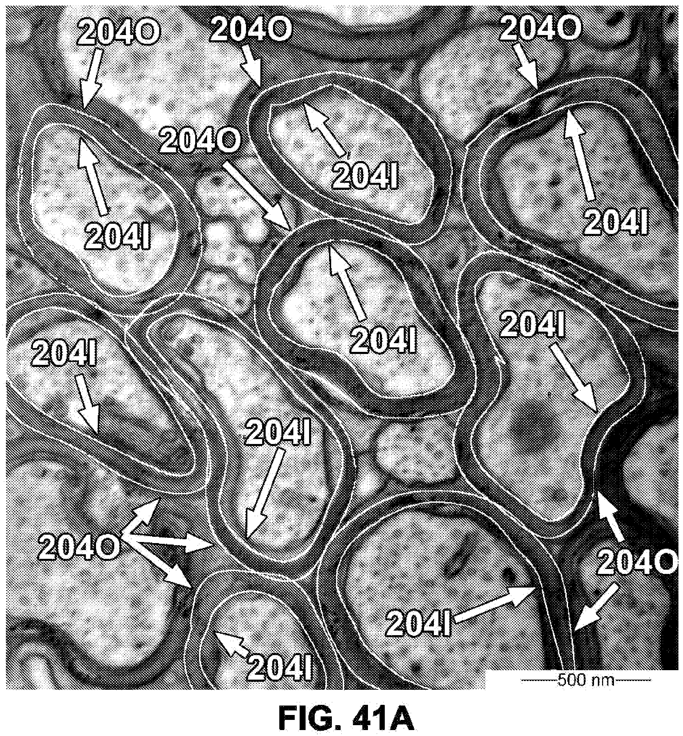

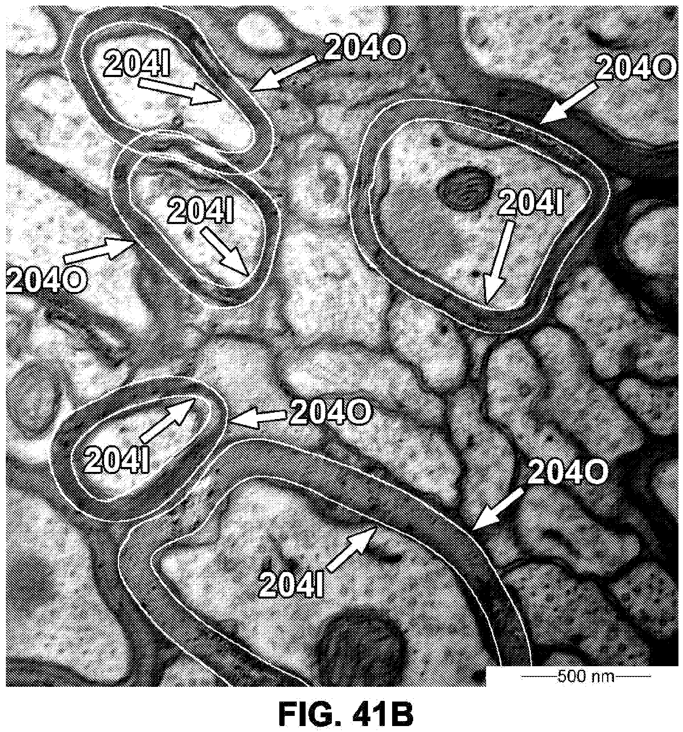

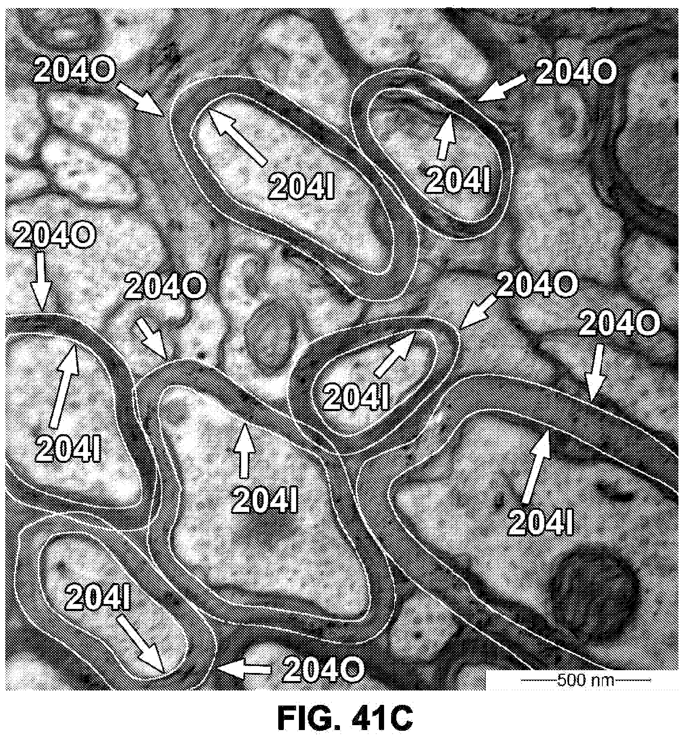

FIGs. 41-47 show representative TEM photomicrographs originally taken at 40,000x, as noted on the scale bars on the images, of representative cross-sectional areas of corpus callosum regions from mice from each of Groups 1-7, respectively. The study details for Groups 1-7, as previously discussed, are set forth in Table 3.

Briefly, inner and outer myelin diameters on representative axons taken from representative TEM images, were marked by tracing, then measured, summed, averaged and used to determine the G-ratios, as discussed herein.

Specifically, randomly selected cross-sectional areas containing neural axons of mice corresponding to mice in each of the seven groups were first selected. TEM photomicrographs originally taken at 40,000x were then made of the randomly selected areas. Using Image J software, the inner (2041) and outer (204O) perimeters of a large numbers of axons shown on the TEM photomicrographs were first traced with a stylus on a computer touch screen. Measurements using the stylus-generated tracings were then made of the inner (2041) and outer (204O) perimeters of the traced axons. In accordance with the literature, the observed cross-sectional areas of the axon/myelin sheath coatings were assumed to be concentric circles.

G-ratios were then calculated by dividing the determined axon outer perimeter (2041) (also corresponding to the myelin inner perimeter and referred to both ways herein) by the outer perimeter (204O) of the axon and myelin sheath added together.

FIGs. 41-47 show some of the randomly selected, representative, TEM images of cross sections of neural axons in the corpus callosum with the tracings corresponding to inner (2041) and outer (204O) myelin perimeter imposed thereon.

FIGs. 41A-41C show representative TEM photomicrograph images which correspond to representative portions of the corpus callosum of mice in Group 1, Example 3. These images are high magnification, 40,000x images, showing that inner (2041) and outer (204O) perimeters of the myelin have been labeled on each axon thereon.

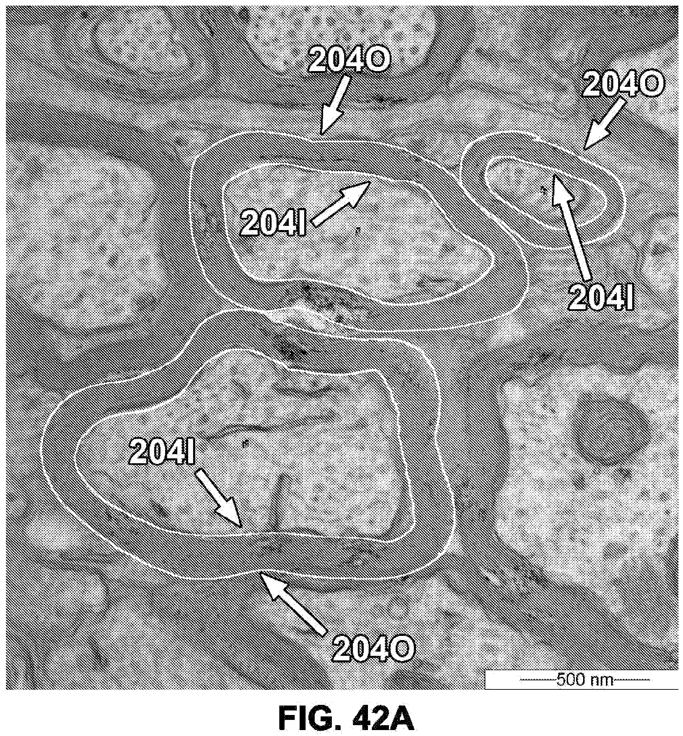

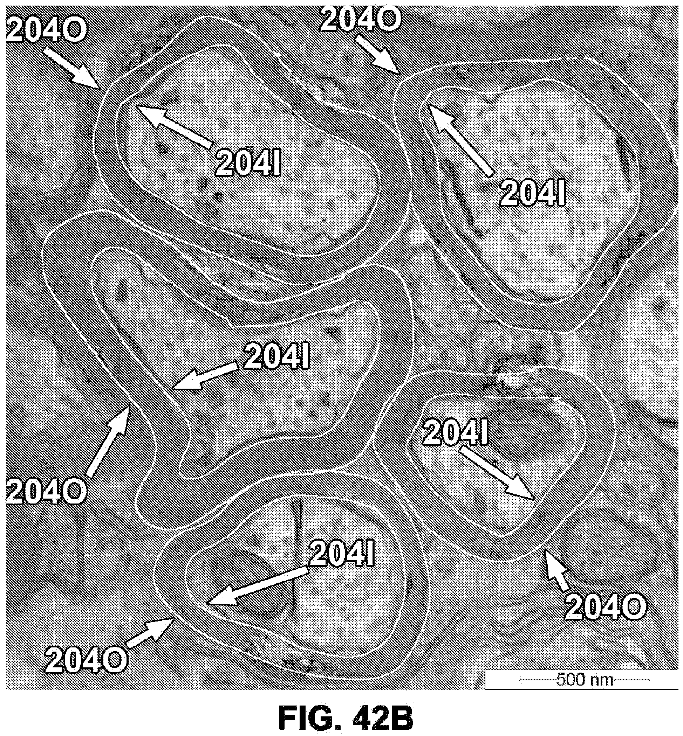

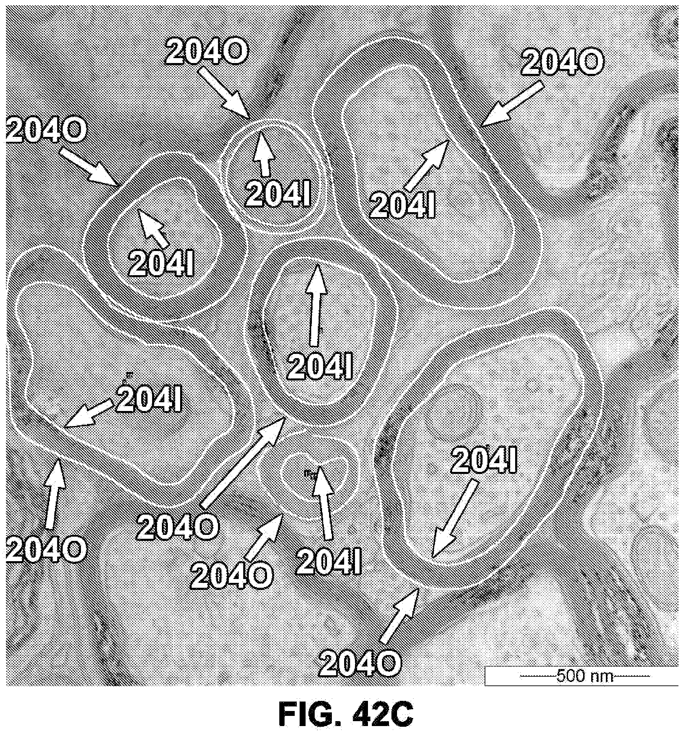

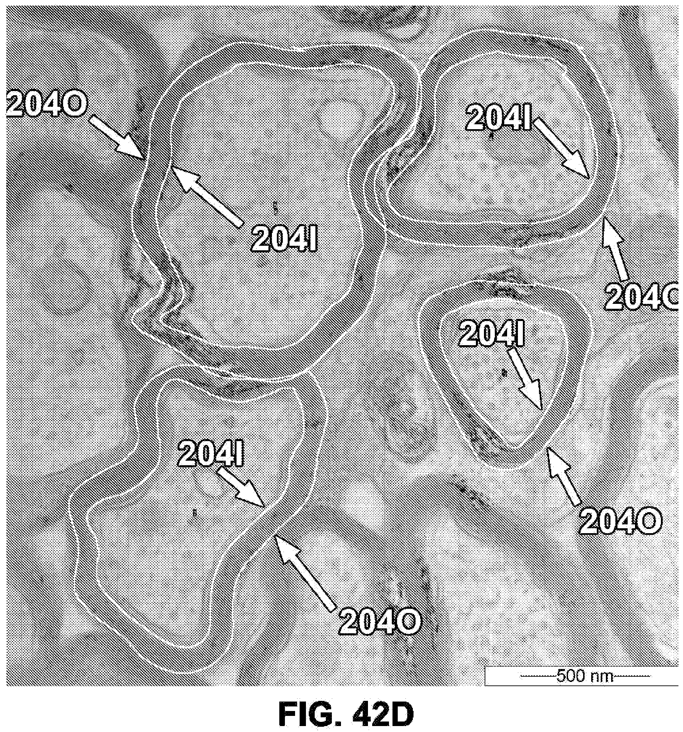

FIGs. 42A-42D show representative TEM photomicrograph images which correspond to representative portions of the corpus callosum of mice in Group 2, Example 3. These images are high magnification, 40,000x images, showing that inner (2041) and outer (204O) perimeters of the myelin have been labeled on each axon thereon.

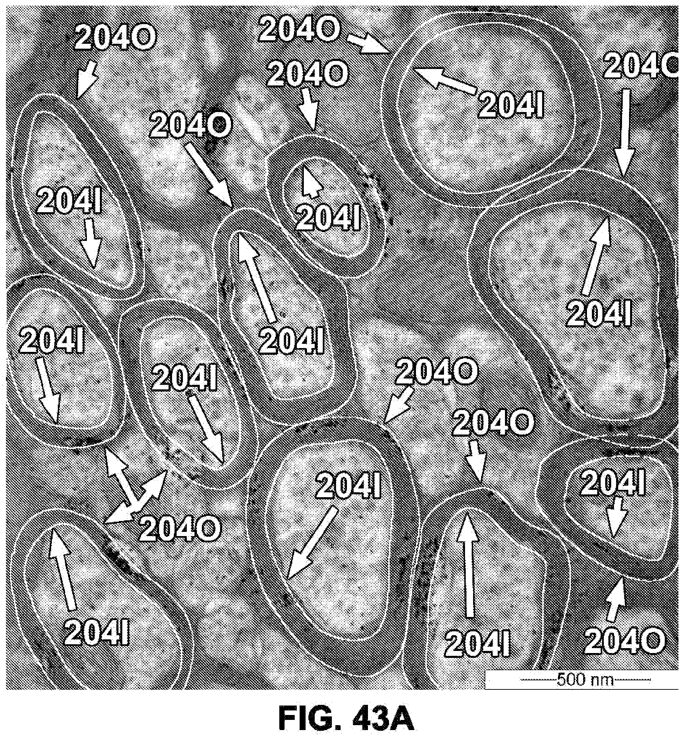

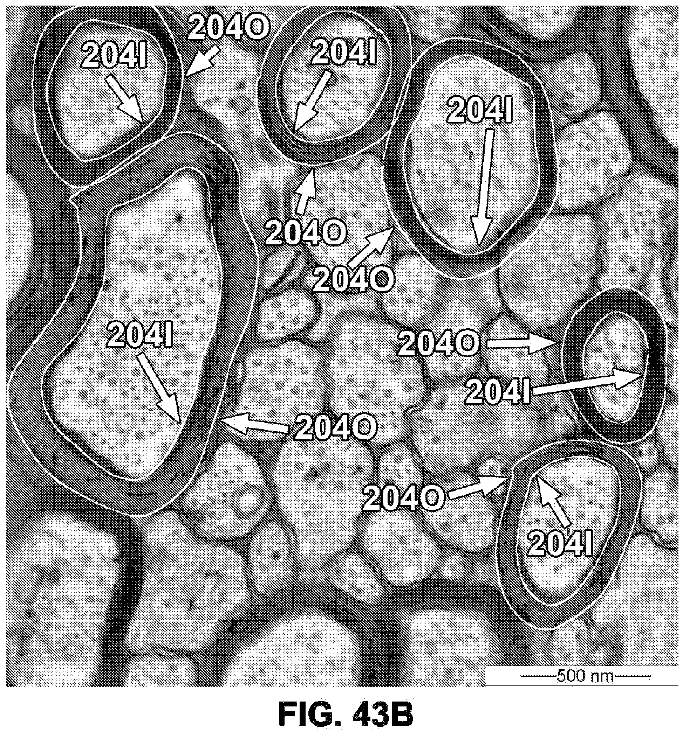

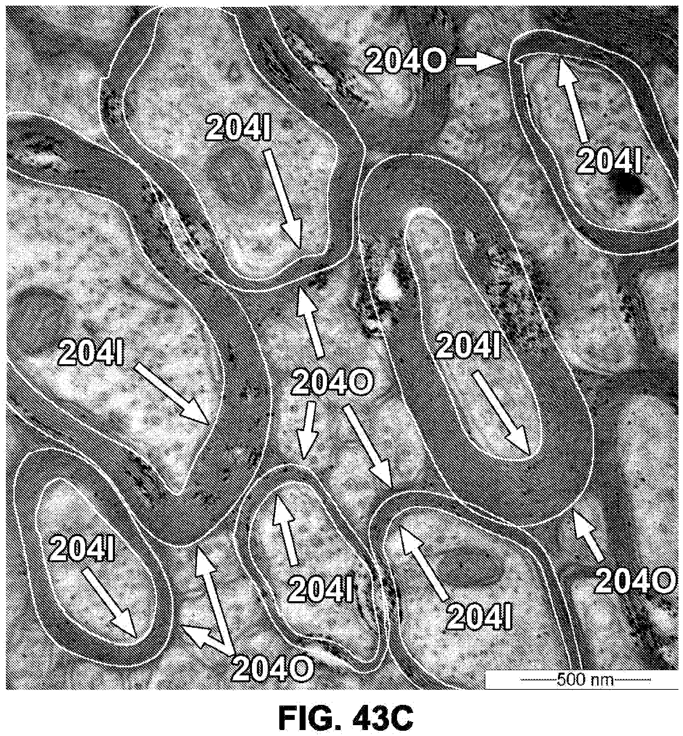

FIGs. 43A-43C show representative TEM photomicrograph images which correspond to representative portions of the corpus callosum of mice in Group 3, Example 3. These images are high magnification, 40,000x images, showing that inner and outer perimeters of the myelin have been labeled on each axon thereon.

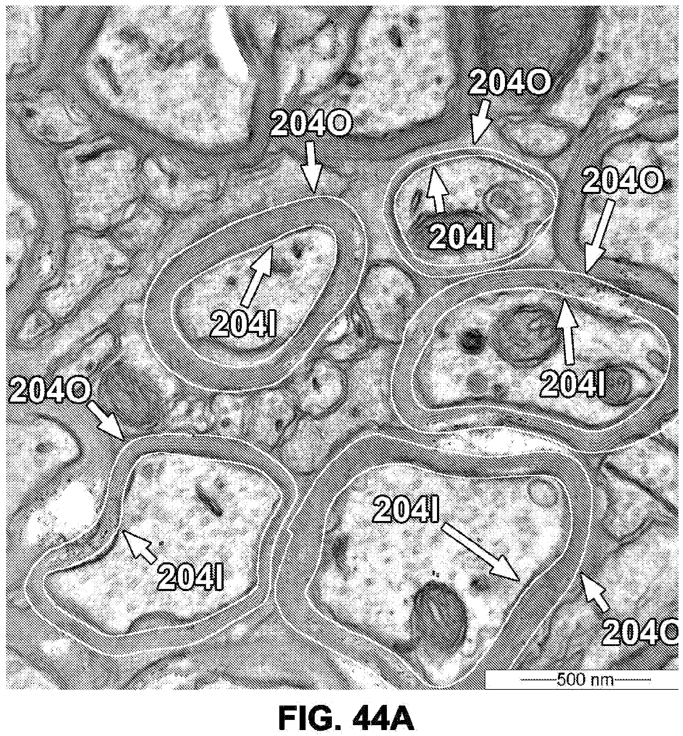

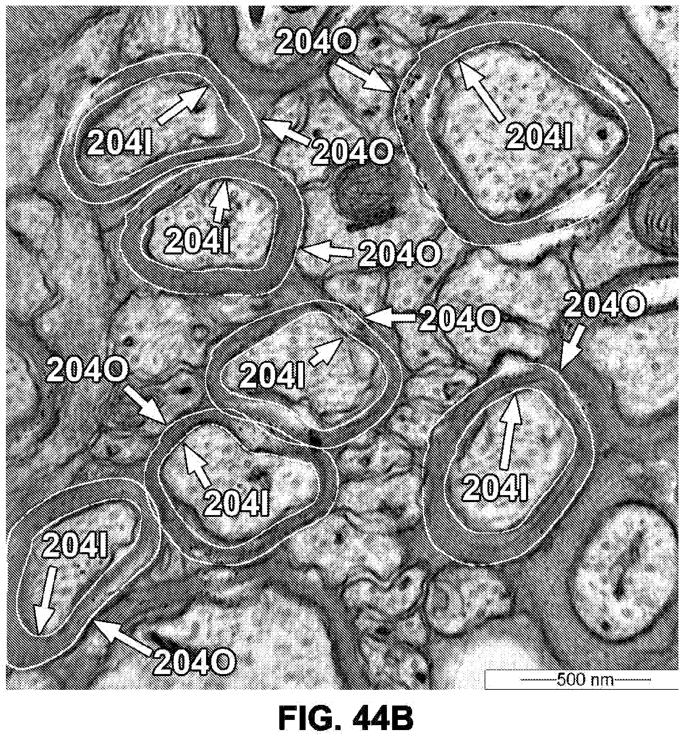

FIGs. 44A-44B show representative TEM photomicrograph images which correspond to representative portions of the corpus callosum of mice in Group 4, Example 3. These images are high magnification, 40,000x images, showing that inner and outer perimeters of the myelin have been labeled on each axon thereon.

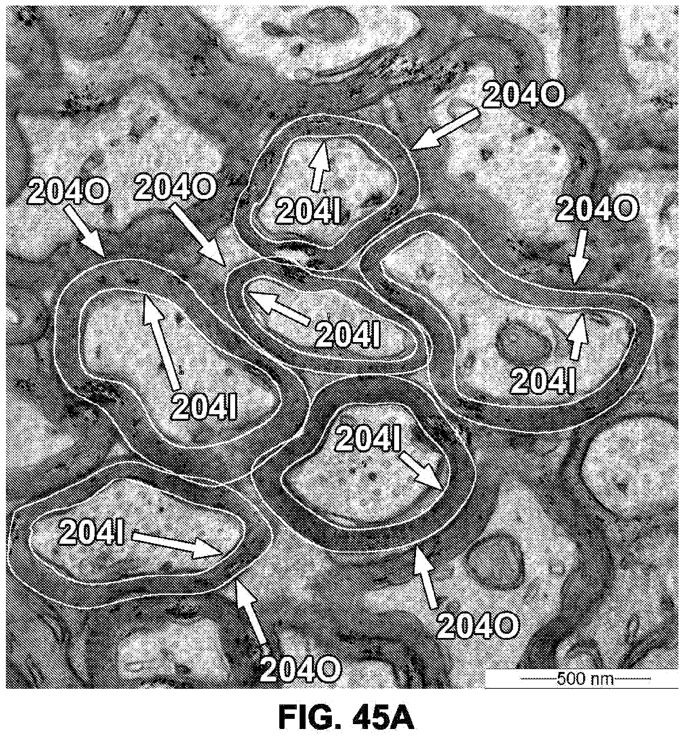

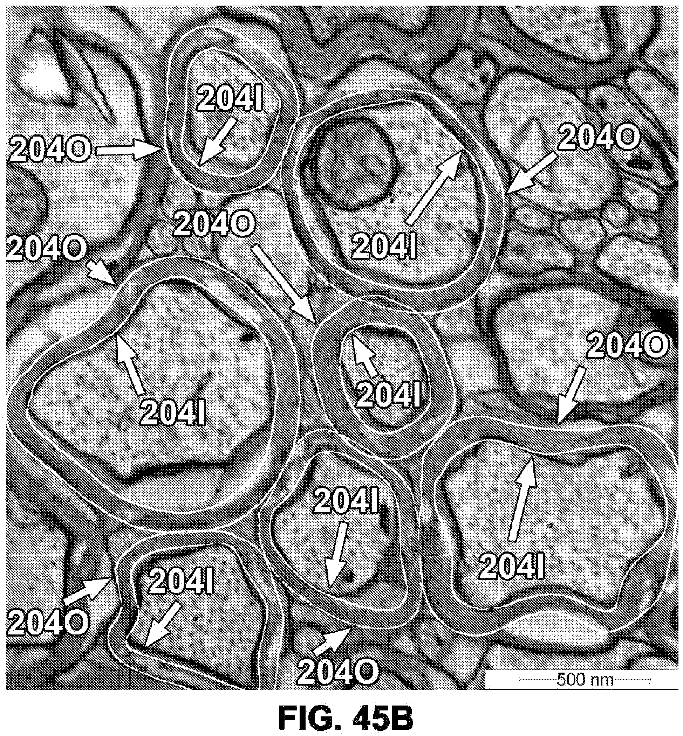

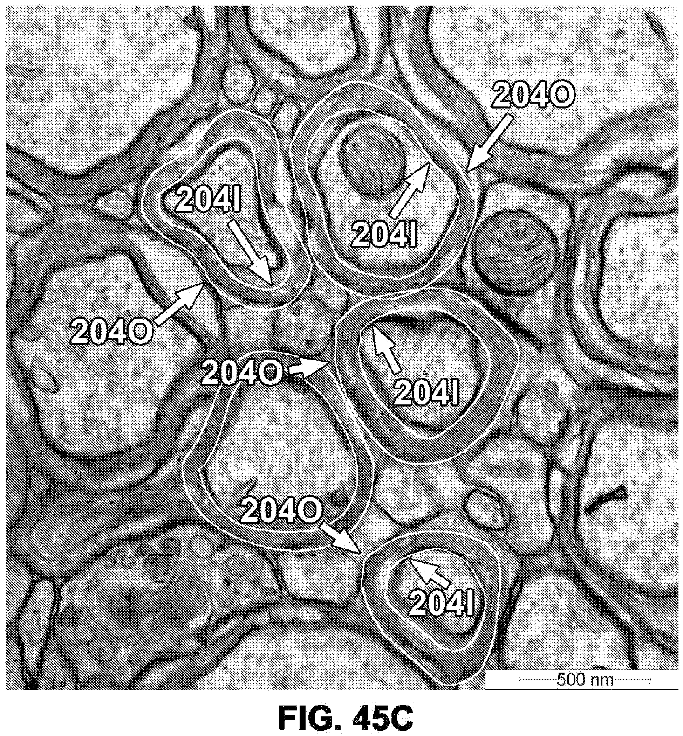

FIGs. 45A-45C show representative TEM photomicrograph images which correspond to representative portions of the corpus callosum of mice in Group 5, Example 3. These images are high magnification, 40,000x images, showing that inner and outer perimeters of the myelin have been labeled on each axon thereon.

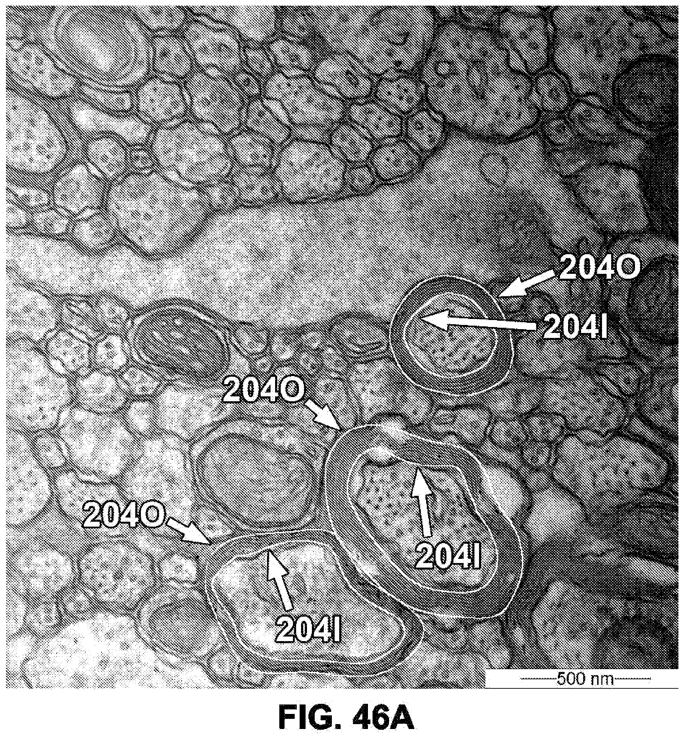

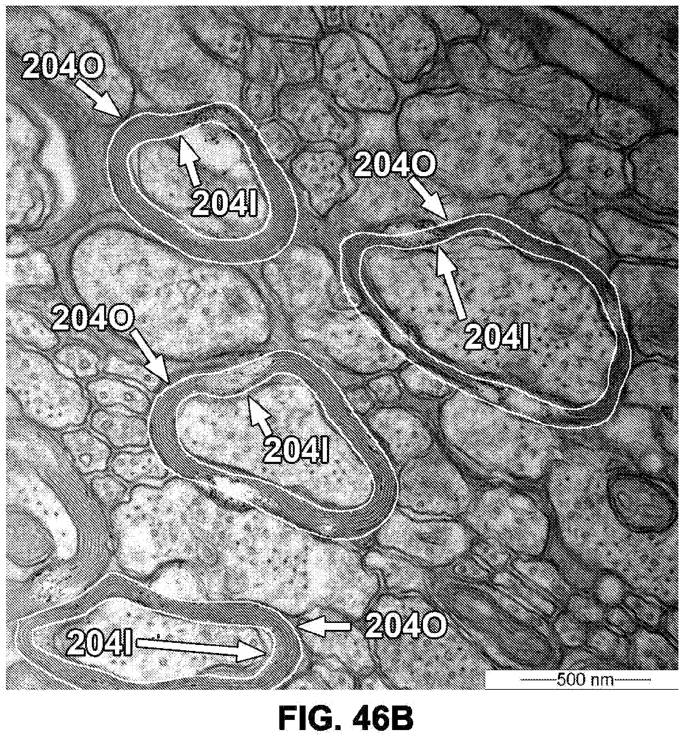

FIGs. 46A-46B show representative TEM photomicrograph images which correspond to representative portions of the corpus callosum of mice in Group 6, Example 3. These images are high magnification, 40,000x images, showing that inner and outer perimeters of the myelin have been labeled on each axon thereon.

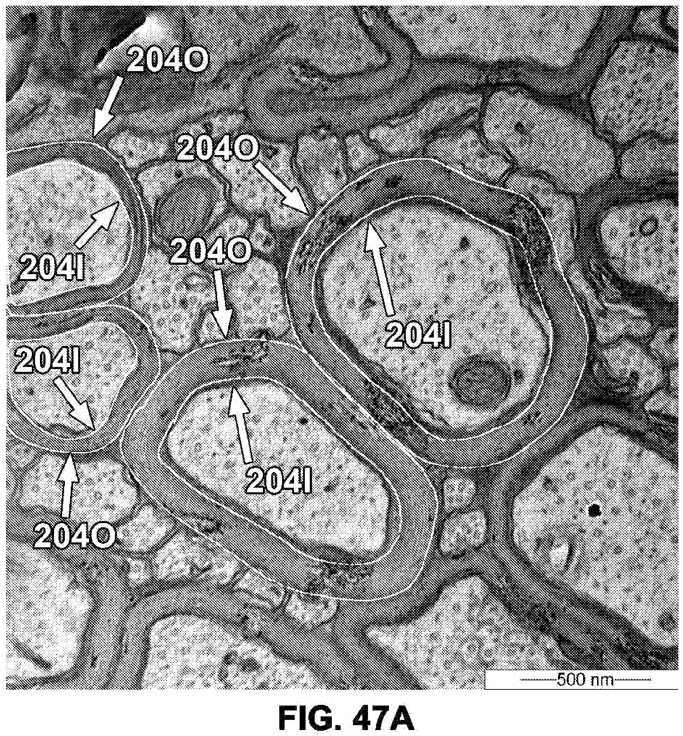

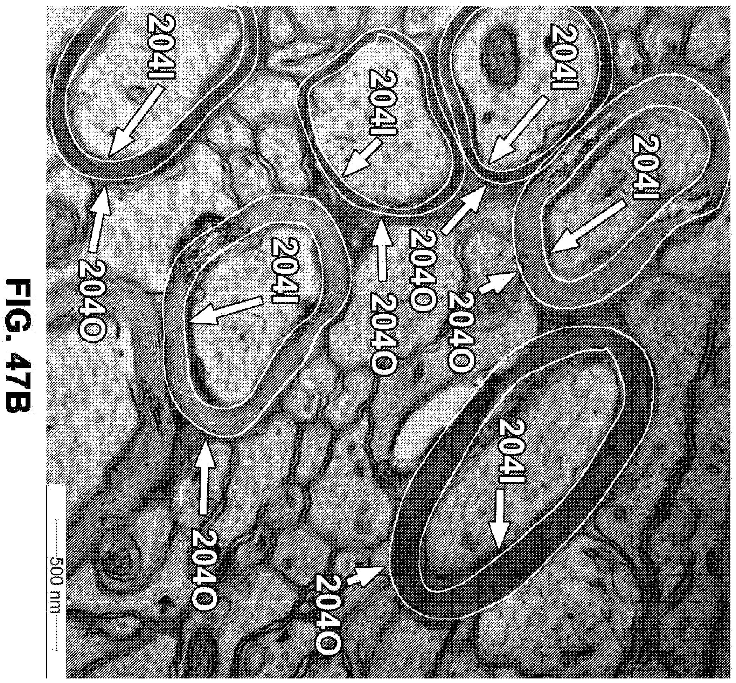

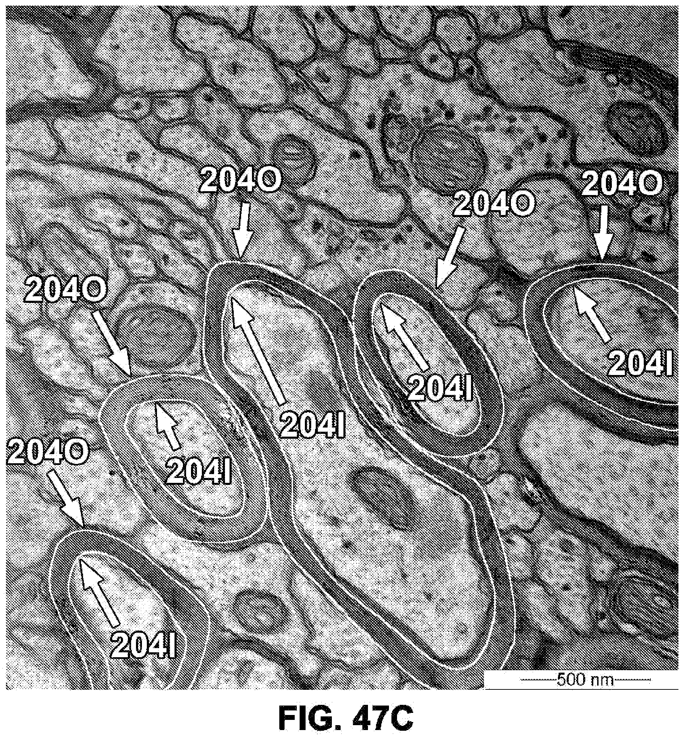

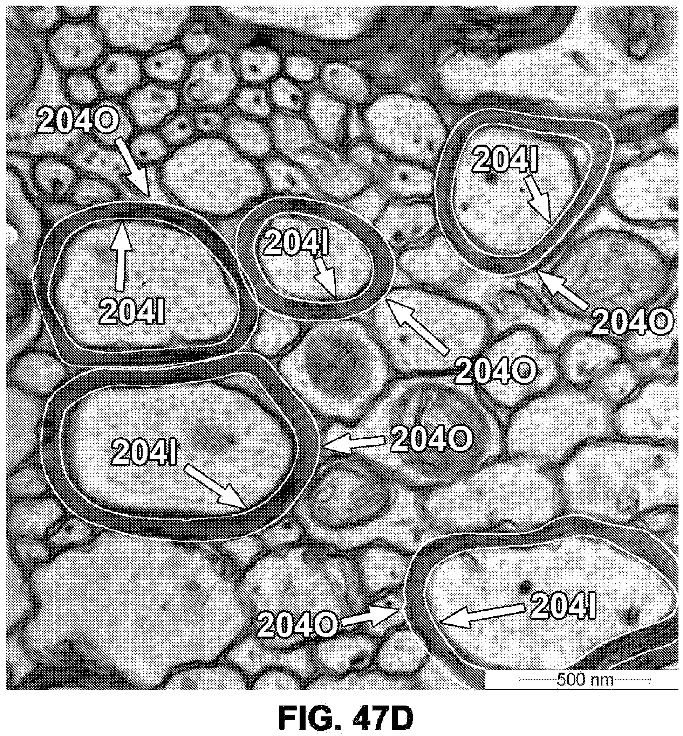

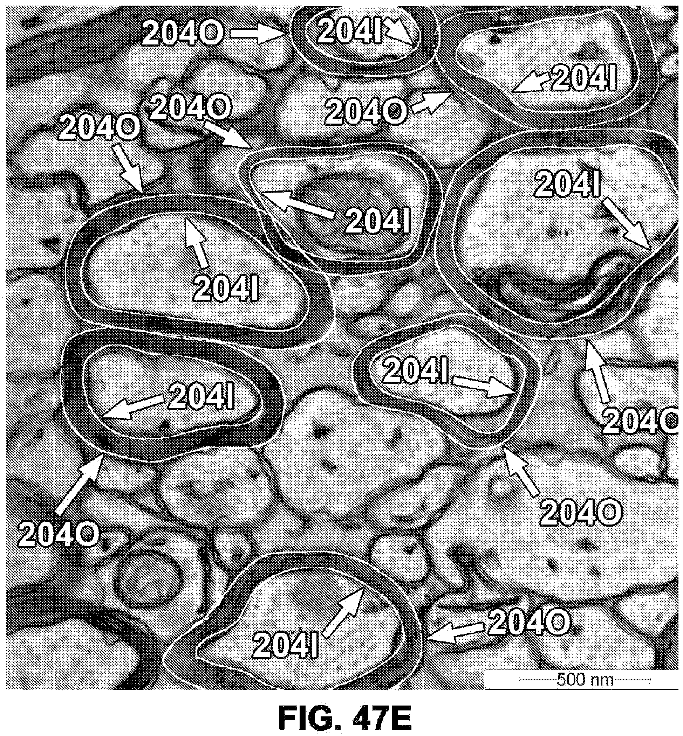

FIGs. 47A-47E show representative TEM photomicrograph images which correspond to representative portions of the corpus callosum of mice in Group 7, Example 3. These images are high magnification, 40,000x images, showing that inner and outer perimeters of the myelin have been labeled on each axon thereon.

Table 5 contains a summary of the results obtained from the aforementioned measurements. Specifically, Table 5 shows the total number of axons that were marked, summed and measured in order to calculate the G-ratios. Measurements were made on a low of 77 axons in Group 1; whereas measurements were made on a high of 374 axons in Group 2.

The "Standard Error of the Mean" ("SEM"), reported in column 4 of Table 5, is the standard deviation of the sample-mean's estimate of a population. (SEM can also be viewed as the standard deviation of the error in the sample mean relative to the true mean, since the sample mean is an unbiased estimator). The SEM was estimated by the sample estimate of the population standard deviation (sample standard deviation) divided by the square root of the sample size.

**Table 5**

| Group Number | Total # of Axons Measured Per Group | Calculated G-ratio For Each Group | Standard Error of The Mean |
|---|---|---|---|
| 1 | 77 | 0.801 | 0.0045 |
| 2 | 374 | 0.754 | 0.0036 |
| 3 | 92 | 0.770 | 0.0055 |
| 4 | 106 | 0.763 | 0.0068 |
| 5 | 103 | 0.778 | 0.0055 |
| 6 | 88 | 0.756 | 0.0066 |
| 7 | 96 | 0.775 | 0.0051 |

As noted previously herein, the myelin degradation caused by the Cuprizone Feed was found to have non-uniform effects on myelinated axons in various portions of the corpus callosum cross-sectional areas viewed by TEM..

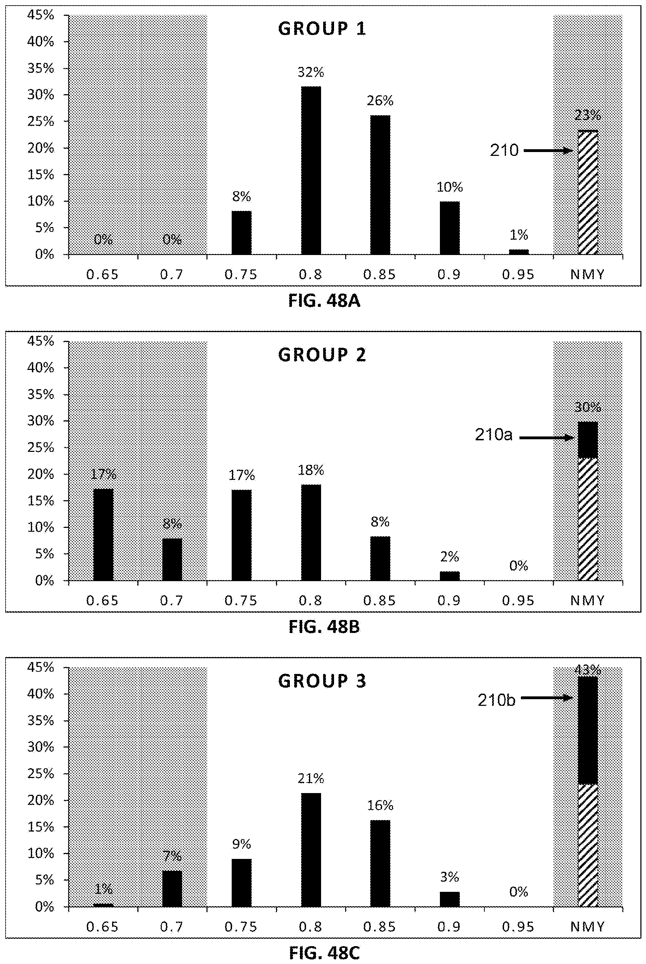

In this regard, FIGs. 48-50 express differently the G-ratio measurements summarized in Table 5. These FIGs. 48-50 are bar chart histograms of G-ratios showing the frequency percentage of axon G-ratios on the Y-axis; versus the G-ratio distribution on the X-axis for each mouse group.

These FIGs. 48-50 also contain a bar labelled "NMY" that is not part of the G-ratio calculations for the other bars in the histogram, but rather, represents, in an area-normalized manner, the number of "demyelinated" axons in each mouse group (i.e., as previously determined and reported in Section II herein entitled "Quantification of Number of Demyelinated Axons/Unit Area"). Because of the presence of the NMY bar, each chart is hereafter referred to as "Modified Bar Chart Histogram".

Modified Bar Chart Histograms containing G-ratio data for mouse Groups 1, 2 and 3 appear in FIGs. 48A, 48B and 48C, respectively.

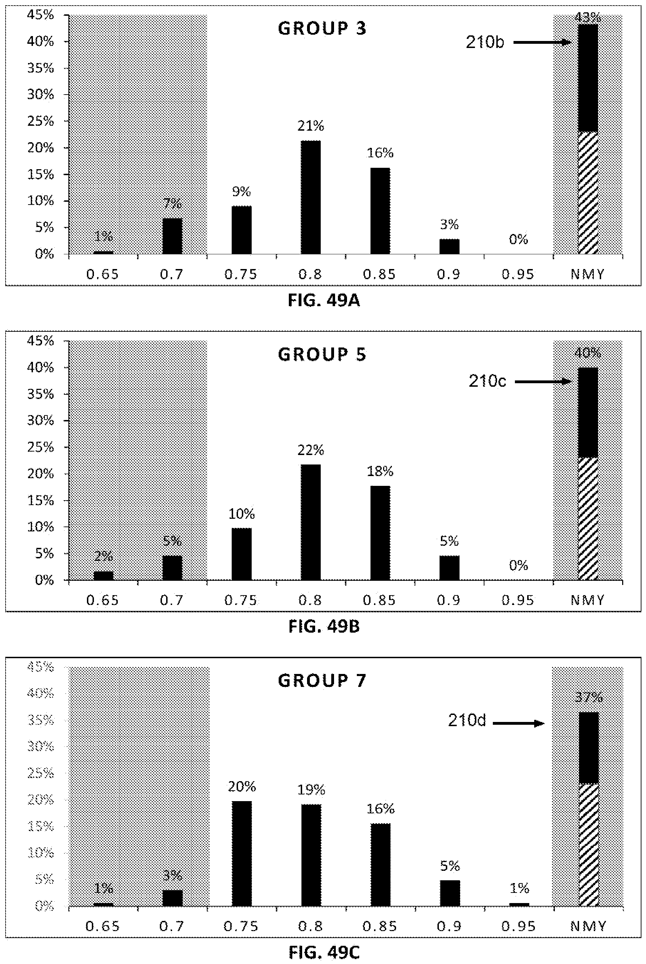

Modified Bar Chart Histograms containing G-ratio data for mouse Groups 3, 5 and 7 appear in FIGs. 49A, 49B and 48C, respectively.

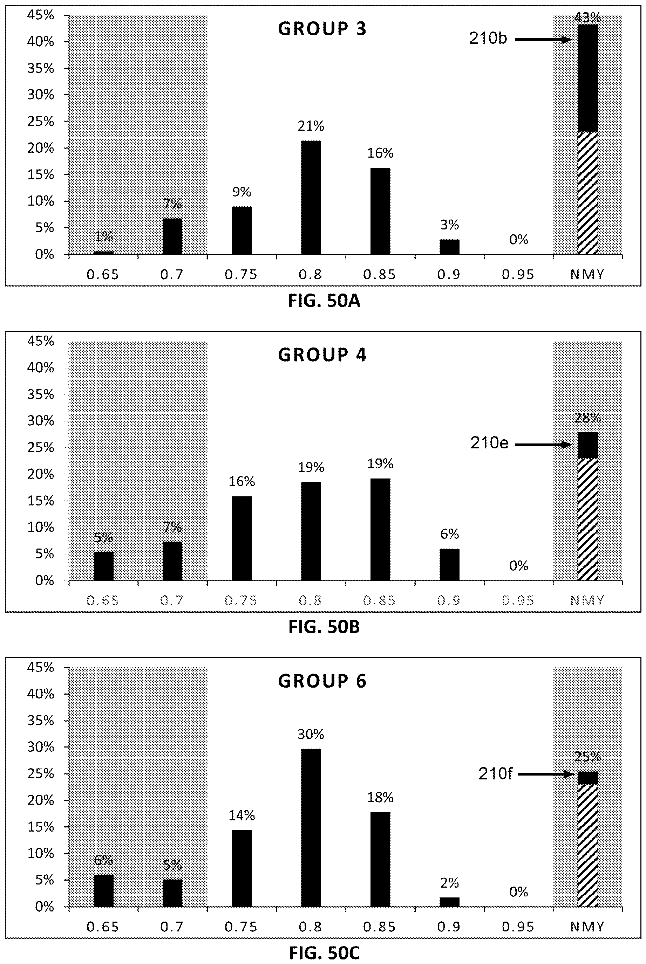

Modified Bar Chart Histograms containing G-ratio data for mouse Groups 3, 4 and 6 appear in FIGs. 50A, 50B and 48C, respectively.

Further, FIG. 48A shows the Modified Bar Chart Histogram containing G-ratio data for mouse Control Group1. Attention is directed to the numbers on the top of each bar. These numbers correspond to the percent occurrence, per unit area, of axons having the G-ratio noted thereon. Specifically, the percent number has been normalized to account for all of the "demyelinated" axons, per the same unit area, that were previously counted (i.e., the percent numbers includes the NMY values). It is believed that reporting both sets of numbers in the Modified Bar Chart Histogram may give a more complete understanding of some of the treatment effects of CNM-Au8 nanosuspensions.

Further attention is directed to both shaded areas on both the left and right sides of the Modified Bar Chart Histogram. These shaded areas overlap with, for example, FIGS. 48B and 48C, and are intended to direct attention to those portions of the Modified Bar Chart Histograms that contain somewhat different or somewhat similar data. Note is also made of the cross-hatching on the NMY bar 210. The same cross-hatching occurs for all the other "NMY" bars on each of the Modified Bar Chart Histograms. Since the mice of Control Group 1 should be considered normal or healthy, the Modified Bar Chart Histogram of Figure 48A could be thought of as a good starting point (i.e., a "positive control") for making comparisons between different groups.

Likewise, Modified Bar Chart Histograms corresponding to mice given Cuprizone Feed appear in FIGs. 48B and 48C. Thus, the Modified Bar Chart Histogram of FIG. 48C could also be thought of as a good starting point (i.e., a "negative control") for making comparisons between different groups. For example, the bar 210b contains the same cross-hatching corresponding to the bar 210 in FIG 48A, but also contains a solid portion showing the greater number of "demyelinated" axons, as discussed above.

FIGs. 49A, 49B and 49C contain Modified Bar Chart Histograms corresponding to mice in Group 3 (negative control), Group 5 and Group 7, respectively. These three Modified Bar Chart Histograms have been placed together for comparison purposes. The features of the Modified Bar Chart Histograms are quite similar.

FIGs. 50A, 50B and 50C also contain Modified Bar Chart Histograms corresponding to mice in Group 3 (negative control), Group 4 and Group 6, respectively. These three Modified Bar Chart Histograms have been placed together for comparison purposes. The features contained in FIGs. 50B and 50C are quite similar to each other, and are also quite different from the negative control shown in FIG. 50A.

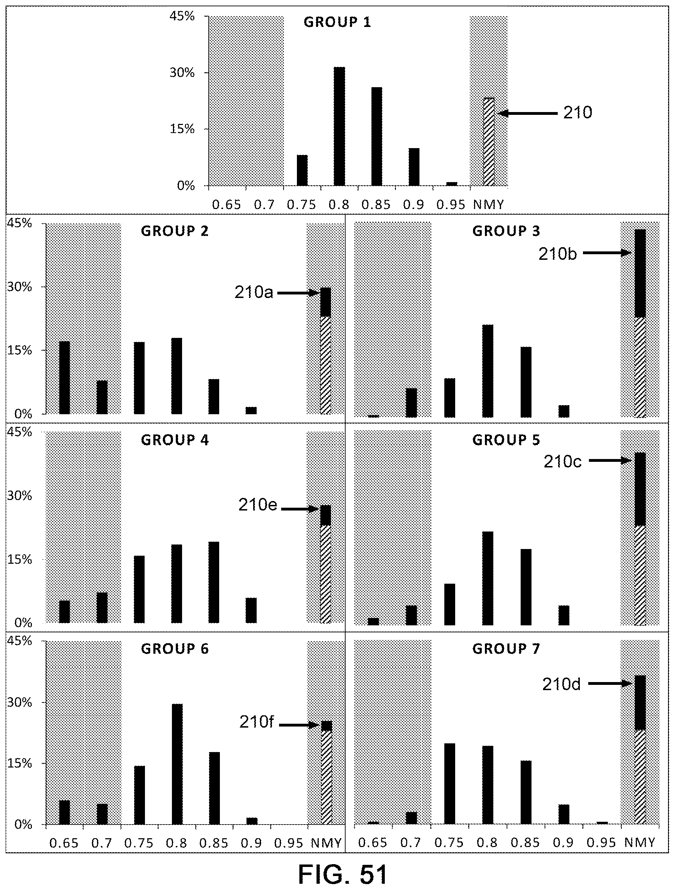

For ease of comparison, the same Modified Bar Chart Histograms all appear in FIG 51.

It should be noted that in FIGs. 48 A, B and C, for a G-ratio size of 0.65, FIG. 48B shows that Group 2 had 17% of its axons having a G-ratio size of 0.65. As discussed in the literature, there is a theoretical limit for individual axons that does not allow for the unlimited expansion of the axons conducting volume to outweigh the benefits associated with myelinating that axon[5]. The expected experimentally observed g-ratio range for coronal axons at optimum efficiency would be on the order of 0.76 to just over 0.80[5]. In FIG. 48B, a small population of axons with a G-ratio size of 0.65 were less than what would be the normal G-ratio and considered to be an early response to Cuprizone Feed. Thus, such axons would not be expected to function normally or well[5].

It should be noted that the similar Modified Bar Chart Histograms Shown in FIGs. 50B and 50C correspond to the mice that were from; (1) **Group 4** and fed Cuprizone Feed for all 5 weeks of the study and were given by **gavage**, for all 5 weeks, once a day, a treatment volume of about 10 mL/kg of a concentrated CNM-Au8 suspension at a gold crystal concentration of about 1000 ppm, to determine if the gold nanosuspension provided was an effective treatment amount; and from (2) **Group 6** which were fed Cuprizone Feed for all 5 weeks of the study and drank *ad libitum* from water bottles a treatment CNM-Au8 suspension at a crystalline gold concentration of about 50 ppm for all 5 weeks of the study, to determine if the gold nanosuspension provided worked as an effective treatment amount.

FIGs. 50B and 50C, show that both Group 4 mice and Group 6 mice have about 5% of their axons at a G-ratio of 0.65. This G-ratio is discussed in the literature as representing the axons/myelin undergoing a recovery process from a demyelinating disease; wherein CNS axons undergo an initial period of hyper-remyelination during recovery and show an increased diameter for some time before eventually reverting to a normal g-ratio5. These data should be understood as meaning that myelin preservation can occur.

While it is difficult to determine needed concentrations, amounts and/or treatment times from this data (as well as all of the other data herein) it is clear that different biological (pathological) events occur as a function of providing the CNM-Au8 treatments discussed herein.

### *Conclusions*

The data suggest:

1. Mice that were given Cuprizone Feed and water for 5 weeks developed typical demyelination that was sought by the investigators as shown by comparing the Modified Bar Chart Histograms in FIG 48A to one or both of the Modified Bar Chart Histograms in Fig 48B and 48C.

2. Mice that were given Cuprizone Feed and CNM-Au8 nanosuspensions (either by gavage or *ad libitum*) for all 5 weeks of the study had similar Modified Bar Chart Histograms (see FIGs 50B and 50C), both of which were superior to negative control (see FIG 50A).

3. The G-ratio data alone suggests that CNM-Au8 nanosuspensions positively affected (i.e., either reduced demyelination or caused remyelination) of the mice in Groups 4 and 6 that were exposed to Cuprizone Feed and CNM-Au8 nanosuspensions for all 5 weeks of the study.

### REFERENCES

1. Cortical Demyelination Is Prominent in the Murine Cuprizone Model and Is Strain Dependent. Skripuletz T, et al. (Apr 2008)

2. The Neurotoxicant, Cuprizone, as a Model to Study Demyelination and Remyelination in the Central Nervous System. Matsushima GK, et al. (Jan 2001)

3. Beneficial Effects of Minocycline on Cuprizone Induced Cortical Demyelination. Skripuletz T, et al. (Sep 2010)

4. Preparation of Mouse Brain Tissue for Immunoelectron Microscopy. Tremblay ME et al. (Jul 2010)

5. What is the Optimal Value of the G-Ratio for Myelinated Fibers in the Rat CNS? A Theoretical Approach. Chomiak T. et al. (Nov 2009)

6. NG2 cells response to axonal alteration in the spinal cord white matter in mice with genetic disruption of neurofilament light subunit expression. Wu YJ et al. (Oct 2008)

7. Remyelination Therapy for Multiple Sclerosis. Keough, Michael B., et al. (Nov 2012)

8. Spontaneous Remyelination Following Prolonged Inhibition of Alpha4 Integrin in Chronic EAE. Piraino P.S. et al (June 2005)

9. The Cuprizone Animal Model: New Insights into an Old Story. Kipp Markus, et al. (Sept 2009)

10. Response of Mice to the Chelating Agents Sodium Diethyldithiocarbamate, Alpha-Benzoinoxime, and Biscyclohexanone Oxaldihydrazone. Carlton WW. (1966)

11. Studies On the Induction of Hydrocephalus and Spongy Degeneration by Cuprizone Feeding and Attempts to Antidote the Toxicity. Carlton WW. (1967)

12. Expression of Carbonic Anhydrase II mRNA and Protein in Oligodendrocytes During Toxic Demyelination in the Young Adult Mouse. Tansey FA, et al. ((1996)

13. Noninvasive Detection of Cuprizone Induced Axonal Damage and Demyelination in the Mouse Corpus Callosum. Sun SW1, et al. (2006)

14. GAS6 Enhances Repair Following Cuprizone-Induced Demyelination. Tsiperson V, et al. (2010)

15. Glial Response During Cuprizone-Induced De- and Remyelination in the CNS: Lessons Learned. Gudi V1, et al. (2014)

**Claims**

1. An elemental gold nanosuspension for use in a method of treatment of a disease selected from the group consisting of Progressive Supranuclear Palsy, Alexander's Disease, Krabbe Disease, Metachromatic Leukodystrophy, Canvan Disease, Leukodistrophies, Central Pontine Myelolysis (CPM), Anti-MAG Disease, Pelizaeus-Merzbacher Disease, Refsum Disease, Cockayne Syndrome, Zellweger Syndrome, Guillain-Barre Syndrome (GBS), Van der Knapp Syndrome, chronic inflammatory demyelinating polyneuropathy (CIDP), multifocal motor neuropathy (MMN), Neuromyelitis Optica (NMO), Progressive Multifocal Leukoencephalopathy (PML), Mild Cognitive Impairment (MCI), Wallerian Degeneration, and Adrenoleukodystrophy.

2. An elemental gold nanosuspension for use according to Claim 1, wherein said disease comprises Neuromyelitis Optica (NMO).

3. An elemental gold nanosuspension for use according to Claim 1 or Claim 2 wherein said gold nanosuspension comprises:

   a.) pharmaceutical grade water;

b.) at least one processing enhancer, selected from the group consisting of:

monosodium phosphate; disodium phosphate; trisodium phosphate;
a potassium phosphate;
a carbonic acid salt, including sodium carbonate, sodium bicarbonate, potassium carbonate and potassium bicarbonate;
sulfite or bisulfite salts, including sodium or potassium; and

c.) gold nanocrystals suspended in said water forming a suspension, wherein said gold nanocrystals:

i.) have surfaces that include at least one characteristic selected from the group of characteristics consisting of: (1) no organic chemical constituents adhered or attached to said surfaces and/or (2) are substantially clean and do not have chemical constituents adhered or attached to surfaces, other than water or said processing enhancer, which alter the functioning of said nanocrystals;
ii.) have a mode particle size of less than about 50nm; and
iii.) are present in said suspension at a concentration of about 2-2000ppm;

wherein said suspension has a pH of between about 5 to about 9.5 and a zeta potential of at least about -20mv.

4. An elemental gold nanosuspension for use according to Claim 3 wherein said processing enhancer is sodium bicarbonate.

5. An elemental gold nanosuspension for use according to Claim 3 wherein said gold nanocrystals are present in said suspension at a concentration of about 2-200ppm.

6. An elemental gold nanosuspension for use according to Claim 3 wherein said nanosuspension has a zeta potential of at least about -30 mV.

7. An elemental gold nanosuspension for use according to Claim 6 wherein said nanosuspension has a zeta potential of at least about -40 mV.


**Patentansprüche**

1. Elementare Gold-Nanosuspension zur Verwendung in einem Verfahren zur Behandlung einer Krankheit, ausgewählt aus der Gruppe bestehend aus progressive supranukleäre Blickparese, Morbus Alexander, Morbus Krabbe, metachromatische Leukodystrophie, Morbus Canavan, Leukodystrophien, zentrale pontine Myelinolyse (CPM), Anti-MAG-Syndrom, Morbus Pelizaeus-Merzbacher, Refsum-Syndrom, Cockayne-Syndrom, Zellweger-Syndrom, Guillain-Barre-Syndrom (GBS), Van der Knapp-Syndrom, chronisch-inflammatorische demyelinisierende Polyneuropathie (CIDP), multifokale motorische Neuropathie (MMN), Neuromyelitis optica (NMO), progressive multifokale Leukoenzephalopathie (PML), leichte kognitive Beeinträchtigung (LKB), Wallersche Degeneration und Adrenoleukodystrophie.

2. Elementare Gold-Nanosuspension zur Verwendung nach Anspruch 1, wobei die Krankheit Neuromyelitis optica (NMO) umfasst.

3. Elementare Gold-Nanosuspension zur Verwendung nach Anspruch 1 oder Anspruch 2, wobei die Gold-Nanosuspension umfasst:

a.) Wasser pharmazeutischer Qualität;
b.) zumindest einen Verarbeitungsverbesserer, ausgewählt aus der Gruppe bestehend aus:

Mononatriumphosphat; Dinatriumphosphat; Trinatriumphosphat;
Kaliumphosphat;
Kohlensäuresalz, einschließlich Natriumcarbonat, Natriumbicarbonat, Kaliumcarbonat und Kaliumbicarbonat;
Sulfit oder Bisulfitsalzen, einschließlich Natrium oder Kalium; und

c.) Gold-Nanokristalle, die in dem Wasser suspendiert sind und eine Suspension bilden, wobei die Gold-Nanokristalle:

i.) Oberflächen aufweisen, die zumindest eine Eigenschaft aufweisen, ausgewählt aus der Gruppe von Eigenschaften bestehend aus: (1) keine organischen chemischen Bestandteile haften an diesen Oberflächen oder sind mit ihnen verbunden und/oder (2) sie sind im Wesentlichen sauber und weisen keine chemischen Bestandteile auf, die an den Oberflächen haften oder mit ihnen verbunden sind, mit Ausnahme von Wasser oder dem Verarbeitungsverbesserer, welche die Funktionsweise der Nanokristalle verändern;

ii.) eine Modalpartikelgröße von weniger als etwa 50 nm aufweisen; und

iii.) in der Suspension in einer Konzentration von etwa 2-2000 ppm vorliegen; wobei die Suspension einen pH-Wert zwischen etwa 5 bis etwa 9,5 und ein Zetapotential von mindestens etwa -20 mV aufweist.

4. Elementare Gold-Nanosuspension zur Verwendung nach Anspruch 3, wobei der Verarbeitungsverbesserer Natriumbicarbonat ist.

5. Elementare Gold-Nanosuspension zur Verwendung nach Anspruch 3, wobei die Gold-Nanokristalle in der Suspension in einer Konzentration von etwa 2-200 ppm vorliegen.

6. Elementare Gold-Nanosuspension zur Verwendung nach Anspruch 3, wobei die Nanosuspension ein Zetapotential von mindestens etwa -30 mV aufweist.

7. Elementare Gold-Nanosuspension zur Verwendung nach Anspruch 6, wobei die Nanosuspension ein Zetapotential von mindestens etwa -40 mV aufweist.

**Revendications**

1. Nanosuspension d'or élémentaire pour une utilisation dans une méthode de traitement d'une maladie choisie dans le groupe constitué par l'ophtalmoplégie supranucléaire progressive, la maladie d'Alexander, la maladie de Krabbe, la leucodystrophie métachromatique, la maladie de Canavan, les leucodystrophies, la myélinolyse centropontine (CPM), la maladie anti-MAG, la maladie de Pelizaeus-Merzbacher, la maladie de Refsum, le syndrome de Cockayne, le syndrome de Zellweger, le syndrome de Guillain-Barré (GBS), le syndrome de Van der Knapp, la polyneuropathie démyélinante inflammatoire chronique (CIDP), la neuropathie motrice multifocale (MMN), la neuromyélite optique (NMO), la leuco-encéphalopathie multifocale progressive (PML), le trouble cognitif léger (MCI), la dégénérescence wallérienne, et l'adrénoleucodystrophie.

2. Nanosuspension d'or élémentaire pour une utilisation selon la revendication 1, dans laquelle ladite maladie comprend la neuromyélite optique (NMO).

3. Nanosuspension d'or élémentaire pour une utilisation selon la revendication 1 ou la revendication 2, laquelle nanosuspension d'or comprend :

a.) de l'eau de qualité pharmaceutique ;

b.) au moins un amplificateur de traitement choisi dans le groupe constitué par :

le phosphate monosodique ; le phosphate disodique ; le phosphate trisodique ;
un phosphate de potassium ;
un sel d'acide carbonique, y compris le carbonate de sodium, le bicarbonate de sodium, le carbonate de potassium et le bicarbonate de potassium ;
les sels sulfites ou bisulfites, y compris de sodium ou de potassium ; et

c.) des nanocristaux d'or en suspension dans ladite eau en formant une suspension, lesquels nanocristaux d'or :

i.) ont des surfaces qui comprennent au moins une caractéristique choisie dans le groupe de caractéristiques consistant en : (1) pas de constituants chimiques organiques adhérant ou attachés auxdites surfaces et/ou (2) elles sont pratiquement propres et n'ont pas de constituants chimiques adhérant ou attachés aux surfaces, autres que l'eau ou ledit amplificateur de traitement, qui altèrent le fonctionnement desdits monocristaux ;

ii.) ont une granulométrie modale inférieure à environ 50 nm ; et

iii.) sont présents dans ladite suspension à une concentration d'environ 2 à 2 000 ppm ;

laquelle suspension a un pH compris entre environ 5 et environ 9,5 et un potentiel zêta d'au moins environ -20 mV.

4. Nanosuspension d'or élémentaire pour une utilisation selon la revendication 3, dans laquelle ledit amplificateur de traitement est le bicarbonate de sodium.

5. Nanosuspension d'or élémentaire pour une utilisation selon la revendication 3, dans laquelle lesdits nanocristaux d'or sont présents dans ladite suspension à une concentration d'environ 2 à 200 ppm.

6. Nanosuspension d'or élémentaire pour une utilisation selon la revendication 3, laquelle nanosuspension a un potentiel zêta d'au moins environ -30 mV.

7. Nanosuspension d'or élémentaire pour une utilisation selon la revendication 6, laquelle nanosuspension a un potentiel zêta d'au moins environ -40 mV.

FIG. 1

FIG. 2A

FIG. 2B

EP 2 994 146 B1

FIG. 2C

30b'

FIG. 3

FIG. 4A

FIG. 4B

FIG. 5A

604

60

Secondary    Primary    Secondary

603            601            603

Ground

**FIG. 5B**

V

C

**FIG. 5C**

V

C

**FIG. 5D**

Straight/Straight
Configuration

7a

6a

FIG. 6

7b

6b

5a

5b

FIG. 7

(L)

(N)

501AC

**Fig. 8**

Fig. 9

FIG. 10A

**Aura-8$_7$ Size Distribution**

FIG. 10B

FIG. 10C

FIG. 11

FIG. 12A

FIG. 12B

**FIG. 13A**

**FIG. 13B**

**FIG. 13C**

**FIG. 13D**

# Group 1

## FIG. 14A

Group 2

FIG. 14B

Group 3

FIG. 14C

Group 4

FIG. 14D

FIG. 15

FIG. 16

FIG. 17A

FIG. 17B

FIG. 17C

FIG. 17D

FIG. 18

Average Weekly consumption

Liquid consumed (mL)

Week

Group 1 — Group 2 — Group 3 — Group 4

**MS mouse model (Average mass of group)**

| | 9/17/2012 | 9/19/2012 | 9/26/2012 | 10/3/2012 | 10/10/2012 | 10/17/2012 | 10/19/2012 | 10/22/2012 | 10/23/2012 | 10/24/2012 | 10/25/2012 |
|---|---|---|---|---|---|---|---|---|---|---|---|
| ◆ Group 1 | 23.3 | 23.6 | 24.6 | 25.9 | 25.9 | 27.3 | | 26.9 | | | |
| ■ Group 2 | 24.5 | 24.9 | 24.9 | 25.1 | 25.0 | 25.3 | | | 25.6 | | |
| △ Group 3 | 24.6 | 25.2 | 26.3 | 27.0 | 27.7 | 29.0 | | | | 29.3 | |
| ✕ Group 4 | 25.1 | 25.5 | 25.7 | 26.2 | 26.0 | 26.6 | 26.6 | | | 27.4 | 27.2 |

Date weighed

Figure 19

EP 2 994 146 B1

63

FIG. 20A

FIG. 20B

FIG. 20C

FIG. 20D

FIG. 20E

FIG. 20F

111

109

110R

110L

## FIG. 21A

120D

120C

120A

120B

120E

## FIG. 21B

**FIG. 22**

**FIG. 23A**

FIG. 23B

**FIG. 23C**

FIG. 24A

**FIG. 24B**

**FIG. 24C**

**FIG. 24D**

FIG. 24E

**FIG. 25A**

**FIG. 25B**

**FIG. 25C**

FIG. 25D

FIG. 25E

**FIG. 25F**

**FIG. 25G**

FIG. 26A

**FIG. 26B**

**FIG. 26C**

**FIG. 26D**

FIG. 26E

**FIG. 27A**

**FIG. 27B**

FIG. 27C

**FIG. 27D**

**FIG. 28A**

**FIG. 28B**

**FIG. 28C**

**FIG. 28D**

**FIG. 28E**

**FIG. 28F**

**FIG. 28G**

**FIG. 29A**

**FIG. 29B**

**FIG. 29C**

FIG. 29D

## FIG. 3OA

## FIG. 3OB

## FIG. 3OC

## FIG. 31A

## FIG. 31B

## FIG. 32A

## FIG. 32B

## FIG. 33A

## FIG. 33B

## FIG. 34A

## FIG. 34B

FIG. 35A

## FIG. 35B

# FIG. 36A

## FIG. 36B

**FIG. 37A**

**FIG. 37B**

FIG. 37C

FIG. 37D

FIG. 37E

FIG. 37F

**FIG. 37G**

**FIG. 37H**

**FIG. 37I**

FIG. 37J

**FIG. 37K**

FIG. 38A

FIG. 38B

FIG. 38C

FIG. 38D

FIG. 38E

**FIG. 38F**

**FIG. 38G**

FIG. 38H

FIG. 38I

FIG. 38J

FIG. 38K

FIG. 38L

FIG. 39A

**FIG. 39B**

**FIG. 39C**

FIG. 39D

FIG. 39E

**FIG. 39F**

FIG. 39G

FIG. 39H

**FIG. 39I**

FIG. 39J

FIG. 40A

FIG. 40B

FIG. 40C

**FIG. 40D**

FIG. 40E

FIG. 40F

**FIG. 40G**

**FIG. 41A**

FIG. 41B

**FIG. 41C**

FIG. 42A

FIG. 42B

**FIG. 42C**

**FIG. 42D**

**FIG. 43A**

**FIG. 43B**

FIG. 43C

**FIG. 44A**

**FIG. 44B**

**FIG. 45A**

FIG. 45B

**FIG. 45C**

**FIG. 46A**

**FIG. 46B**

**FIG. 47A**

**FIG. 47B**

**FIG. 47C**

**FIG. 47D**

**FIG. 47E**

FIG. 48A

FIG. 48B

FIG. 48C

FIG. 49A

FIG. 49B

FIG. 49C

FIG. 50A

FIG. 50B

FIG. 50C

FIG. 51

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011006007 A
- US 2010041427 W

**Non-patent literature cited in the description**

- *Histochemistry and Cell Biology,* 2012, vol. 138 (5), 787-802
- **SKRIPULETZ T et al.** *Cortical Demyelination Is Prominent in the Murine Cuprizone Model and Is Strain Dependent,* April 2008
- **MATSUSHIMA GK et al.** *The Neurotoxicant, Cuprizone, as a Model to Study Demyelination and Remyelination in the Central Nervous System,* January 2001
- **SKRIPULETZ et al.** *Beneficial Effects of Minocycline on Cuprizone Induced Cortical Demyelination,* September 2010
- **TREMBLAY ME et al.** *Preparation of Mouse Brain Tissue for Immunoelectron Microscopy,* July 2010
- **CHOMIAK T. et al.** What is the Optimal Value of the G-Ratio for Myelinated Fibers in the Rat CNS?. *A Theoretical Approach,* November 2009
- **WU YJ et al.** *NG2 cells response to axonal alteration in the spinal cord white matter in mice with genetic disruption of neurofilament light subunit expression,* October 2008
- **KEOUGH, MICHAEL B. et al.** *Remyelination Therapy for Multiple Sclerosis,* November 2012
- **PIRAINO P.S. et al.** *Spontaneous Remyelination Following Prolonged Inhibition of Alpha4 Integrin in Chronic EAE,* June 2005
- **KIPP MARKUS et al.** *The Cuprizone Animal Model: New Insights into an Old Story,* September 2009
- **CARLTON WW.** Response of Mice to the Chelating Agents Sodium Diethyldithiocarbamate. *Alpha-Benzoinoxime, and Biscyclohexanone Oxaldihydrazone,* 1966
- **CARLTON WW.** *Studies On the Induction of Hydrocephalus and Spongy Degeneration by Cuprizone Feeding and Attempts to Antidote the Toxicity,* 1967
- **TANSEY FA et al.** *Expression of Carbonic Anhydrase II mRNA and Protein in Oligodendrocytes During Toxic Demyelination in the Young Adult Mouse,* 1996
- **SUN SW1 et al.** *Noninvasive Detection of Cuprizone Induced Axonal Damage and Demyelination in the Mouse Corpus Callosum,* 2006
- **TSIPERSON V et al.** *GAS6 Enhances Repair Following Cuprizone-Induced Demyelination,* 2010
- **GUDI V1 et al.** *Glial Response During Cuprizone-Induced De- and Remyelination in the CNS: Lessons Learned,* 2014